(19) Europäisches Patentamt
European Patent Office
Office européen des brevets

(11) EP 3 383 380 B1

(12) # EUROPEAN PATENT SPECIFICATION

(45) Date of publication and mention of the grant of the patent:
**04.01.2023 Bulletin 2023/01**

(21) Application number: **16808759.1**

(22) Date of filing: **30.11.2016**

(51) International Patent Classification (IPC):
***A61K 31/16*** (2006.01) ***A61K 31/202*** (2006.01)
***C07C 323/52*** (2006.01) ***C07C 323/54*** (2006.01)
***C07C 323/58*** (2006.01) ***C07C 229/22*** (2006.01)
***C07C 229/30*** (2006.01) ***C07C 233/09*** (2006.01)
***C07C 57/02*** (2006.01) ***C07C 57/52*** (2006.01)
***C07C 59/42*** (2006.01) ***C07C 59/56*** (2006.01)
***C07C 59/58*** (2006.01) ***C07C 59/60*** (2006.01)

(52) Cooperative Patent Classification (CPC):
**C07C 229/22; A61K 31/16; A61K 31/202; A61P 27/06; A61P 27/12; C07C 57/02; C07C 57/03; C07C 57/52; C07C 59/42; C07C 59/56; C07C 59/58; C07C 59/60; C07C 59/76; C07C 229/30; C07C 233/09;** (Cont.)

(86) International application number:
**PCT/GB2016/053769**

(87) International publication number:
**WO 2017/093732 (08.06.2017 Gazette 2017/23)**

(54) **LIPID COMPOUNDS AND COMPOSITIONS AND THEIR OPTHALMIC USE**

LIPIDVERBINDUNGEN UND ZUSAMMENSETZUNGEN UND DEREN OPHTHALMOLOGISCHE VERWENDUNG

COMPOSÉS LIPIDIQUES ET COMPOSITIONS ASSOCIÉES ET LEUR UTILISATION OPHTALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2015 GB 201521085**

(43) Date of publication of application:
**10.10.2018 Bulletin 2018/41**

(73) Proprietor: **Biozep AS**
**0484 Oslo (NO)**

(72) Inventor: **HOLMEIDE, Anne Kristin**
**0484 Oslo (NO)**

(74) Representative: **Aera A/S**
**Niels Hemmingsens Gade 10, 5th Floor**
**1153 Copenhagen K (DK)**

(56) References cited:
**EP-A1- 0 413 528** **EP-A1- 2 409 963**
**WO-A1-2006/044391** **WO-A2-01/30337**
**WO-A2-2005/060542** **WO-A2-2011/064779**
**IL-A- 113 228** **US-A- 3 972 907**
**US-A1- 2005 137 123** **US-A1- 2006 269 495**

- **DANIEL H. LOPEZ ET AL: "2-Hydroxy Arachidonic Acid: A New Non-Steroidal Anti-Inflammatory Drug", PLOS ONE, vol. 8, no. 8, 27 August 2013 (2013-08-27) , page e72052, XP055345829, DOI: 10.1371/journal.pone.0072052**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PARKKARI T ET AL: "alpha-Methylated derivatives of 2-arachidonoyl glycerol: Synthesis, CB1 receptor activity, and enzymatic stability", BIOORGANIC & MEDICINAL CHEMISTRY LETTERS, PERGAMON, AMSTERDAM, NL, vol. 16, no. 9, 1 May 2006 (2006-05-01), pages 2437-2440, XP025106878, ISSN: 0960-894X, DOI: 10.1016/J.BMCL.2006.01.101 [retrieved on 2006-05-01]**
- **RYAN W J ET AL: "Synthesis of (+)- and (-)-2-methylarachidonyl-2'-fluoroethylamid e (O-689)", BIOORGANIC & MEDICINAL CHEMISTRY LETTERS, PERGAMON, AMSTERDAM, NL, vol. 7, no. 20, 21 October 1997 (1997-10-21), pages 2669-2672, XP004136509, ISSN: 0960-894X, DOI: 10.1016/S0960-894X(97)10047-6**
- **Allyn C Howlet ET AL: "SIGNAL TRANSDUCTION OF EICOSANOID CB, RECEPTOR LIGANDS", Life Sciences, 65,, 1 January 1999 (1999-01-01), pages 617-625, XP055346140, Retrieved from the Internet: URL:http://www.sciencedirect.com/science/article/pii/S0024320599002842/pdf?md5=44d83d42df4d613c0b96a148a91039ea&pid=1-s2.0-S0024320599002842-main.pdf [retrieved on 2017-02-15]**
- **DATABASE MEDLINE [Online] US NATIONAL LIBRARY OF MEDICINE (NLM), BETHESDA, MD, US; 1980, LIANG C D ET AL: "Synthesis and biological activities of arachidonic and alpha- and beta-alkyl-substituted arachidonic acid derivatives.", XP002767284, Database accession no. NLM7386264 & ADVANCES IN PROSTAGLANDIN AND THROMBOXANE RESEARCH 1980, vol. 6, 1980, pages 235-238, ISSN: 0361-5952**


(52) Cooperative Patent Classification (CPC): (Cont.)
**C07C 323/52; C07C 323/54; C07C 323/58; C07C 323/64**

## Description

### Field of invention

The present invention relates to the use of fatty acid derivatives in the treatment and/or prevention of ophthalmic disorders, in particular retinal disorders such as age-related macular degeneration and diabetic retinopathy, and ocular inflammatory diseases. It further relates to novel omega-6 polyunsaturated fatty acid derivatives, to pharmaceutical compositions containing them, and to their use in such treatment.

### Background of the invention

Millions of people live with varying degrees of irreversible vision loss because they have an untreatable, degenerative eye disorder which affects the retina. In these conditions, the delicate layer of tissue that lines the inside back of the eye is damaged affecting its ability to send light signals to the brain. Diseases of the retina and retinal function can lead to permanent loss of visual function for which there is no definitive treatment. Vision loss has serious consequences for the individual as well as society. Reduced vision among mature adults has been shown to result in social isolation, family stress, and ultimately a greater tendency to experience other health conditions. Experts predict that by 2030 rates of vision loss will double along with the aging population.

According to Prevent Blindness America (2008), the four leading eye diseases affecting older Americans are age-related macular degeneration (AMD), cataracts, diabetic retinopathy, and glaucoma. As people age, they are far more likely to have serious age-related eye conditions.

AMD is a leading cause of blindness in adults over 55 years of age in the developed world. It afflicts an estimated 30 to 50 million people worldwide and is the leading cause of severe vision loss in Western societies. AMD causes the loss of photoreceptor cells in the central part of the retina (the macula) which is the part of the retina that supplies high acuity central vision. The loss of central vision affects activities such as reading, driving and face recognition, and has a significant negative impact on daily function and quality of life.

Macular degeneration can be classified into two types: dry-form and wet-form. The dry-form is more common than the wet: about 90% of age-related macular degeneration patients are diagnosed with the dry-form. The wet-form of the disease and geographic atrophy, which is the end-stage phenotype of dry-form AMD, causes the most serious vision loss. All patients who develop wet-form AMD are believed to previously have developed dry-form AMD for a prolonged period of time. The exact causes of AMD are still unknown. Although some treatments to slow progression are available for wet AMD, there is currently no cure for this irreversible disease. For the most advanced form, wet AMD, anti-VEGF therapy dominates the market. Despite this significant treatment advancement for wet AMD, sustained visual acuity improvements can only be maintained with burdensome monthly dosing and monitoring. For the vast majority of patients who have the dry form of AMD, no effective treatment is yet available. Because the dry-form of AMD precedes development of the wet-form, therapeutic intervention to prevent or delay disease progression in the dry-form of AMD would benefit those patients with the dry-form of AMD and could serve to reduce the incidence of the wet-form.

The pathological mechanism(s) for AMD have not been definitively elucidated. However, converging evidence from multiple studies implicates mitochondrial dysfunction in the AMD disease process. As a high energy demand organ, the eye is particularly susceptible to the consequences of mitochondrial damage. This damage occurs before vision loss and early intervention targeting the mitochondria function would likely protect or rescue RPE mitochondrial function and therefore attenuate disease progression (The Journal of Neuroscience, May 2015, 7304).

Diabetic retinopathy (DR) is one of the most common microvascular complications of diabetes and remains a major cause of preventable blindness among adults of working age. The prevalence of DR increases with the duration of diabetes, and nearly all patients with type I diabetes and more than 60% with type II diabetes have some degree of retinopathy after 20 years. Current treatment for DR (laser photocoagulation, intravitreal corticosteroids, intravitreal anti-VEGF agents and vitreoretinal surgery) are applicable only at advanced stages of the disease and are associated with significant adverse effects (R. Simo et al., Diabetes Care, 2009, 1556). Therefore, new pharmacological treatments for early stages of the disease are needed.

Diabetic macular edema (DME) is an advanced, vision-limiting complication of DR that affects nearly 30% of patients who have had diabetes for at least 20 years and is responsible for much of the vision loss due to DR. The historic standard of care for DME has been macular laser photocoagulation, which has been shown to stabilize vision and reduce the rate of further vision loss by 50%; however, macular laser photocoagulation leads to significant vision recovery in only 15% of treated patients.

Ophthalmic diseases such as AMD which affect the back of the eye, in particular the retina, choroid and surrounding tissues, are very difficult to treat. Accessibility to the back of eye is one of the main reasons for this difficulty. Currently, most back of the eye or retinal/choroidal diseases are treated using intravitreal injections which deliver a drug into the vitreous adjacent to the retina, or by systemic administration. Intravitreal injections expose the back of the eye

to a concentration of the drug that is sufficiently high to be effective to treat the disease. With systemic delivery, however, it is difficult to achieve adequate drug concentrations in the retina and high systemic doses may cause adverse side-effects.

Retinal disorders are a large and diverse group of conditions affecting young and old from many cultures, races and ethnicities. Many ophthalmic disorders are inherited, meaning that they are due to a genetic mutation. An individual can inherit such a mutation, even if they have no clear family history of vision loss. In other instances, many members and generations of a family may experience vision loss.

Retinitis pigmentosa (RP) affects the pediatric and young adult population, and is the leading cause of inherited retinal degeneration-associated blindness. Diabetic retinopathy (DR) is the principal cause of blindness in middle-aged working adults. Stargardt's disease is the most common form of inherited juvenile macular degeneration for which there is currently no treatment. The progressive vision loss associated with Stargardt's disease is caused by the death of photoreceptor cells in the macula. Decreased central vision is a hallmark of Stargardt's disease. Side vision is usually preserved. Stargardt's disease typically develops during childhood and adolescence.

A topical treatment would be a welcome relief to people suffering from retinal diseases such as AMD. The adverse effects associated with intravitreal injections are mainly related to complications with the injection procedure and can include inflammation within the eye (endophthalmitis), increased eye pressure, traumatic cataract and a detached retina. A topical treatment, for example with a cream or with eye drops, would make AMD treatment (both dry and wet) and treatment of other retinal diseases significantly more affordable and available for a larger patient group. The ophthalmic pharmaceutical industry has generally been unable to find drugs that upon topical dosing provide sufficient drug concentration to the back of the eye. Thus, finding a drug that will penetrate the cornea, sclera and/or conjunctiva would be a significant advancement.

There is thus an acute need to find alternative methods of treating ophthalmic disorders, such as AMD (both dry and wet forms), diabetic retinopathy, and Stargardt's disease. In particular, there is a need for alternative drug candidates which are able to treat such diseases and which are capable of accumulating in the retina following topical administration.

A need for suitable drug candidates which may be formulated for topical treatment thus exists. This would make treatment regimens more cost-effective and improve patient compliance, thereby resulting in the availability of treatment for many more patients than at present. A topical treatment would also reduce the adverse effects often associated with systemic treatments, especially for elderly people.

The beneficial effects of fenofibrate in diabetic retinopathy have been demonstrated in two large clinical trials (FIELD and ACCORD-EYE). These trials show that fenofibrate treatment provides a relative reduction in DR progression of 30-40% over 4-6 years, with greater benefit in patients with pre-existing DR. These benefits are achieved despite a lack of significant reduction in triglycerides and small LDL cholesterol which is the main indication for treatment with fenofibrate (A. Ciudin et al., PPAR Res., 2013, 686525).

Fenofibrate activates the peroxisome proliferator activator receptor alpha (PPAR$\alpha$) with an $EC_{50}$ value in the micromolar range. PPAR$\alpha$ is a ligand-activated transcription factor and belongs to the nuclear receptor superfamily. It is highly expressed in the liver, and also in the microvascular, neuronal, and glial tissues of many organs, including the retina. PPAR$\alpha$ is an attractive therapeutic target for many diseases and pathological conditions due in part to its antioxidant, anti-inflammatory, and hypolipidemic effects. Interestingly, PPAR$\alpha$ is downregulated in the diabetic retina and kidney, and although the regulatory mechanisms responsible for diabetes-induced PPAR$\alpha$ downregulation are unclear, decreased PPAR$\alpha$ levels may play a pathological role in diabetic microvascular complications. It has also been found that retinal levels of PPAR$\alpha$, but not PPAR$\gamma$ or PPAR$\beta/\delta$, are decreased in diabetes, suggesting that PPAR$\alpha$ plays a more crucial role than other PPARs in repressing development of diabetic retinopathy (Y. Hu et al., Proceedings of the National Academy of Sciences of the United States of America, vol. 110, no. 38, pp. 15401-15406, 2013). A role for PPAR$\alpha$ in protective pathways in AMD models has also been highlighted in studies which demonstrated a potent effect of PPAR$\alpha$ agonists on inhibiting pathological neovascularization in the retina (Del. V. Cano et al., PPAR Res., 2008, 821592).

PPAR agonists for systemic use have, however, been hampered by serious side effects. For example, the fibrates show a variety of negative pharmacotoxicological profiles. Most fibrates cause some level of myopathy, including pain and creatine phosphokinase release, a consequence of muscle death. Mitochondrial impairment is increasingly implicated in the etiology of toxicity caused by some fibrates and it has been shown that the rank order of mitochondrial impairment accords with clinical adverse events observed with different fibrates (Toxicology and Applied Pharmacology 223 (2007) 277-287). There is thus a need for alternative PPAR agonists which do not impair the mitochondrial function.

Polyunsaturated fatty acids and their metabolites are involved in many physiological and pathophysiological reactions and as such possess a range of important biological activities. They affect plasma lipids and lipid metabolism. They are incorporated into cell membranes where they influence different cell functions. They are also involved in inflammatory diseases and they also influence and control gene expression. However, due to their limited stability *in vivo* and their lack of biological specificity, PUFAs have not achieved widespread use as therapeutic agents except as

triglyceride lowering agents. The only approved indication for polyunsaturated fatty acid derived drugs is the reduction of triglyceride (TG) levels in adult patients with severe (≥500 mg/dL) hypertriglyceridemia (HTG).

The Age-Related Eye Disease Study (AREDS) was designed to determine if daily intake of certain vitamins and minerals could reduce the risk of cataract and advanced age-related macular degeneration (AMD). This study included a placebo-controlled trial, launched in 1992, to evaluate a combination of vitamins E and C, beta-carotene, and zinc - known as the AREDS formulation. In 2001, the investigators reported that the AREDS formulation reduced the risk of advanced AMD by about 25 % over a five-year period. There was no effect on cataract. In 2006, the investigators began a separate clinical trial called AREDS2. The primary goal was to determine if adding omega-3 fatty acids or the antioxidants lutein and zeaxanthin to the original AREDS formulation would make it more effective for reducing the risk of advanced AMD or cataract. In the AREDS2 trial, adding DHA/EPA or lutein/zeaxanthin to the original AREDS formulation (containing beta-carotene) had no additional overall effect on the risk of advanced AMD (information from the national eye institute - see: https://nei.nih.gov/areds2/MediaQandA).

In WO 2006/117664 it is suggested that alpha-ethyl DHA derivatives have a combined PPAR$\gamma$ and PPAR$\alpha$ effect which may be advantageous to patients with insulin resistance, metabolic syndrome and type II diabetes. In Larsen et al. (Lipids, 2005: 49) alpha alkylation of saturated fatty acids and omega-3 PUFAs is suggested to increase their activation of PPAR receptors compared to their non-alkylated analogues. Alpha-substituted, sulfur or oxygen containing omega-3 PUFAs are also described in WO 2010/128401 and in WO 2010/008299. Some of these compounds are reported to activate PPAR$\alpha$ with an $EC_{50}$ value of 200-400 nM and an efficacy of 80-85% compared to the positive control GW7647 ($EC_{50}$ 0.45 nM, efficacy 100%). However, none of these earlier documents suggests the use of such compounds in the treatment of any ophthalmic disorder or in retinal diseases such as diabetic retinopathy, AMD, and diabetic macular edema.

Against this background, it has now surprisingly been found that certain fatty acid derivatives as described herein have good PPAR$\alpha$ activating properties and are thus particularly suitable for use in the treatment and/or prevention of ophthalmic disorders, in particular retinal diseases such as diabetic retinopathy and AMD. We have also surprisingly found that these derivatives serve to protect against oxidative stress-induced mtDNA damage that has been found to positively correlate with progression of retinal disorders such as AMD (Lin et al., Invest. Ophthalmol. Vis. Sci. 2001, 3521). Whilst not wishing to be bound by theory, since mtDNA damage occurs before vision loss, early intervention to protect or rescue mitochondria function would be expected to attentuate disease progression.

RPE cells are essential for photoreceptor cell survival. When RPE cells are damaged or die, photoreceptor function is impaired, and the photoreceptor cells die as a consequence. Thus, oxidative stress-mediated injury and cell death in RPE cells impairs vision, particularly when the cells of the macula are affected. The macula is the area of the retina responsible for visual acuity. The pathophysiology of many retinal degradations involves oxidative stress leading to apoptosis of RPE cells (Mukherjee, PNAS, 2004, 101, 8491).

IL113228 discloses polyunsaturated fatty acid amids, anadamide derivatives.

WO 01/30337, US 2005/137123, WO 2005/060542, WO 2011/064779, and WO 2006/044391 disclose unsaturated carboxylic acids.

EP 2409963 discloses hydroxy(poly)unsaturated fatty acids and their esters, for use in the treatment of various diseases.

Lopez et al. (Plos One, vol. 8, no. 8, 27 August, 2023) discloses 2-hydroxy arachidonic acid as an orally available non-steroidal anti-inflammatory drug.

US 2006/269495 discloses a topical alpha-hydroxy acid composition comprising 2-hydroxyeicosatetraenoic acid useful for the treatment of skin disorders.

Parkkari et al. (Bioorganic & Medicinal Chemistry Letters, vol. 16, no. 9, 2006) discloses alphamethyl arachidonic acid and glycerol esters thereof as CB1 receptor agonists.

Ryan et al. (Bioorganic & Medicinal Chemistry Letters, vol. 7, no. 20, 1997) discloses (+) and (-)-2methylarachidonyl-2'-fluroethylamide as CB1 agonist.

US 3972907 discloses that 2,2-dialkylated arachidonic dihydroarachidonic and tetrahydroarachidonic acids and their esters are able to inhibit lipogenesis.

EP 0413528 discloses that alphahydroxy fatty acids and esters thereof are useful to treat dermatological disorders.

Howlet et al. (Life Sciences, 65, 1999) discloses that alpha substituted unsaturated fatty acid amides are useful as OBI ligands.

Liang et al. (Advances in Prostaglandin and Thromoxane Research, 1980, vol. 6) discloses that in the male rat, arachidonic acid 1a and 2,2-dimethylarachidonic acid 3a exhibited plasma platelet disaggregation.

We have surprisingly found that compounds of the invention effectively protect RPE cells from apoptosis during conditions of oxidative stress. This observation substantiates the possible use of these compounds in the treatment of degenerative eye diseases (e.g. age related macular degeneration (AMD), Stargardt's disease, retinitis pigmentosa, and glaucoma) and the use in treatment for other ocular diseases caused by oxidative stress (e.g. cataract, dry eye

disease, uveitis, etc.).

**Summary of the invention**

In one aspect the present invention relates to a lipid compound of formula (I), or a pharmaceutically acceptable salt thereof, for use in the prevention and/or treatment of an ophthalmic disorder, in particular a retinal degenerative disorder or an ocular inflammatory disease:

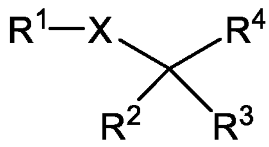

(I)

wherein

$R^1$ is a $C_9$ to $C_{22}$ alkenyl group having from 1 to 6 double bonds;
$R^2$ is selected from the group consisting of a halogen atom, a hydroxyl group, an alkyl group, an alkoxy group, an alkylthio group, a carboxy group, an acyl group, an amino group, and an alkylamino group;
$R^3$ is a hydrogen atom, or a group $R^2$;
$R^4$ is a carboxylic acid or a derivative thereof which is a carboxylic ester; and
X is methylene ($-CH_2-$), or an oxygen or sulfur atom.

In another aspect the disclosure relates to a novel omega-6 lipid compound of formula (II), or a pharmaceutically acceptable salt thereof:

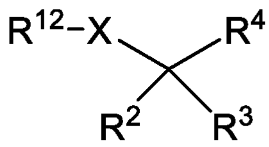

(II)

wherein

$R^{12}$ is a $C_9$ to $C_{22}$ alkenyl group having from 1 to 5 double bonds (e.g. 2 to 5 double bonds) in which:

- the first double bond counting from the ω-end is at carbon 6; and
- where two or more double bonds are present, at least one pair of consecutive double bonds is interrupted by a single methylene group;

$R^2$ is selected from the group consisting of a halogen atom, a hydroxy group, an alkyl group, an alkoxy group, an alkylthio group, a carboxy group, an acyl group, an amino group, and an alkylamino group;
$R^3$ is a hydrogen atom, or a group $R^2$;
$R^4$ is a carboxylic acid or a derivative thereof which is a carboxylic ester; and
X is an oxygen or sulfur atom.

In a further aspect the disclosure relates to an omega-6 lipid compound of formula (II), or a pharmaceutically acceptable salt thereof for use as a medicament.

In a further aspect the disclosure relates to an omega-6 lipid compound of formula (II), or a pharmaceutically acceptable salt thereof for use in the prevention and/or treatment of an ophthalmic disorder, in particular a retinal degenerative disorder or an ocular inflammatory disease.

In a further aspect the disclosure relates to a process for the preparation of an omega-6 lipid compound of formula (II), or a pharmaceutically acceptable salt thereof.

In a further aspect the present disclosure provides a pharmaceutical composition comprising an omega-6 lipid compound of formula (II), or a pharmaceutically acceptable salt thereof, together with one or more pharmaceutically acceptable carriers, excipients or diluents.

A further aspect of the present disclosure relates to a lipid composition comprising an omega-6 lipid compound

of formula (II), or a pharmaceutically acceptable salt thereof. The use of such a lipid composition as a medicament, in particular in the prevention and/or treatment of an ophthalmic disorder (e.g. a retinal degenerative disease or an ocular inflammatory disease), forms a further aspect of the invention.

## Detailed description of the invention

### Definitions

As used herein, the term "lipid compound" relates to a fatty acid analogue derived from a saturated or unsaturated fatty acid, e.g. from a mono-unsaturated fatty acid, or a poly-unsaturated fatty acid.

As used herein, the term "lipid composition" relates to a composition comprising at least one lipid compound as herein defined, together with one or more naturally occurring or non-naturally occurring lipid components, for example a saturated fatty acid or a mono- or poly-unsaturated fatty acid. It is envisaged that a "lipid compound" as herein described for use according to the invention will form the major component of any lipid composition.

A "pharmaceutically effective amount" relates to an amount that will lead to the desired pharmacological and/or therapeutic effect, i.e. an amount of the agent which is effective to achieve its intended purpose. While individual patient needs may vary, determination of optimal ranges for effective amounts of the active agent is within the capability of one skilled in the art. Generally, the dosage regimen for treating an ophthalmic disorder with any of the compounds described herein is selected in accordance with a variety of factors including the the nature of the medical condition and its severity.

By "a pharmaceutical composition" is meant a composition in any form suitable to be used for a medical purpose.

"Treatment" includes any therapeutic application that can benefit a human or non-human animal (e.g. a non-human mammal). Both human and veterinary treatments are within the scope of the present invention, although primarily the invention is aimed at the treatment of humans. Veterinary treatment includes the treatment of livestock and domestic animals (e.g. pets such as cats, dogs, rabbits, etc.). It also includes the treatment of farmed fish, e.g. salmon. Treatment may be in respect of an existing ophthalmic disorder or it may be prophylactic.

Fatty acids are straight-chained hydrocarbons having a carboxylic acid (-COOH) group at one end, conventionally denoted the $\alpha$ (alpha) end. By convention, the numbering of the carbon atoms starts from the $\alpha$-end such that the carbon atom of the carboxylic acid group is carbon atom number 1. The $\alpha$-carbon is the carbon atom in the hydrocarbon chain adjacent to the carbon atom of the -COOH group (i.e. carbon number 2 is the $\alpha$-carbon). The $\beta$ (beta) carbon is the next carbon atom along in the hydrocarbon chain (i.e. carbon number 3 is the $\beta$-carbon). The other end, which is usually a methyl ($-CH_3$) group, is conventionally denoted $\omega$ (omega) such that the terminal carbon atom is the $\omega$-carbon. Any double bonds present are labelled with cis-/trans- notation or E-/Z- notation, where appropriate.

The term "alpha-substituted" or "alpha-substitution" refers to a substitution at the carbon atom denoted 2 in accordance with the numbering of the carbon chain as described above.

In "$\omega$-x" (omega-x; also sometimes described as n-x) nomenclature a double bond is located on the xth carbon-carbon bond, counting from the terminal carbon (i.e. the $\omega$-carbon, equivalently referred to as the n-carbon) toward the carbonyl carbon. For example, $\alpha$-linolenic acid is classified as an n-3 or omega-3 (or simply "$\omega$-3") fatty acid.

As used herein, the term "methylene interrupted double bonds" relates to the case where a methylene group ($-CH_2-$) is located in between two separate double bonds in a carbon chain of a lipid compound with no other types of functional group located in between these two double bonds. Successive double bonds can be interrupted by one or more than one (e.g. two or three) methylene groups. In certain embodiments, successive or consecutive double bonds are separated by only one methylene group.

As will be understood, the compounds described herein may exist in various stereoisomeric forms, including enantiomers, diastereomers, and mixtures thereof. The invention encompasses all optical isomers of the compounds described herein and mixtures of optical isomers. Hence, compounds that exist as diastereomers, racemates and/or enantiomers are within the scope of the invention.

As used herein, the term "ophthalmic disorder" is to be construed broadly to encompass any disease or condition which affects any part or parts of the eye. The disorder may involve the optic nerve, retina, extraocular eye muscles, eyelids, anterior segment of the eye, posterior segment of the eye, eye surface, or cornea, for example. It may be hereditary meaning that it is due to a genetic mutation, but it need not be. Examples of specific ophthalmic disorders which may be treated or otherwise prevented in accordance with the invention are provided herein. Those conditions affecting the back of the eye, for example the optic nerve or the retina (or parts of the retina, e.g. the macula), are of particular interest.

## Compounds useful in the invention

The invention is based in part on the discovery that certain lipid compounds are capable of treating and/or preventing ophthalmic disorders, in particular retinal disorders such as diabetic retinopathy and age-related macular

degeneration (AMD), and ocular inflammatory disorders.

In one aspect the present invention therefore relates to a lipid compound of formula (I), or a pharmaceutically acceptable salt thereof, for use in the treatment and/or prevention of an ophthalmic disorder:

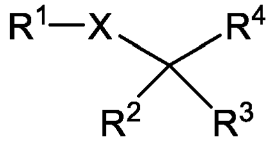

(I)

In formula (I):

$R^1$ is a $C_9$ to $C_{22}$ alkenyl group having from 1 to 6 double bonds;
$R^2$ is selected from the group consisting of a halogen atom, a hydroxyl group, an alkyl group, an alkoxy group, an alkylthio group, a carboxy group, an acyl group, an amino group, and an alkylamino group;
$R^3$ is a hydrogen atom, or a group $R^2$;
$R^4$ is a carboxylic acid or a derivative thereof which is a carboxylic ester; and
X is methylene ($-CH_2-$), or an oxygen or sulfur atom.

As will be understood from general formula (I), the lipid compounds for use in the invention have at least one alpha-substituent, i.e. $R^2$ is other than hydrogen. In some cases two alpha-substituents may be present, i.e. where $R^3$ is a group $R^2$. In these cases, the two alpha-substituents may be the same or different, i.e. $R^3$ can be independently selected from any of the groups listed for $R^2$. In certain embodiments $R^2$ and $R^3$ may be identical. Certain compounds for use in the invention may additionally include a heteroatom at the beta-position, i.e. where X is either an oxygen or sulfur atom. Although not wishing to be bound by theory, alpha-substitution and/or the presence of a beta-heteroatom is considered to improve the metabolic stability of the lipid compounds compared to their corresponding natural fatty acids, e.g. to improve their stability with respect to $\beta$-oxidation.

Where $R^2$ and/or $R^3$ is a halogen atom, this may be selected from the group consisting of fluorine, chlorine, bromine, and iodine. However, most typically this will be fluorine.

Where $R^2$ and/or $R^3$ is an alkyl group this may be straight-chained or branched, preferably a straight-chained or branched $C_{1-6}$ alkyl. For example, the alkyl group may be selected from methyl, ethyl, n-propyl, isopropyl, n-butyl, sec-butyl, and n-hexyl. Preferred alkyl groups are short-chained, e.g. having 1 to 3 carbon atoms. The alkyl group may be substituted or unsubstituted. Where this is substituted it may be mono- or poly-substituted. Examples of suitable substituents include halogen (e.g. fluorine), hydroxy, thiol, or methoxy, so for example the alkyl group may be selected from among $-CF_3$, $-CH_2CF_3$, $-CH_2OCH_3$, $-CH_2OCF_3$, and $-CH_2CH_2OCH_3$. In one embodiment, however, the alkyl group will be unsubstituted. Unsubstituted $C_{1-6}$ alkyl groups, preferably $C_{1-3}$ alkyl groups, e.g. methyl and ethyl, are preferred.

Where $R^2$ and/or $R^3$ is an alkoxy group, the alkyl component of the alkoxy group may be any alkyl group as defined above. For example, the alkoxy group may be selected from the group consisting of methoxy, ethoxy, propoxy, isopropoxy, sec-butoxy, $-OCH_2CF_3$, and $-OCH_2CH_2OCH_3$. It is preferred that the alkyl component is a straight-chained alkyl having from 1 to 6 carbons, e.g. 1 to 3 carbons. Methoxy and ethoxy are particularly preferred.

Where $R^2$ and/or $R^3$ is an alkylthio group, the alkyl component of said alkylthio group is preferably an alkyl group as defined above. For example, the alkylthio group may be selected from the group consisting of methylthio, ethylthio, and isopropylthio.

Where $R^2$ and/or $R^3$ is an acyl group, the alkyl component of the acyl group may be any alkyl group as defined above. Typically, this will be an unsubstituted, straight-chained alkyl group. For example, the acyl group may be a group of the formula $-C(O)C_{1-6}$ alkyl, e.g. $-C(O)CH_2CH_3$ or $-C(O)CH_3$.

Where $R^2$ and/or $R^3$ is an alkylamino group this may be a group of the formula -NHR', or a group of the formula $-NR'_2$ wherein each R' is independently a $C_{1-3}$ alkyl group, e.g. methyl. For example, the alkylamino group may be selected from the group consisting of methylamino, dimethylamino, ethylamino, and diethylamino.

In an embodiment the compounds for use in the invention include a single alpha-substituent, i.e. $R^3$ is a hydrogen atom.

In an embodiment $R^3$ is hydrogen, and X is selected from oxygen and sulfur. Such compounds include both an alpha-substituent and a beta-heteroatom and may be represented by the general formulae (Ia) and (Ib):

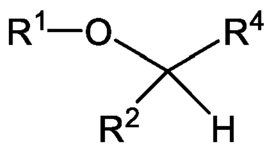
(Ia)

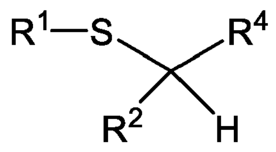
(Ib)

In formula (Ia) and (Ib), $R^1$, $R^2$ and $R^4$ are as described herein. Preferably $R^2$ is a fluorine atom, optionally substituted $C_{1-3}$ alkyl (e.g. methyl, ethyl, $-CF_3$ or $-CH_2CF_3$), $C_{1-3}$ alkoxy (e.g. methoxy or ethoxy), $C_{1-3}$ alkylthio (e.g. $-SCH_3$ or $-SCH_2CH_3$), an amino group, or an alkylamino group of the formula $-NR'_2$ where each R' is independently a hydrogen atom or a $C_{1-3}$ alkyl group (e.g. methylamino or dimethyl amino). More preferably, $R^2$ is methyl or ethyl.

In an embodiment $R^3$ is hydrogen and X is $-CH_2-$. In such compounds $R^1$, $R^2$ and $R^4$ are as described herein. Preferably $R^2$ is a hydroxy group, an optionally substituted $C_{1-3}$ alkyl (e.g. methyl, ethyl, $-CF_3$ or $-CH_2CF_3$), $C_{1-3}$ alkoxy (e.g. methoxy or ethoxy), $C_{1-3}$ alkylthio (e.g. $-SCH_3$ or $-SCH_2CH_3$), a carboxy group, an acyl group (e.g. $-C(O)CH_3$), an amino group, or an alkylamino group of the formula $-NR'_2$ where each R' is independently a hydrogen atom or a $C_{1-3}$ alkyl group (e.g. methylamino or dimethyl amino). Preferably, $R^2$ is $C_{1-3}$ alkyl, e.g. methyl or ethyl, or $C_{1-3}$ alkoxy, e.g. methoxy or ethoxy.

In an embodiment the compounds for use in the invention include two alpha-substituents, i.e. $R^3$ is other than hydrogen. In this embodiment, $R^2$ and $R^3$ may be the same or different. Preferably they will be same and may, for example, both represent an unsubstituted $C_{1-3}$ alkyl, e.g. methyl or ethyl.

The substituent $R^4$ is a carboxylic acid (-COOH) group or a derivative thereof. Suitable derivatives include a carboxylic ester, a carboxylic anhydride, a carboxamide, a monoglyceride, a diglyceride, a triglyceride, and a phospholipid.

Where $R^4$ is a carboxylic ester then the compounds for use in the invention may be a compound of formula (I) in which $R^4$ is a group of the formula $-COOR^5$ where $R^5$ is an alkyl group as defined herein, preferably a $C_{1-6}$ alkyl group. Preferably $R^4$ may be selected from the group consisting of ethyl carboxylate, methyl carboxylate, n-propyl carboxylate, isopropyl carboxylate, n-butyl carboxylate, sec-butyl carboxylate, and n-hexyl carboxylate.

Where $R^4$ is a carboxylic anhydride then the compounds for use in the disclosure may be a compound of general formula (III):

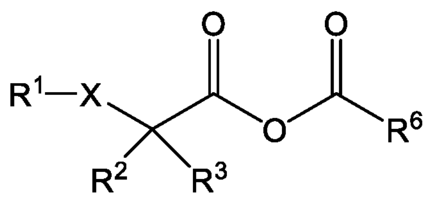
(III)

wherein

$R^1$, $R^2$, $R^3$ and X are as defined herein; and
$R^6$ is an alkyl or alkoxy group as defined herein, preferably a $C_{1-6}$ alkyl or $C_{1-6}$ alkoxy group.

In a further embodiment where $R^4$ is a carboxylic anhydride the compounds for use in the disclosure may be a compound of formula (IV):

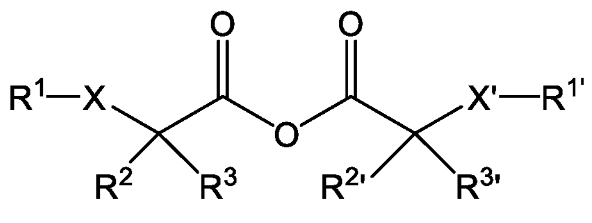
(IV)

wherein
$R^{1'}$, $R^{2'}$, $R^{3'}$ and X' are respectively chosen from among the same groups as $R^1$, $R^2$, $R^3$ and X as herein defined. In this embodiment $R^1$, $R^2$, $R^3$ and X may respectively be the same as or different to $R^{1'}$, $R^{2'}$, $R^{3'}$ and X'. In one embodiment of the compounds of formula (IV), $R^1$, $R^2$, $R^3$ and X, respectively, may be identical to $R^{1'}$, $R^{2'}$, $R^{3'}$ and X'.

Where $R^4$ is a carboxamide then the compound of formula (I) may be a compound of formula (V):

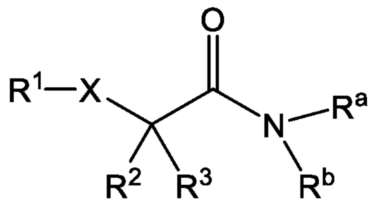

(V)

(wherein

$R^1$, $R^2$, $R^3$ and X are as defined herein; and
$R^a$ and $R^b$ are independently selected from hydrogen and $C_{1-3}$ alkyl (e.g. methyl)).

Where $R^4$ is a carboxamide group, this may be selected from the group consisting of N-methyl carboxamide, N,N- dimethyl carboxamide, N-ethyl carboxamide, and N,N-diethyl carboxamide.

Where $R^4$ is a monoglyceride the compounds for use in the invention may be selected from the following compounds of formula (VI) and (VII):

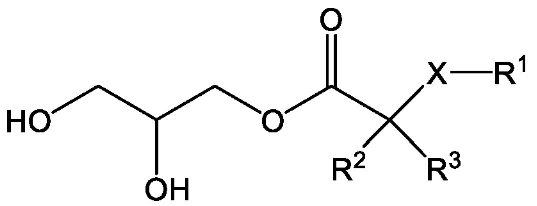

(VI)

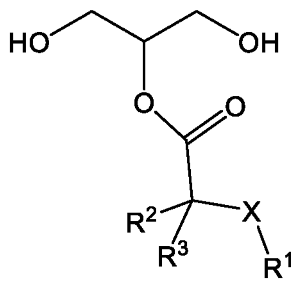

(VII)

wherein $R^1$, $R^2$, $R^3$ and X are as defined herein.

Where $R^4$ is a diglyceride the compounds for use in the disclosure may be selected from the following compounds of formula (VIII) and (IX):

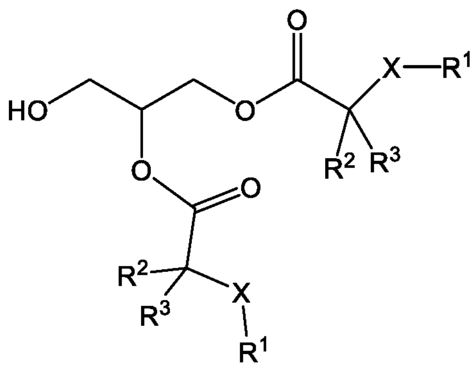

(VIII)

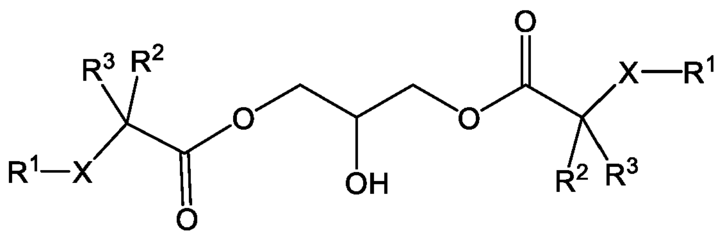

(IX)

wherein $R^1$, $R^2$, $R^3$ and X are as defined herein. In either of these compounds identically labelled substituents may be the same or different, although typically these will be identical to one another.

Where $R^4$ is a triglyceride the compounds for use in the disclosure may be a compound of formula (X):

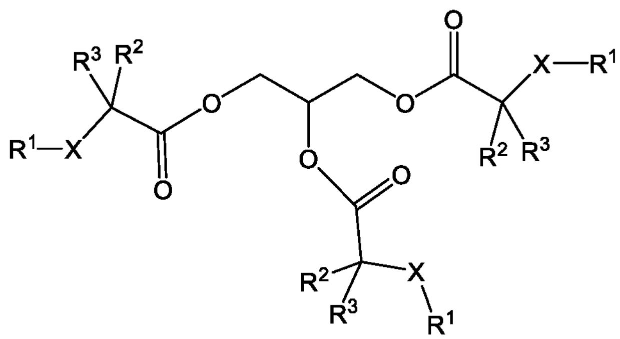

(X)

wherein $R^1$, $R^2$, $R^3$ and X are as defined herein. In the compound of formula (X) identically labelled substituents may be the same or different, although typically these will be identical to one another.

Where the compound of formula (I) is a phospholipid, such compounds may be represented by formulae (XI), (XII) and (XIII):

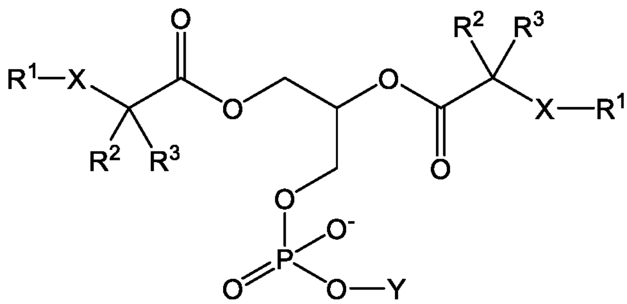

(XI)

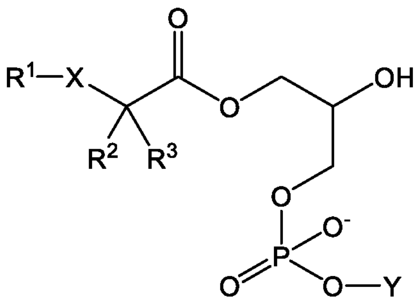

(XII)

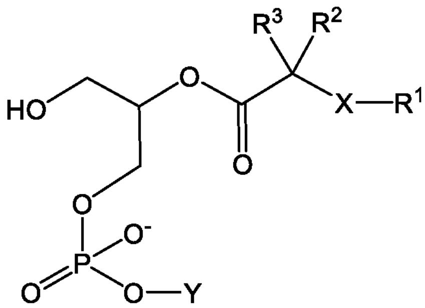

(XIII)

wherein $R^1$, $R^2$, $R^3$ and X are as defined herein, and wherein Y is selected from the following:

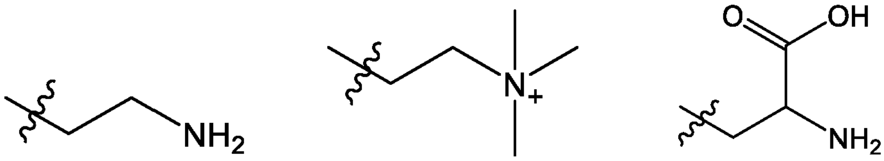

and

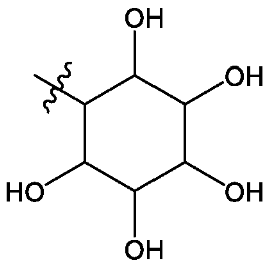

In the compound of formula (XI) identically labelled substituents may be the same or different, although typically these will be identical to one another.

In any of the lipid compounds for use in the invention $R^1$ is either a $C_9$ to $C_{22}$ alkyl group, or a $C_9$ to $C_{22}$ alkenyl group having from 1 to 6 double bonds.

In an embodiment of the disclosure $R^1$ is a $C_9$ to $C_{22}$ alkyl group. In this embodiment, the compounds will typically be derived from a saturated fatty acid. The alkyl group may be straight-chained or branched, although straight-chained $C_9$ to $C_{22}$ alkyl groups are generally preferred.

In certain embodiments the group $R^1$ is a $C_{10}$ to $C_{22}$ alkyl group, preferably a $C_{12}$ to $C_{20}$ alkyl group, more preferably a $C_{12}$ to $C_{16}$ alkyl group, e.g. a $C_{14}$ alkyl group. Such groups may be straight-chained or branched, however these will typically be straight-chained. Shorter chain alkyl groups, such as $C_{12}$ to $C_{16}$ alkyl groups, are generally preferred.

Where $R^1$ is a $C_9$ to $C_{22}$ alkyl group, it is preferred that X is oxygen or sulphur.

In one embodiment where $R^1$ is a $C_9$ to $C_{22}$ alkyl group, X may be oxygen or sulphur, and $R^2$ may be a $C_{1-6}$ alkyl group, e.g. a $C_{1-3}$ alkyl. An example of such a compound is $\alpha$-methyl TTA:

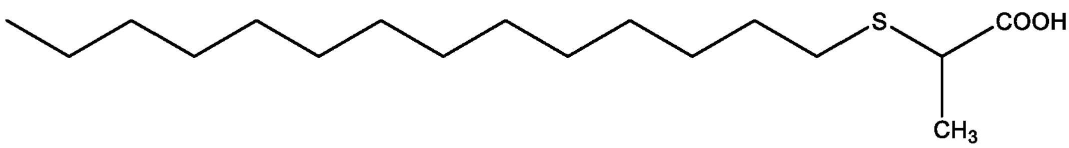

$R^1$: $C_{14}$ alkyl

X: -S-
$R^2$: $-CH_3$
$R^3$: H

$R^4$: $-CO_2H$

In another embodiment the substituent $R^1$ is a $C_9$ to $C_{22}$ alkenyl group having from 1 to 6 double bonds. In this embodiment, the compound will typically be derived from a mono- or poly-unsaturated fatty acid. The alkenyl group may be straight-chained or branched, although straight-chained $C_9$ to $C_{22}$ alkenyl groups are generally preferred.

In certain embodiments the group $R^1$ is a $C_{10}$ to $C_{22}$ alkenyl group, preferably a $C_{12}$ to $C_{19}$ alkenyl group, e.g. a $C_{19}$ alkenyl group, more preferably a $C_{14}$ to $C_{18}$ alkenyl group, e.g. a $C_{15}$ or $C_{17}$ alkenyl group. Such groups may be straight-chained or branched, however these will typically be straight-chained. Shorter chain alkenyl groups, such as $C_{12}$ to $C_{15}$, $C_{13}$ to $C_{17}$ or $C_{14}$ to $C_{18}$ alkenyl groups, are generally preferred. However, in certain embodiments the group $R^1$ is a $C_{19}$ to $C_{22}$ alkenyl group, e.g. a $C_{20}$ alkenyl group.

Preferably $R^1$ is a straight-chained $C_9$ to $C_{22}$ alkenyl group. In one embodiment $R^1$ is a straight-chained $\omega$-3 $C_9$ to $C_{22}$ alkenyl group having from 1 to 6 double bonds. In another embodiment $R^1$ is a straight-chained $\omega$-6 $C_9$ to $C_{22}$ alkenyl group having from 1 to 5 double bonds.

Where $R^1$ is an alkenyl group this may have from 1 to 6 double bonds, preferably from 2 to 4 double bonds, e.g. 2, 3 or 4 double bonds. Where more than one double bond is present it is preferred that at least one pair of successive double bonds is interrupted by no more than one methylene group. In an embodiment each pair of consecutive double bonds is interrupted by no more than one methylene group. Each double bond may independently be in the E-configuration or the Z-configuration. Preferably, where more than one double bond is present, all double bonds are in the same configuration; particularly preferably all are in the Z-configuration. Thus in an embodiment $R^1$ may be a $C_9$ to $C_{22}$ alkenyl group with 2 to 6, preferably 2 to 4, e.g. 2, 3 or 4, double bonds in the Z-configuration where at least one pair of successive double bonds is interrupted by a single methylene group.

In an embodiment X is $-CH_2-$ and $R^1$ is a $C_9$ to $C_{22}$ alkenyl having 2 to 6 double bonds, e.g. a $C_{19}$, $C_{17}$, or $C_{15}$ alkenyl having 3, 4, 5 or 6 double bonds.

In an embodiment X is S or O and $R^1$ is a $C_9$ to $C_{22}$ alkenyl having 2 to 6 double bonds, e.g. a $C_{22}$, $C_{20}$, $C_{18}$, or $C_{15}$ alkenyl having 3, 4, 5 or 6 double bonds.

Where 2 or more double bonds are present in group $R^1$ the compounds for use according to the invention may be derived from known polyunsaturated fatty acids (PUFAs). Particularly preferably the compound for use in the invention is either an $\omega$-3 PUFA derivative or an $\omega$-6 PUFA derivative. Such derivatives may typically retain the structure of group $R^1$ (i.e. retain the same chain length, number and position of double bonds, and E/Z bond configuration) as found in the parent molecule (i.e. PUFA) but are derivatised in that groups X, $R^2$, $R^3$ and/or $R^4$ differ from those normally found in the PUFA.

Examples of PUFAs from which the compounds disclosed herein may be derived include (all-Z)-4,7,10,13,16,19-docosahexaenoic acid (omega-3 docosahexaenoic acid or omega-3 DHA), (all-Z)-5,8,11,14,17-eicosapentaenoic acid (omega-3 eicosapentaenoic acid or omega-3 EPA), (all-Z)-7,10,13,16,19-docosapentaenoic acid (omega-3 docosapentaenoic acid or omega-3 DPA), (all-Z)-9,12,15-octadecatrienoic acid (omega-3 $\alpha$-linoleic acid or omega-3 ALA), (all-Z)-5,8,11,14-icosatetraenoic acid (omega-6 arachidonic acid or omega-6 AA), (all-Z)-4,7,10,13,16-docosapentaenoic acid (omega-6 docosapentaenoic acid or omega-6 DPA), (all-Z)-8,11,14-eicosatrienoic acid (omega-6 dihomo-$\gamma$-linolenic acid or omega-6 DGLA) and (all-Z)-9,12-octadecadienoic acid (omega-6 linoleic acid or omega-6 LA).

In an embodiment $R^1$ is a $C_9$ to $C_{22}$ alkenyl having 2 to 6 double bonds, e.g. a $C_{19}$ alkenyl with 6 double bonds, preferably methylene interrupted double bonds in the Z-configuration (e.g. derived from omega-3 DHA), a $C_{17}$ alkenyl with 5 double bonds, preferably methylene interrupted double bonds in the Z-configuration (e.g. derived from omega-3 EPA); a $C_{15}$ alkenyl with 3 double bonds, preferably methylene interrupted double bonds in the Z-configuration (e.g. derived from omega-3 alpha-linolenic acid); a $C_{19}$ alkenyl with 6 double bonds, preferably methylene interrupted double bonds in the Z-configuration (e.g. derived from omega-3 DHA), a $C_{19}$ alkenyl with 5 double bonds, preferably methylene interrupted double bonds in the Z-configuration (e.g. derived from omega-3 DPA); a $C_{15}$ alkenyl with 4 double bonds, preferably methylene interrupted double bonds in the Z-configuration (e.g. derived from degradation of EPA) or 3 methylene interrupted double bonds in the Z-configuration and one double bond in the E-configuration (e.g. derived from degradation of EPA); or a $C_{18}$ alkenyl with 5 double bonds, preferably methylene interrupted double bonds in the Z-configuration or 4 methylene interrupted double bonds in the Z-configuration and one double bond in the E-configuration (e.g. both derived from degradation of DHA).

In another embodiment $R^1$ is a $C_{17}$ alkenyl with 4 double bonds, preferably methylene interrupted double bonds in the Z-configuration (e.g. derived from omega-6 AA); a $C_{19}$ alkenyl with 5 double bonds, preferably methylene interrupted double bonds in the Z-configuration (e.g. derived from omega-6 DPA); a $C_{15}$ alkenyl with 2 double bonds, preferably methylene interrupted double bonds in the Z-configuration (e.g. derived from omega-6 linoleic acid); a $C_{15}$ alkenyl with 3 double bonds, preferably methylene interrupted double bonds in the Z-configuration (e.g. derived from degradation of AA).

In an embodiment $R^1$ may be a $C_{15}$ alkenyl with 3 or 4 methylene interrupted double bonds in the Z-configuration, a $C_{18}$ alkenyl with 3 to 5 double bonds, e.g. a $C_{18}$ alkenyl with 5 methylene interrupted double bonds in the Z-configuration;

a $C_{14}$ to $C_{22}$ alkenyl group with at least one double bond in the Z-configuration, and having the first double bond at the third carbon-carbon bond from the ω-end of the carbon chain; a $C_{14}$ to $C_{22}$ alkenyl group with at least one double bond in the Z-configuration, and having the first double bond at the sixth carbon-carbon bond from the ω-end of the carbon chain; a $C_{18}$ alkenyl with 3 methylene interrupted double bonds in the Z-configuration; a $C_{20}$ alkenyl with 5 methylene interrupted double bonds in the Z-configuration; or a $C_{22}$ alkenyl with 6 methylene interrupted double bonds in the Z-configuration; a $C_{20}$ alkenyl with 4 methylene interrupted double bonds in the Z-configuration; a $C_{22}$ alkenyl with 5 methylene interrupted double bonds in the Z-configuration; or a $C_{18}$ alkenyl with 2 methylene interrupted double bonds in the Z-configuration.

In one embodiment the substituent $R^1$ is a $C_9$ to $C_{22}$ alkenyl having 2 to 6 double bonds, and the compound of formula (I) is derived from an ω-3 or ω-6 polyunsaturated fatty acid; $R^2$ and $R^3$ are as herein defined, preferably $R^2$ is an alkyl group and $R^3$ is hydrogen; and $R^4$ is a carboxylic acid in the form of a free acid.

In one embodiment the lipid compound of formula (I) is an ω-6 lipid compound wherein $R^1$ is a $C_9$ to $C_{22}$ alkenyl group having 1 to 5 double bonds as herein defined and further wherein the first double bond counting from the ω-end is at carbon 6; and $R^2$, $R^3$, $R^4$ and X are as defined herein.

Preferred ω-6 lipid compounds for use in the invention are those in which $R^1$ has 2 to 4 double bonds, e.g. 2, 3 or 4 double bonds. In certain embodiments such ω-6 lipid compounds may be derivatives of arachidonic acid or linoleic acid.

In certain embodiments, the lipid compound of formula (I) for use in the invention is an ω-6 lipid compound wherein $R^1$ is a $C_{15}$ to $C_{20}$ alkenyl group having 2 to 4 double bonds.

Particularly preferably, the ω-6 lipid compounds for use in the invention have 2 to 4 double bonds as described above which are each methylene-interrupted, i.e. successive double bonds in the alkenyl chain are separated only by $-CH_2-$ groups.

Particularly preferably, where the ω-6 lipid compounds for use in the invention have 2 to 4 double bonds, the double bonds are all in the Z-configuration.

In an embodiment, where the ω-6 lipid compounds for use in the invention have 2 to 4 double bonds, the pairs of successive double bonds are each methylene-interrupted and are all in the Z-configuration.

In certain embodiments the ω-6 lipid compounds according to the invention are those in which X is either oxygen or sulphur and $R^3$ is hydrogen. In an embodiment the ω-6 lipid compounds according to the invention are those in which X is either oxygen or sulphur, $R^3$ is hydrogen and $R^1$ is a $C_9$ to $C_{22}$ alkenyl having 2 to 5 double bonds, e.g. a $C_{20}$, $C_{18}$, or $C_{15}$ alkenyl having 2, 3 or 4 double bonds.

In certain embodiments the ω-6 lipid compounds according to the invention are those in which X is $-CH_2-$ and $R^3$ is hydrogen. In an embodiment the ω-6 lipid compounds according to the invention are those in which X is $-CH_2-$, $R^3$ is hydrogen and $R^1$ is a $C_9$ to $C_{22}$ alkenyl having 2 to 5 double bonds, e.g. a $C_{19}$, $C_{17}$, or $C_{15}$ alkenyl having 2, 4 or 5 double bonds.

In one embodiment the ω-6 lipid compounds for use in the invention include those where $R^1$ is a $C_9$ to $C_{22}$ alkenyl group, X is $-CH_2-$ and $R^2$ is a $C_{1-6}$ alkyl group, e.g. a $C_{1-3}$ alkyl. An example of such a compound is α-ethyl AA:

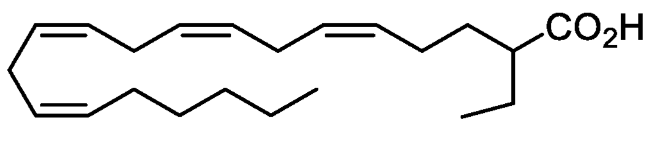

$R^1$: $C_{17}$ alkenyl having four double bonds

X: $-CH_2-$
$R^2$: $-CH_2CH_3$
$R^3$: H
$R^4$: a carboxylic ester

In further embodiments of the invention the lipid compound of formula (I) for use according to the invention is an ω-3 lipid compound wherein $R^1$ is a $C_9$ to $C_{22}$ alkenyl group having 1 to 6 double bonds as herein defined and further wherein the first double bond counting from the ω-end is at carbon 3; and $R^2$, $R^3$, $R^4$ and X are as defined herein.

Preferred ω-3 lipid compounds for use in the invention are those in which $R^1$ has 3 to 6 double bonds, preferably 3 to 5 double bonds, e.g. 3, 4 or 5 double bonds.

In certain embodiments, the lipid compound of formula (I) for use in the invention is an ω-3 lipid compound wherein $R^1$ is a $C_{15}$ to $C_{20}$ alkenyl group having 2 to 5 double bonds, e.g. 3, 4 or 5 double bonds.

Particularly preferably, the ω-3 lipid compounds for use in the invention have 2 to 5 double bonds as described above which are each methylene-interrupted, i.e. successive double bonds in the alkenyl chain are separated only by

$-CH_2-$ groups.

Particularly preferably, where the ω-3 lipid compounds for use in the invention have 2 to 5 double bonds, the double bonds are all in the Z-configuration.

In an embodiment, where the ω-3 lipid compounds for use in the invention have 2 to 5 double bonds, the double bonds are each methylene-interrupted and are all in the Z-configuration.

In certain embodiments the ω-3 lipid compounds for use in the invention are those in which X is either oxygen or sulphur and $R^3$ is hydrogen. In an embodiment the ω-3 lipid compounds for use in the invention are those in which X is either oxygen or sulphur, $R^3$ is hydrogen and $R^1$ is a $C_9$ to $C_{22}$ alkenyl having 2 to 6 double bonds, e.g. a $C_{22}$, $C_{20}$, $C_{18}$, or $C_{15}$ alkenyl having 3, 4, 5 or 6 double bonds.

In certain embodiments the ω-3 lipid compounds for use in the invention are those in which X is $-CH_2-$ and $R^3$ is hydrogen. In an embodiment the ω-3 lipid compounds for use in the invention are those in which X is $-CH_2-$, $R^3$ is hydrogen and $R^1$ is a $C_9$ to $C_{22}$ alkenyl having 2 to 6 double bonds, e.g. a $C_{19}$, $C_{17}$, or $C_{15}$ alkenyl having 3, 4, 5 or 6 double bonds.

In certain embodiments, the lipid compound for use in the invention is an ω-3 lipid compound wherein $R^1$ is a $C_{15}$ to $C_{18}$ alkenyl group with 2 to 5 double bonds, e.g. 4 double bonds.

The lipid compounds for use in the invention may be provided in the form of a pharmaceutically acceptable salt. Suitable salts are well known to those skilled in the art and include, but are not limited to, the lithium, sodium, potassium, ammonium, meglumine, tris(hydroxymethyl)aminomethane, diethylamine, arginine, ethylenediamine, piperazine and chitosan salts.

Examples of suitable omega-6 lipid compounds for use in the disclosure include the following and their pharmaceutically acceptable salts:

| | |
|---|---|
| 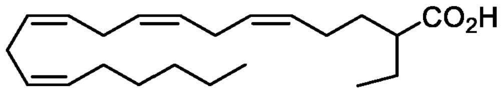 | 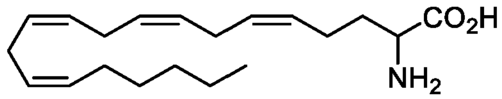 |
| 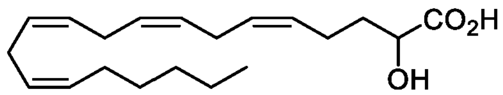 | 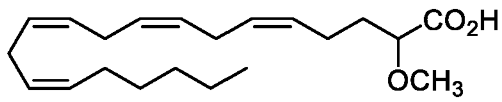 |
| 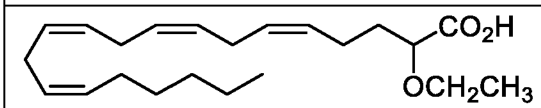 | 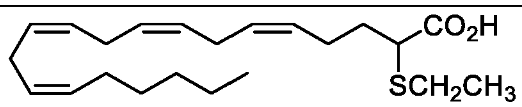 |
| 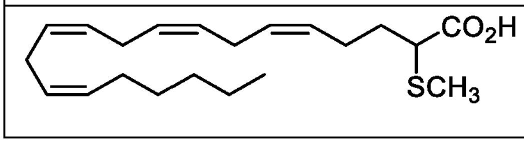 | 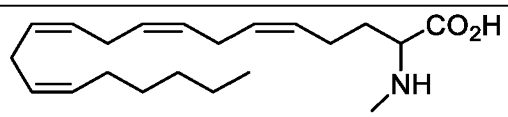 |

| | |
|---|---|
| 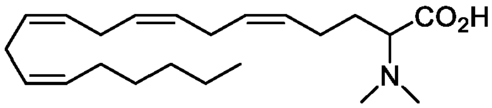 | 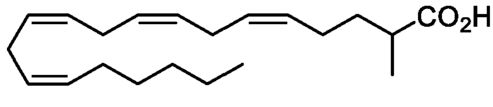 |
| 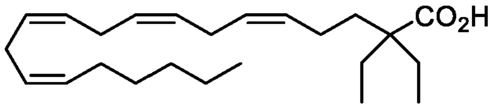 | 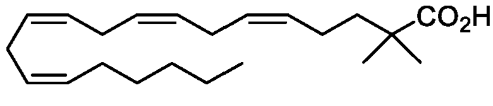 |
| 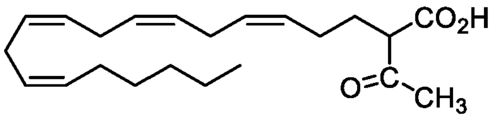 | 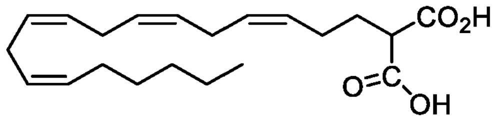 |
| 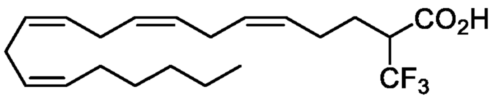 | 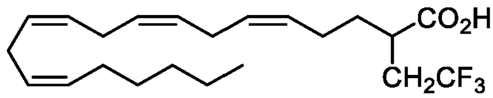 |
| 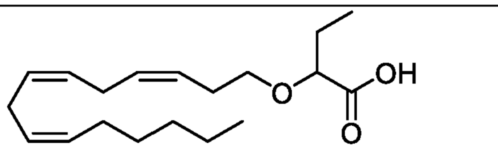 | 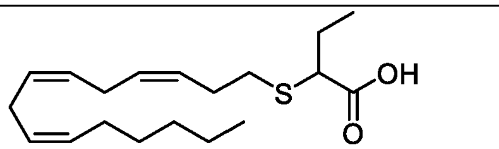 |
| 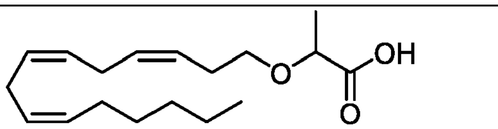 | 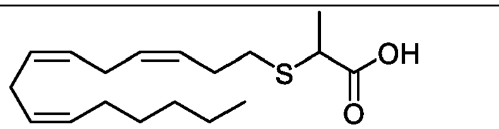 |
| 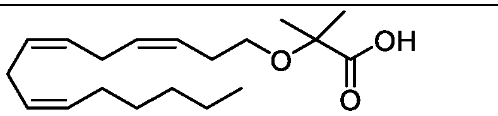 | 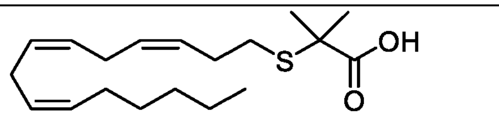 |
| 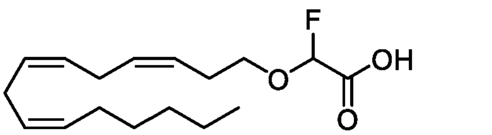 | 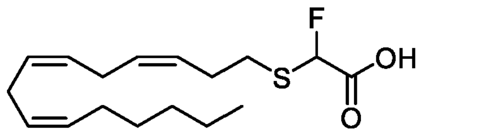 |
| 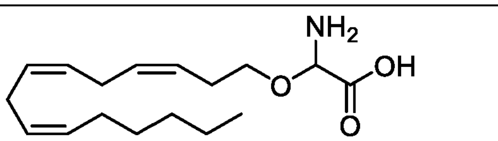 | 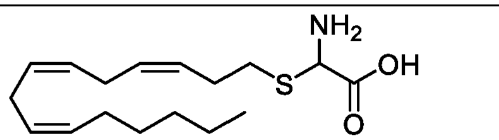 |
| 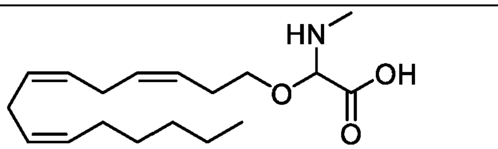 | 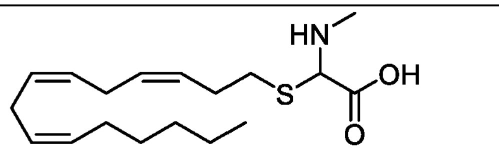 |
| 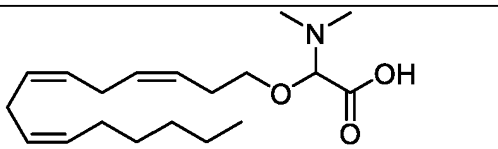 | 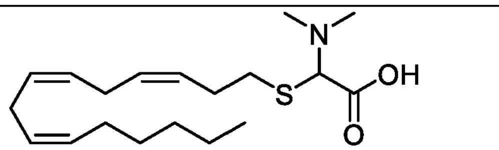 |
| 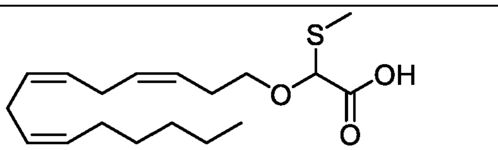 | 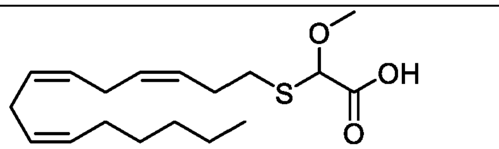 |

| | |
|---|---|
| 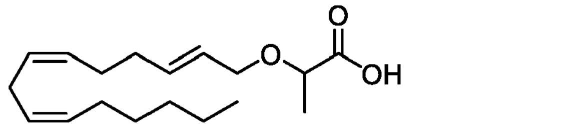 | 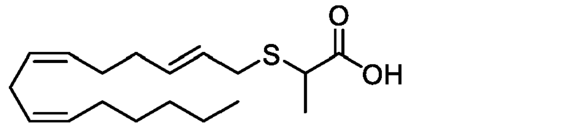 |
| 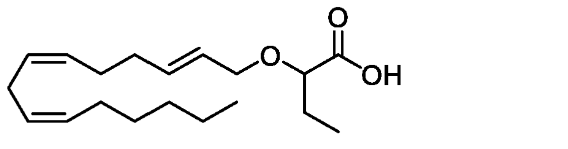 | 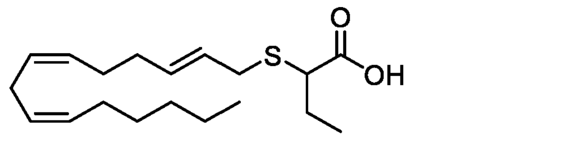 |
| 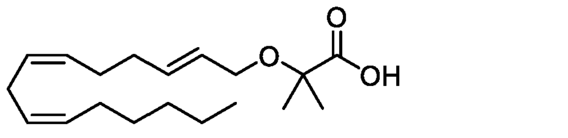 | 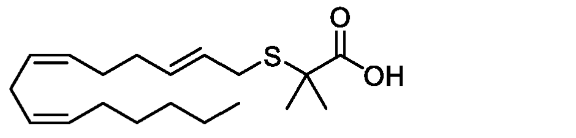 |
| 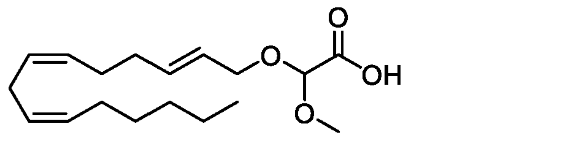 | 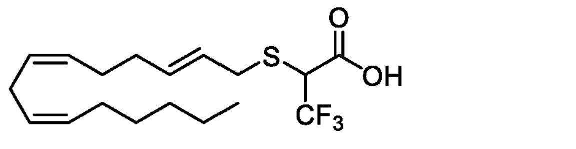 |
| 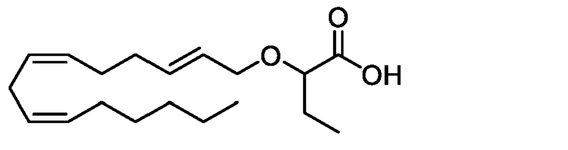 | 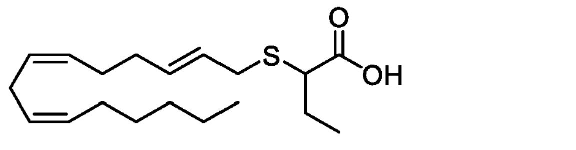 |
| 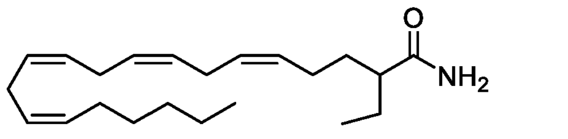 | 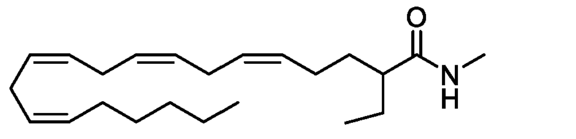 |
| 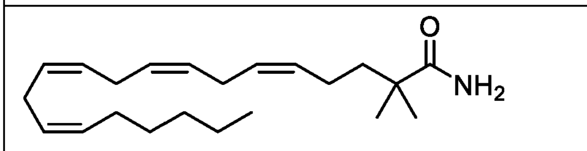 | 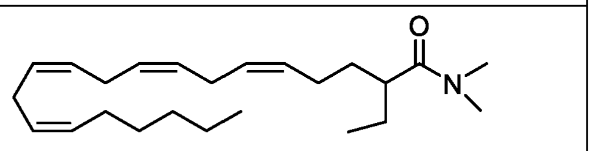 |
| 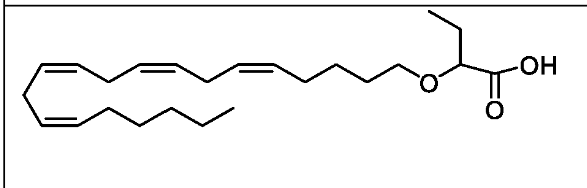 | 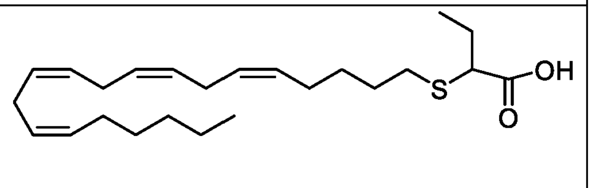 |
| 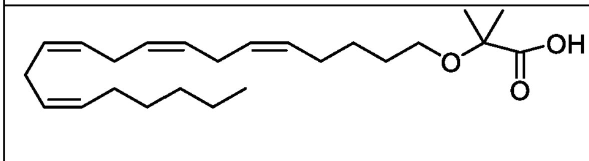 | 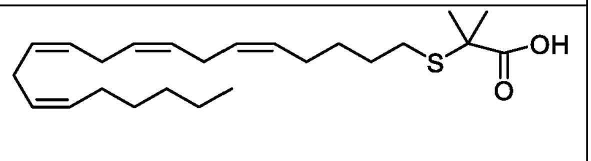 |
| 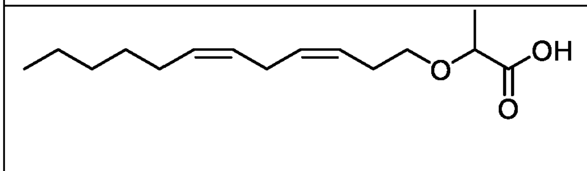 | 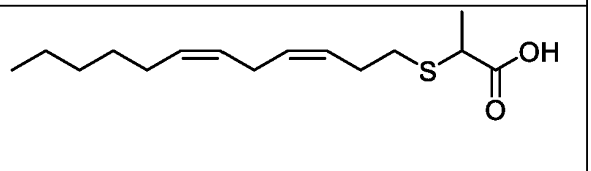 |
| 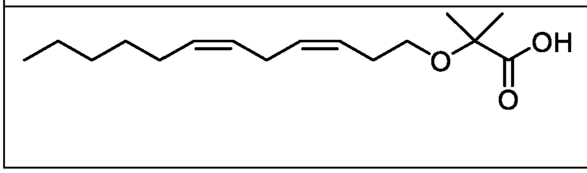 | 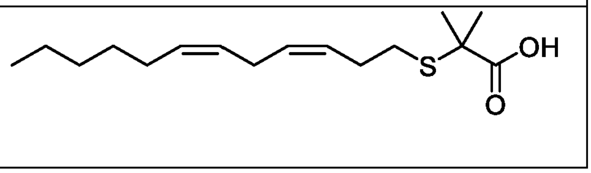 |

| | |
|---|---|
| 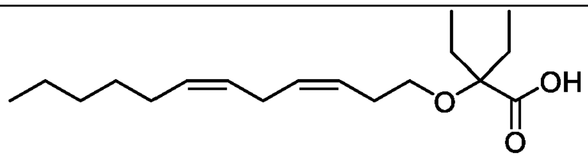 | 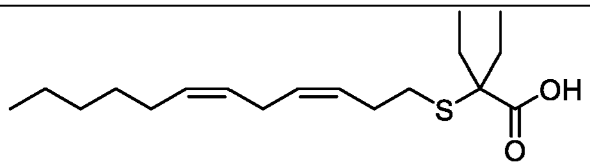 |
| 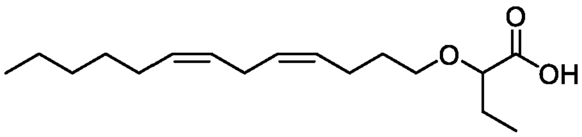 | 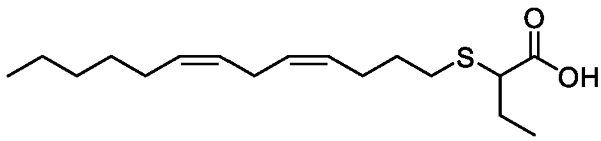 |
| 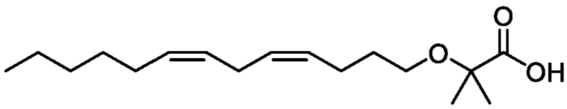 | 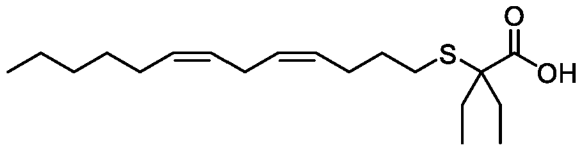 |
| 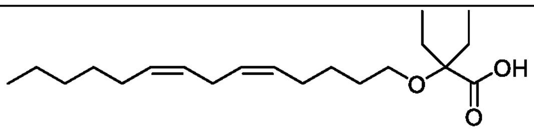 | 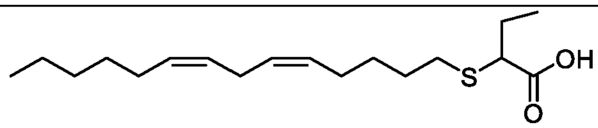 |
| 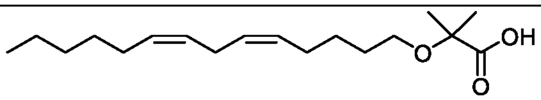 | 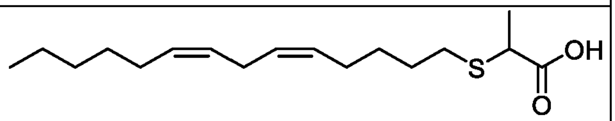 |
| 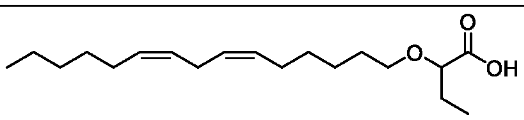 | 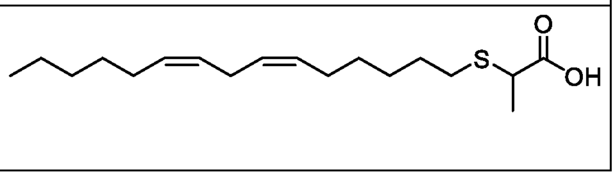 |

Examples of suitable omega-3 lipid compounds for use in the disclosure include the following and their pharmaceutically acceptable salts:

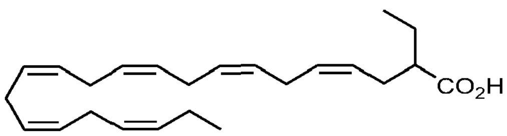
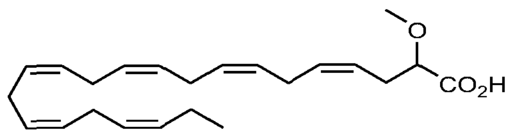

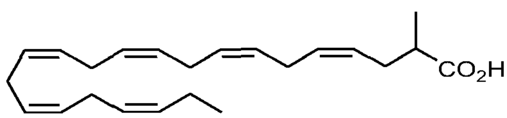
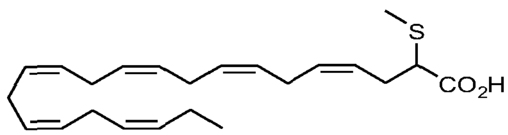

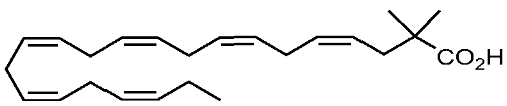
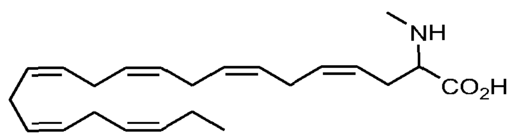

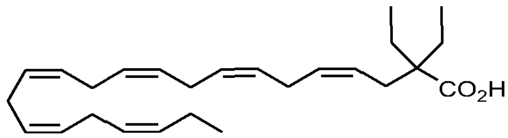

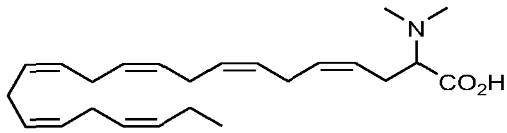

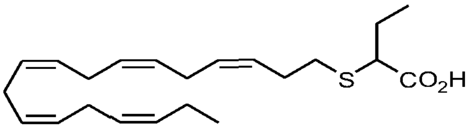

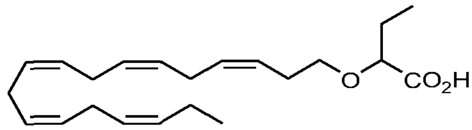

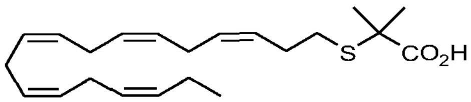

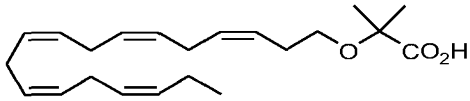

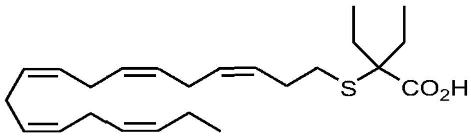

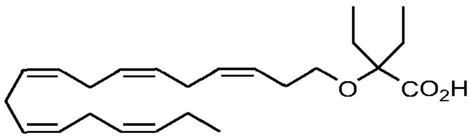

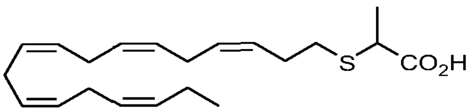

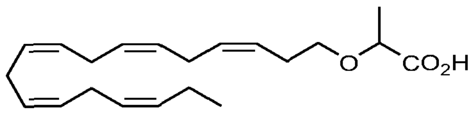

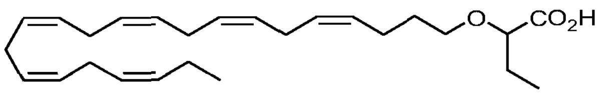

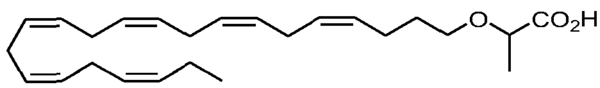

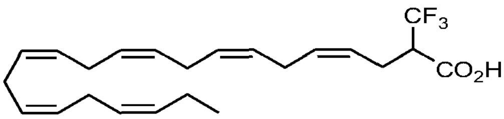

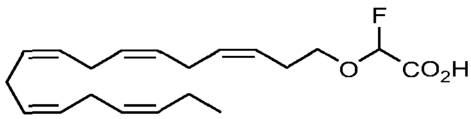

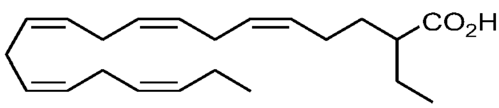

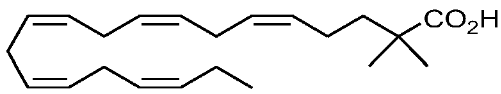

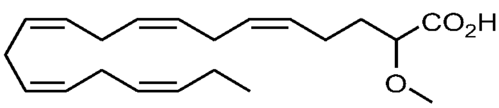

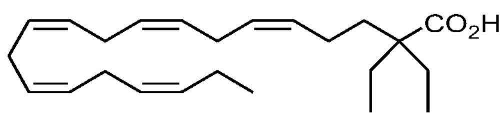

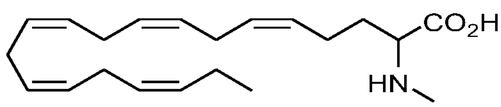

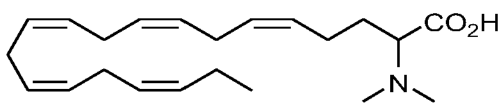

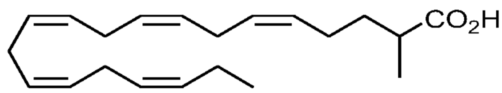

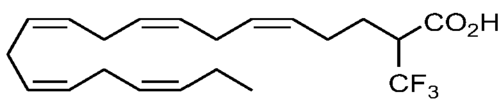

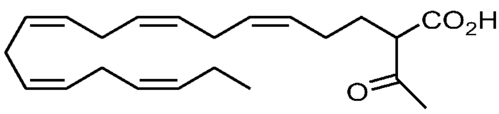

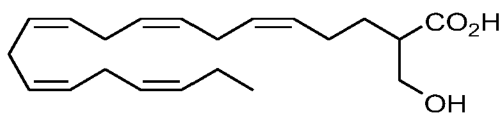

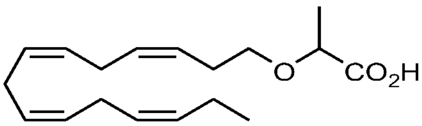

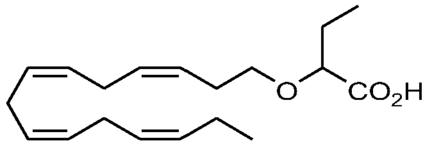

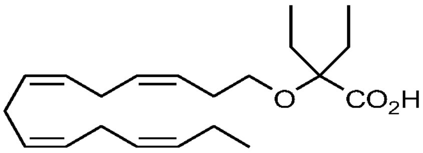

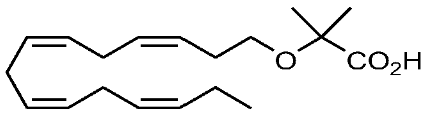

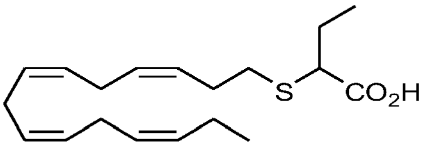

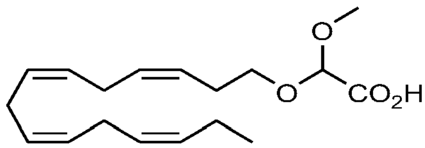

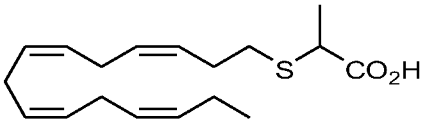

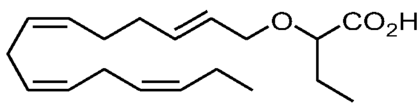

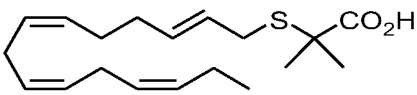

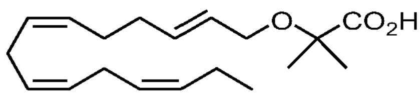

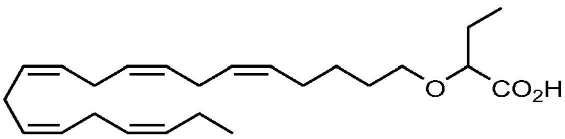

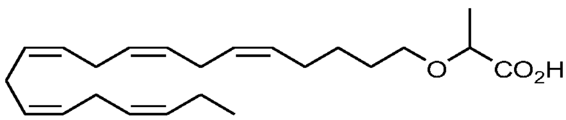

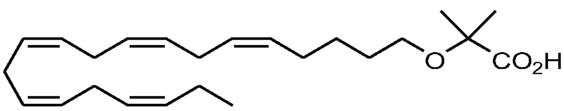

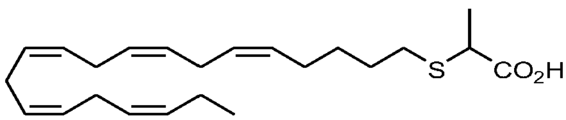

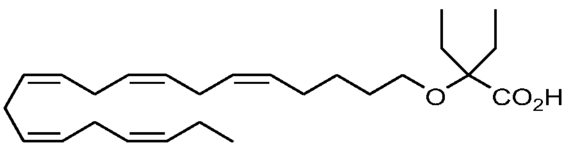

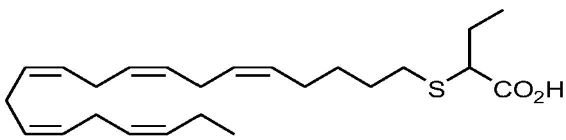

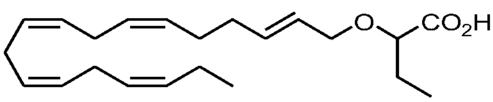

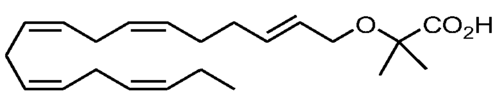

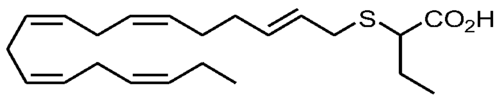

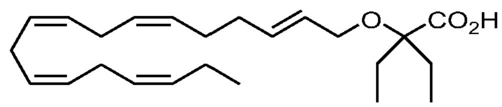

Examples of suitable compounds for use in the disclosure in which $R^1$ is an alkyl group include the following and their pharmaceutically acceptable salts:

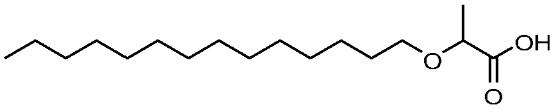

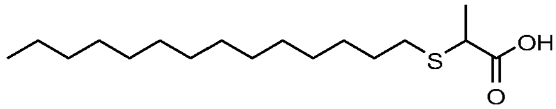

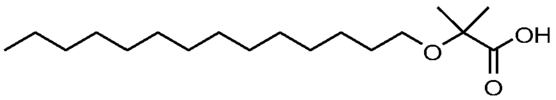

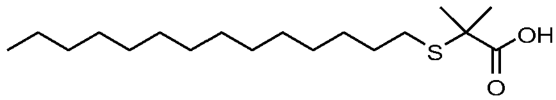

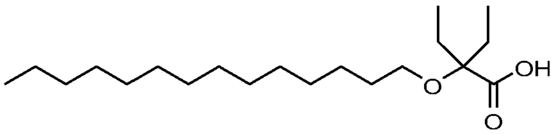

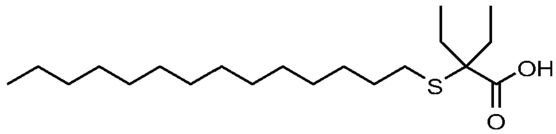

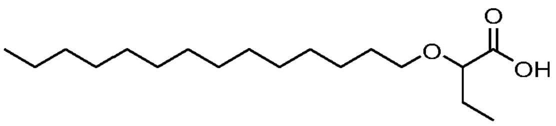

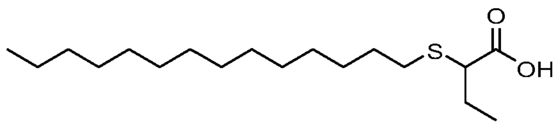

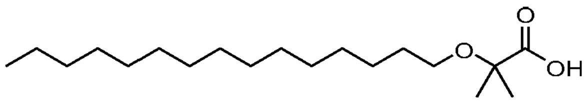

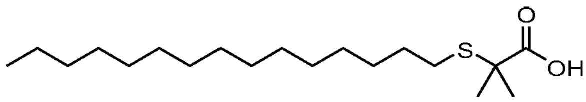

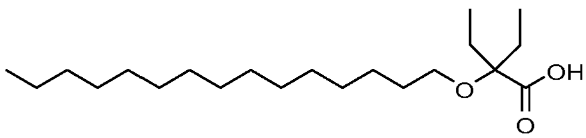

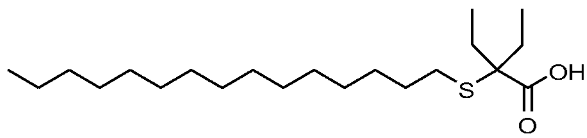

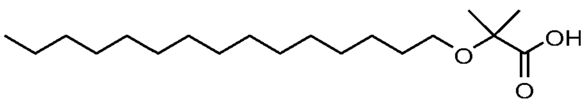

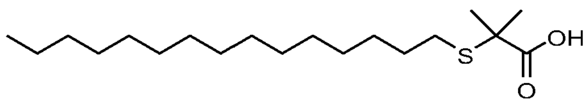

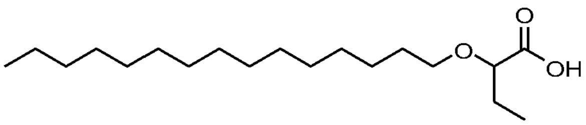

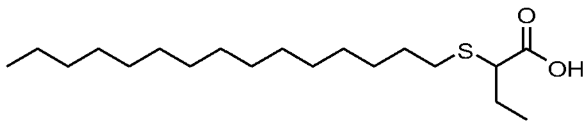

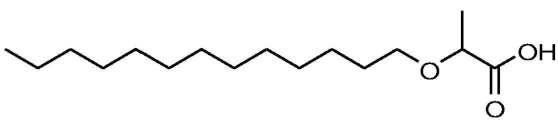

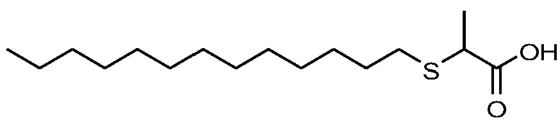

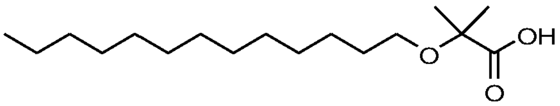

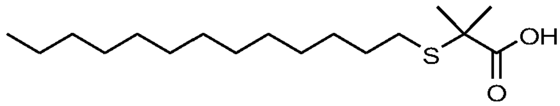

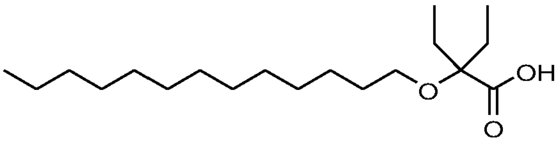

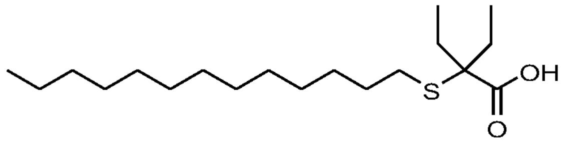

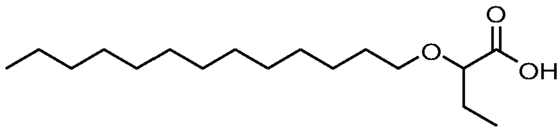

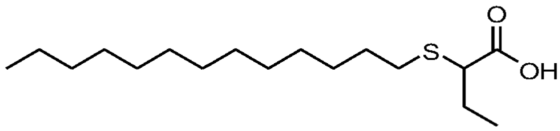

**Compounds of the invention**

Certain of the omega-6 compounds described herein are novel and these form a further aspect of the invention. Thus, in a further aspect, the present invention provides an omega-6 lipid compound of formula (II), or a pharmaceutically acceptable salt thereof:

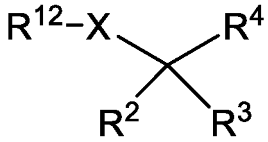
(II)

wherein

$R^{12}$ is a $C_9$ to $C_{22}$ alkenyl group having from 1 to 5 double bonds, preferably 2 to 5 double bonds, in which:

- the first double bond counting from the ω-end is at carbon 6; and
- where two or more double bonds are present, at least one pair of consecutive double bonds is interrupted by a single methylene group;

$R^2$ is selected from the group consisting of a halogen atom, a hydroxy group, an alkyl group, an alkoxy group, an alkylthio group, a carboxy group, an acyl group, an amino group, and an alkylamino group;
$R^3$ is a hydrogen atom, or a group $R^2$;
$R^4$ is a carboxylic acid or a derivative thereof; which is a carboxylic ester, and
X is methylene ($-CH_2-$), or an oxygen or sulfur atom.

Any of the omega-6 compounds described herein with reference to the medical use aspect of the invention represent preferred embodiments of the compounds of the invention. In formula (II), $R^2$, $R^3$, $R^4$ and X may thus correspond, respectively, to $R^2$, $R^3$, $R^4$ and X in any of the embodiments described above relating to the medical use of the lipid compounds. As will be understood, the group $R^{12}$ in formula (II) may correspond to any of the groups $R^1$ described herein in the case where $R^1$ is an alkenyl group and subject to the further requirements that the first double bond counting from the ω-end is at carbon 6 and that where two or more double bonds are present at least one pair of consecutive double bonds is interrupted by a single methylene group.
Preferred ω-6 lipid compounds according to the disclosure are those of formula (II) in which $R^{12}$ has 2 to 5 double bonds, e.g. 2 to 4 double bonds.
In certain embodiments, the compound of formula (II) is an omega-6 lipid compound wherein $R^{12}$ is a $C_{15}$ to $C_{20}$ alkene with 2 to 4 double bonds.
Particularly preferably, the ω-6 lipid compounds of formula (II) have 2 to 4 double bonds as described above and all of said double bonds are methylene-interrupted, i.e. successive double bonds in the alkenyl chain are separated only by $-CH_2-$ groups, preferably by no more than one $-CH_2-$ group.
Particularly preferably, where the ω-6 lipid compounds of formula (II) have 2 to 4 double bonds, these are all

in the Z-configuration.

In an embodiment, where the ω-6 lipid compounds of formula (II) have 2 to 4 double bonds, the double bonds are methylene-interrupted and are all in the Z-configuration.

The omega-6 lipid compounds of formula (II) according to the disclosure may be provided in the form of a free carboxylic acid (-COOH), or a derivative thereof, such as a carboxylic ester, a carboxylic anhydride, a carboxamide, a monoglyceride, a diglyceride, a triglyceride or a phospholipid as described above.

The omega-6 lipid compounds of formula (II) according to the invention may further be provided in the form of a pharmaceutically acceptable salt such as a lithium, sodium, potassium, ammonium, meglumine, tris(hydroxymethyl)aminomethane, diethylamine, arginine, ethylenediamine, piperazine or chitosan salt.

In a further aspect the present invention provides an omega-6 lipid compound of formula (II), or a pharmaceutically acceptable salt thereof, for use as a medicament.

In a further aspect the present invention provides an omega-6 lipid compound of formula (II), or a pharmaceutically acceptable salt thereof, for use in the prevention and/or treatment of an ophthalmic disorder.

In a further aspect the present invention provides a pharmaceutical composition comprising an omega-6 lipid compound of formula (II), together with one or more pharmaceutically acceptable carriers, excipients or diluents.

A further aspect of the present invention relates to a lipid composition comprising an omega-6 lipid compound of formula (II). The lipid composition may comprise in the range of 60 to 100% by weight of the omega-6 lipid compounds of formula (II), all percentages by weight being based on the total weight of the lipid composition. For example, at least 60%, at least 70%, at least 80%, or at least 95% by weight of the lipid composition is comprised of omega-6 lipid compounds of formula (II). The lipid composition may further comprise a pharmaceutically acceptable antioxidant, e.g. tocopherol.

The invention further provides a lipid composition comprising an omega-6 lipid compound of formula (II) for use as a medicament.

The invention further provides a lipid composition comprising an omega-6 lipid compound of formula (II) for the treatment and/or prevention of an ophthalmic disorder as herein described.

**Pharmaceutical formulations and methods of administration**

Any of the compounds herein described may be administered in the form of a pharmaceutical composition comprising said compound, together with one or more pharmaceutically acceptable carriers, excipients or diluents. Acceptable carriers, excipients and diluents for therapeutic use are well known in the art and can be selected with regard to the intended route of administration and standard pharmaceutical practice. Examples include binders, lubricants, suspending agents, coating agents, solubilising agents, preserving agents, wetting agents, emulsifiers, surfactants, sweeteners, colourants, flavouring agents, odorants, buffers, antioxidants, stabilising agents and/or salts.

The compounds described herein may be formulated with one or more conventional carriers and/or excipients according to techniques well known in the art. For example, these may be formulated in conventional oral administration forms, e.g. tablets, coated tablets, capsules, powders, granulates, solutions, dispersions, suspensions, syrups, emulsions, etc. using conventional excipients, e.g. solvents, diluents, binders, sweeteners, aromas, pH modifiers, viscosity modifiers, antioxidants, etc. Suitable excipients may include, for example, corn starch, lactose, glucose, microcrystalline cellulose, magnesium stearate, polyvinylpyrrolidone, citric acid, tartaric acid, water, ethanol, glycerol, sorbitol, polyethylene glycol, propylene glycol, cetylstearyl alcohol, carboxymethylcellulose or fatty substances such as hard fats or suitable mixtures thereof, etc.

It is envisaged that the compositions described herein will generally be administered by other conventional administration routes, e.g. topically or parenterally. Where parenteral administration is employed this may, for example, be by means of intravenous, subcutaneous, intramuscular or intraocular injection. Intravenous injection typically requires high dosages of the active to achieve efficacious drug levels within the eye and can be subject to physiological barriers to success due to the need for the active to cross the blood-retina barrier. Intraocular injection (intravitreal) is thus generally preferred. For this purpose, sterile solutions containing the active compound may be employed, such as an oil-in-water emulsion.

The use of topical administration forms, e.g. eye drops, lotions, creams, ointments, irrigants, gels, lenses, foams, sprays, tinctures, or pastes, is especially preferred since these permit delivery of the active compound directly to the eye and thus avoid side effects of systemic administration, e.g. adverse effects on the heart or liver. Such administration forms are especially advantageous due to their ease of administration and low attendant risk of infection (as can be the case with intravitreal injection, for example). Various types of carriers may be used for topical formulations, including both aqueous and non-aqueous carriers.

Any of the topical formulations described herein may further comprise at least one delivery agent that assists in the penetration of at least one surface of the eye. In certain embodiments, the delivery agent may assist in delivery of the active agent to the cornea and/or the retina of the eye. In order for a topical application to be effective in treatment

of conditions at the back of the eye, the active compound needs to be able to penetrate the surface of the eye so that it can reach the posterior segment of the eye, i.e. the retina. The penetration rate should be sufficient to impart an effective dose. Pharmaceutically acceptable drug delivery agents include any of the agents disclosed in WO 2013/049621. Examples of such agents include lecithin, D-α-tocopherol, polyethylene glycol 1000 succinate, surfactants such as Tweens and other similar polymeric delivery matrices.

Other delivery agents capable of targeting the active agent to the posterior segment of the eye include non-aqueous liquid vehicles comprising perfluorocarbons, semifluorinated alkanes, polysiloxanes or mixtures of these. Examples of such delivery agents are disclosed in US 9,241,900, EP 2444063 and US 5,874,469.

As would be understood, topical administration to the eye generally refers to localised administration to a surface of the eye, e.g. to any exterior surface of the eye normally accessible between the eyelids.

Solutions comprising the compounds described herein are particularly preferred due to the patient's ability to easily administer such compositions by means of instilling one to two drops of the solution into the affected eye(s). However, the compounds for use according to the invention may also be readily incorporated into other types of compositions, such as suspensions, emulsions, viscous or semi-viscous gels, or other types of semi-solid compositions. Suspensions or emulsions, such as oil-in-water emulsions, are preferred. The compositions may also include various other ingredients, such as buffers, preservatives, cosolvents, and/or viscosity enhancing agents. Where water is present, an appropriate buffer system (e.g., sodium phosphate, sodium acetate or sodium borate) may be added to prevent pH drift under storage conditions. Where a viscosity enhancing agent is present this typically enhances the viscosity of the ophthalmic/topical formulation to increase retention time of the solution on the eye. Viscosity enhancing agents include, among others, carbopol gels, dextran 40, dextran 70, gelatine, glycerin, polyoxyethylene-polyoxypropylene block copolymer, carboxymethylcellulose, hydroxyethyl cellulose, hydroxypropyl methylcellulose, polyethylene glycol, polysorbate 80, propylene glycol, polyvinyl alcohol and polyvinlylpyrrolodine (povidone). The desired amount of viscosity enhancing agent for use in the ophthalmic formulations can be determined by one skilled in the art.

Emulsions (e.g. microemulsions) comprising a polar phase (e.g. water), a non-polar phase (e.g. oil), and at least one surfactant represent a preferred delivery vehicle for the active compounds herein described. Oil-in-water emulsions, e.g. oil-in-water microemulsions, are particularly preferred. In microemulsions the oil phase droplets typically have a mean diameter of less than about 300 nm, e.g. between about 5 nm and about 200 nm. Such formulations are considered to be effective even in treating diseases of the posterior segment of the eye. Suitable microemulsions include those described in US 2014/0275263.

An oil-in-water emulsion (e.g. an oil-in-water microemulsion) comprising any of the lipid compounds herein described (e.g. a compound of formula (I) or a compound of formula (II)), or a pharmaceutically acceptable salt thereof, represents a further embodiment of the invention.

Cyclodextrins are useful excipients in eye drop formulations for a variety of lipophilic drugs and may be used in any of the formulations herein desrcibed. They facilitate eye drop formulations for drugs that otherwise might not be available for topical use, while improving absorption and stability and decreasing local irritation.

Lipid nanoparticles may be used as a drug delivery system for the compounds herein described in order to enhance their ocular bioavailability following topical administration. Lipid nanoparticles of mean diameter between about 50 and 400 nm are suitable for ocular administration. Their typical composition comprises physiological and biodegradable/biocompatible lipids which are suitable for the incorporation of lipophilic and hydrophilic drugs within the lipid matrix in large amounts. The matrix is stabilized with one or more surfactants in aqueous dispersion. Suitable lipids for lipid nanoparticle preparation include triglycerides, propylene glycol dicaproylcaprate, diglycerides, monoglycerides, glyceryl palmitostearate, aliphatic alcohols and fatty acids (E.B. Suoto et al, Current Eye Research, 2010, 35 (7), 537-552).

Ophthalmic products are typically packaged in multidose form. Preservatives are thus required to prevent microbial contamination during storage and use. Suitable preservatives include: benzalkonium chloride, thimerosal, chlorobutanol, methyl paraben, propyl paraben, phenylethyl alcohol, edetate disodium, sorbic acid, polyquaternium-1, or other agents known to those skilled in the art. Such preservatives are typically employed at a level of from 0.001 to 1.0% w/v.

The dosage required to achieve the desired activity of the compounds herein described will depend on various factors, such as the compound selected, its mode of administration, whether the treatment is therapeutic or prophylactic, and the nature and severity of the ocular disorder, etc. Typically, a physician will determine the actual dosage which will be most suitable for an individual subject. The specific dose level and frequency of dosage for any particular patient may be varied and will depend upon factors such as the activity of the specific compound employed, the metabolic stability and length of action of that compound, the age of the patient, the mode and time of administration, and the severity of the particular condition. The compound and/or the pharmaceutical composition may be administered in accordance with a regimen of from 1 to 10 times per day, such as once or twice per day. For oral, topical and parenteral administration to human patients, the daily dosage level of the agent may be in single or divided doses.

Suitable daily dosages of the compounds herein described are 0.1 mg to 1 g of said compound; 1 mg to 500 mg of said compound; 5 mg to 100 mg of said compound; 5 mg to 50 mg of said compound, or 10 mg to 50 mg of said

compound, or 0.1 mg to 10 mg of said compound per day. By a "daily dosage" is meant the dosage per eye per 24 hours. Where provided in a form for topical administration (e.g. instillation) directly into the eye, a suitable amount of the active compound may be in the range of 0.001 to 95 % (w/v) of the composition; a range from 0.001% to 50% (w/v) of the composition; a range from 0.005% to about 40% (w/v); a range from 0.01% to 35% (w/v), a range from 0.05% to 30% (w/v), a range from 0.1% to 25% (w/v); a range from 1% to 20% (w/v), a range from 1% to 10% (w/v), or a range from 1% to 5% (w/v). For example, when the compositions are dosed topically, they will generally be employed in a concentration range of from about 5 to about 10% w/v, with 1-2 drops administered per eye 1-4 times per day. A suitable daily dosage of the active compound for topical administration may be up to 100 mg per eye per day.

## Clinical conditions

The ophthalmic disorder to be treated or prevented by the compounds herein described may be any disease or disorder associated with the eye. It may be a disease of the anterior segment of the eye. Examples of these include cataract, corneal neovascularization, dry eye syndrome, glaucoma, keratitis and keratocornus. Alternatively, it may be a disease of the posterior segment of the eye which includes the vitreous humor, retina, retinal blood vessels, macula, choroid and optic nerve. Of particular interest are inflammatory, autoimmune, vascular and infectious diseases of the eye (e.g. those affecting the posterior segment of the eye). Examples of these include age-related macular degeneration (including dry-form AMD and wet-form AMD), diabetic retinopathy, diabetic macular edema, uveitis, retinitis pigmentosa, Stargardt's disease, retinal inflammation, ocular inflammation, corneal inflammation, retinal vascular leakage and endophthalmitis.

The compounds herein described are particularly suitable for the treatment or prevention of any ocular inflammatory disease (OID). Ocular inflammatory disease is a general term for inflammation affecting any part of the eye or surrounding tissue. Inflammation involving the eye can range from the familiar allergic conjunctivitis associated with hayfever to rare, potentially blinding conditions such as uveitis, scleritis, episcleritis, optic neuritis, keratitis, orbital pseudotumor, retinal vasculitis, and chronic conjunctivitis.

Many retinal degenerative disorders are inherited, meaning that they are due to a genetic mutation. There are many types of inherited retinal degenerations which may be treated according to the invention. Examples of these include retinitis pigmentosa, choroideremia (affects males), Leber congenital amaurosis, retinoschisis (juvenile), Stargardt's disease, Usher disease, and Bardet Biedl disease.

A number of ophthalmologic diseases have increasingly been recognised to result in part from impaired mitochondrial function, increased oxidative stress, and increased apoptosis. For example, primary mitochondrial diseases that are caused by mutations in either the nuclear genome or mitochondrial genome frequently involve clinically significant ophthalmologic disease that most commonly involves the optic nerve, retina, extraocular eye muscles, and eyelids. Amongst these diseases are the following, all of which may be treated or prevented in accordance with the invention:

Primary inherited mitochondrial diseases:

Dominant Optic Atrophy (DOA). DOA is a genetic disease that primarily affects the retinal ganglion cells (RGC) and nerve fiber layer of the retina. The prevalence of DOA is estimated at 1 in 35,000 individuals in northern Europe. Visual acuity typically decreases over the first two decades of life to a mean of 20/80 to 20/120.

Leber Hereditary Optic Neuropathy (LHON). This is characterized by acute and painless central vision loss of both eyes in a sequential fashion over a period of days to months. LHON was the first maternally-inherited ophthalmologic disorder to be linked to a point mutation in mitochondrial DNA. LHON has a recognised disease prevalence estimated at 1 in 25,000 in England and other areas of Europe.

Pigmentary retinopathy and other ophthalmologic problems. Pigmentary retinopathy is a non-specific finding that may be found in several mitochondrial diseases. The best described primary mtDNA disease in which pigmentary retinopathy may be seen is Neurogenic weakness, Ataxia, and Retinitis Pigmentosa (NARP). Pigmentary retinopathy can also occur in a range of other mtDNA cytopathies including Leigh syndrome (a degenerative disorder involving the basal ganglia and brainstem), Mitochondrial Encephalomyopathy Lactic Acidosis and Stroke (MELAS), Myoclonic Epilepsy and Ragged Red Fibers (MERRF), LHON, Kearns-Sayre Syndrome (KSS), and mitochondrial myopathy

As a high energy demand organ, the eye is particularly susceptible to the consequences of mitochondrial damage. Mitochondria are a major site of oxidative stress generation and scavenging. In addition, mitochondria are the mediators of cellular apoptosis that is initiated by the release of cytochrome c from the mitochondrial intermembrane space, where it plays an integral role in energy generation within the respiratory chain. Oxidative damage that results over time from mtDNA instability leads to cumulative mitochondrial damage, which is recognized to be an important pathogenic factor in age-related ophthalmologic disorders such as diabetic retinopathy, age-related macular degeneration, and glaucoma. This understanding has unleashed a range of emerging therapeutic approaches for mitochondrial-based ophthalmologic disorders directed at optimizing mitochondrial function (Schrier and Falk, Curr. Opin. Ophthalmol.

September 2011; 22(5): 325-331). In recent years, it has become evident that mitochondrial dysfunction, perhaps through alterations in oxidative stress balance, contribute to a wide range of common and complex ophthalmologic diseases of aging, such as diabetic retinopathy, AMD, and glaucoma. These are described in more detail below:
Age-related macular degeneration (AMD) - retinal degeneration, particularly including AMD, is responsible for a large proportion of blindness in the elderly population. Light appears to have a deleterious effect on retinal cells that already have compromised mitochondrial function. Wavelengths of light ranging from 400 to 760 nm appear to specifically affect tissues that are replete with mitochondria by reducing the activity of mitochondrial dehydrogenases and increasing the release of reactive oxygen species.

Diabetic retinopathy - this is the leading cause of blindness in young adults. The pathogenesis of diabetic retinopathy involves progressive dysfunction of retinal mitochondria in the setting of hyperglycemia, with mtDNA damage and accelerated apoptosis occurring in retinal capillary cells.

Glaucoma - this is the second-leading cause of blindness worldwide. It is an optic neuropathy that manifests with optic nerve cupping and atrophy similar to what is observed in primary mitochondrial optic neuropathies. Increasingly persuasive evidence suggests that glaucomatous tissue damage is initiated by elevated intraocular pressure and/or tissue hypoxia also involves oxidative stress. Experimental elevation of intraocular pressure induces oxidative stress in the retina. It also appears that mitochondrial oxidative stress may have an important role in glaucomatous neurodegeneration.

Uveitis - Intraocular inflammation, commonly referred to as uveitis, is a principal causative factor underlying blindness from retinal photoreceptor degeneration. Oxidative retinal damage in uveitis is caused by activated macrophages, which generate various cytotoxic agents, including inducible nitric oxide produced by inducible nitric oxide synthase, O2· and other reactive oxygen species (ROS).

Cataract - oxidative stress plays a significant role in cataractogenesis. The lens is highly susceptible to ROS, and mitochondria are located in the epithelium and superficial fiber cells. In these cell types, the mitochondria have been confirmed as the major source of ROS generation. Several *in vitro* studies have demonstrated that human lens cells are highly susceptible to oxidative insults, in which antioxidant activity is generally inversely proportional to cataract severity (see Jarrett et al., Ophthalmic Res. 2010; 44:179-190).

The compounds described herein may also be used to treat any ophthalmic diseases or disorders related to oxidative stress in any compartment of the eye, a dysregulation of the NF-κB signaling pathway or mitochondrial dysregulation and mtDNA damage such as: Stargardt's macular dystrophy, age related macular degeneration, retinal detachment, hemorrhagic retinopathy, retinitis pigmentosa, cone-rod dystrophy, Sorsby's fundus dystrophy, optic neuropathy, inflammatory retinal disease, diabetic retinopathy, diabetic maculopathy, retinal blood vessel occlusion, retinopathy of prematurity, ischemia reperfusion related retinal injury, proliferative vitreoretinopathy, retinal ischemia, retinal dystrophy, hereditary optic neuropathy, uveitis, any retinal injury, blepharitis, nya retinal disorder associated with Alzheimer's disease, any retinal disorder associated with multiple sclerosis, any retinal disorder associated with Parkinson's disease, any retinal disorder associated with a viral infection, any retinal disorder related to light overexposure, myopia, any retinal disorder associated with AIDS, macular edema, cataract, keroconjunctivitis sicca, Stevens-Johnson syndrome, Sjögrens syndrome, post-cataract surgery, dry eye, allergic conjunctivitis, neuropathic ocular pain, posterior capsular opacification and intraocular tumors.

## Preparation of compounds for use in the invention

Certain compounds of formula (I) are known in the art, or can be prepared by methods known to those skilled in the art. For example, omega-3 lipid compounds and methods for their preparation are described in WO 2010/008299, WO 2008/053331, WO 2010/128401, WO 2008/142482 and WO 2012/059818.

The novel omega-6 lipid compounds of formula (II) may be prepared from readily available starting materials using synthetic methods known in the art, for example, using methods analogous to those described in WO 2010/008299, WO 2008/053331, WO 2010/128401, WO 2008/142482 and WO 2012/059818 (see above). Suitable starting materials include natural omega-6 fatty acids such as linoleic acid (LA) ((all-Z)-9,12-octadecadienoic acid), gamma-linolenic acid (GLA) ((all-Z)-6,9,12-octadecatrienoic acid), calendic acid (8E,10E,12Z-octadecatrienoic acid), eicosadienoic acid ((all-Z)-11,14-eicosadienoic acid), dihomo-gamma-linolenic acid (DGLA) ((all-Z)-8,11,14-eicosatrienoic acid), arachidonic acid (AA) ((all-Z)-5,8,11,14-eicosatetraenoic acid), docosadienoic acid ((all-Z)-13,16-docosadienoic acid), adrenic acid ((all-Z)-7,10,13,16-docosatetraenoic acid), and docosapentaenoic acid ((all-Z)-4,7,10,13,16-docosapentaenoic acid).

Compounds of the general formula (II) where X is $-CH_2-$ may be prepared by Methods 1 to 4 as described below:

Method 1:

This method is suitable for preparing compounds of formula (II) where $R^3$ is hydrogen and $R^2$ denotes a $C_{1-6}$ alkyl group, a halogen atom, or an acyl group, and $R^4$ is a group of the formula $-COOR^5$ where $R^5$ is hydrogen or an

alkyl group:

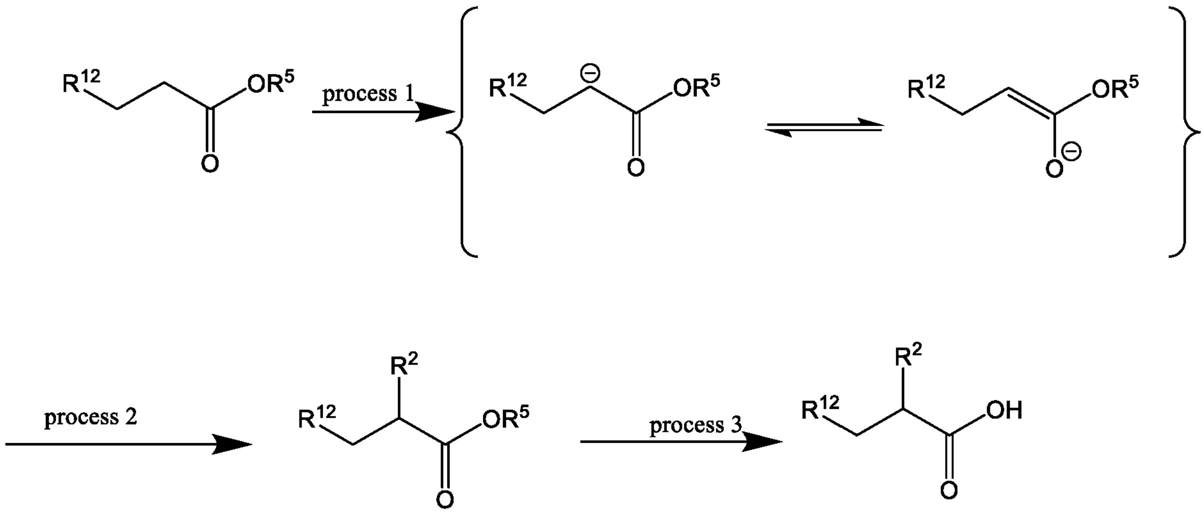


A long chain omega-6 polyunsaturated ester is reacted with a strong non-nucleophilic base (e.g. lithium diisopropylamine, potassium/sodium hexamethyldisilazide or KH/NaH/DMF) in a solvent such as tetrahydrofuran or diethylether at temperatures of -60 to -78°C, to provide the ester enolate (process 1). This ester enolate is reacted with an electrophilic reagent such as an alkylhalide (e.g. ethyliodine), an acylhalide (e.g. acetylchloride), a carboxylic anhydride (e.g. acetic anhydride) or an electrophilic halogenation reagent (e.g. N-fluorobenzene sulfonamide (NFSI), N-bromosuccinimide or iodine) to provide a mono-substituted derivative (process 2). The resulting ester is optionally further hydrolysed in a solvent such as methanol or ethanol to produce the carboxylic acid derivative by addition of a base such as lithium/sodium hydroxide in water at temperatures between 15 and 80°C (process 3).

Method 2:

This method is suitable for preparing compounds of formula II where $R^3$ is hydrogen, $R^2$ is hydroxy or an alkoxy group, and $R^4$ is a group of the formula $-COOR^5$ where $R^5$ is hydrogen or an alkyl group:

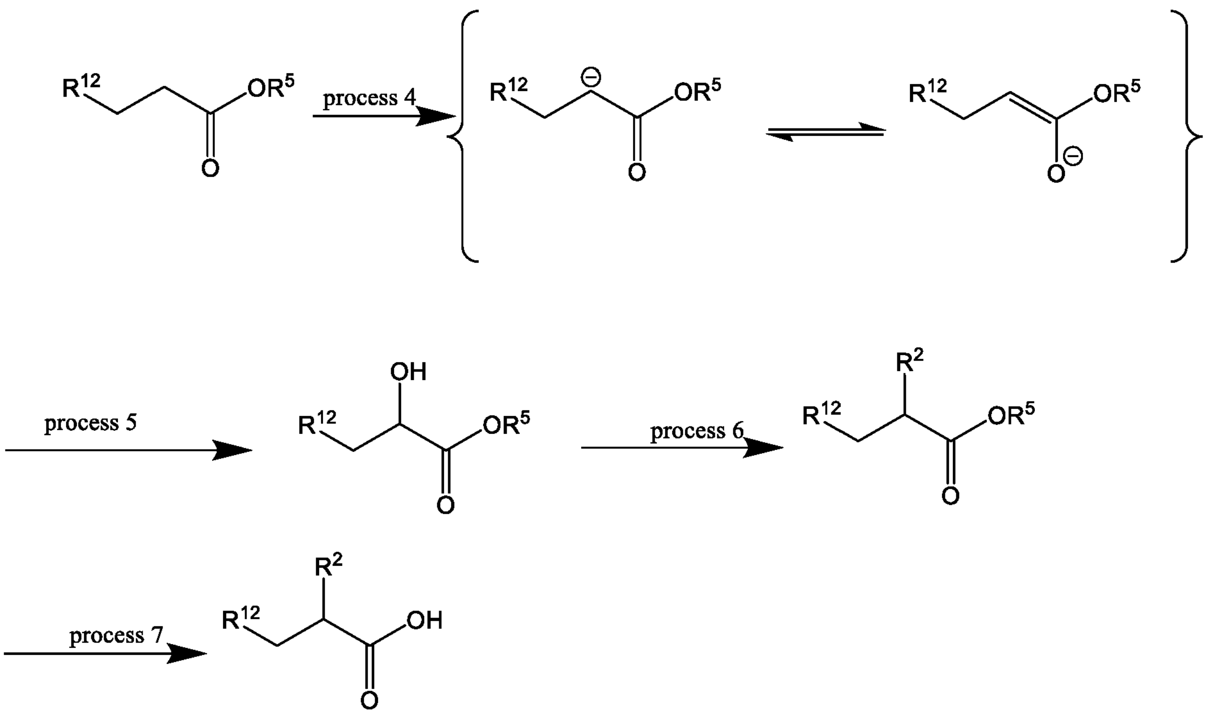

An omega-6 acid ester is reacted with a strong nucleophilic base such as lithium diisopropylamine or potassium/sodium hexamethyldisilazideane in a solvent such as tetrahydrofuran or diethyl ether at a temperature of -60 to -78°C to provide an ester enolate (process 4). The ester enolate is reacted with an oxygen source such as dimethyldioxirane, 2-(phenylsulfonyl)-3-phenyloxaziridine, or molecular oxygen, optionally with additives such as trimethylphosphite or catalysts such as a Ni(II) complex, to provide an alpha-hydroxy ester (secondary alcohol) (process 5). Reaction of the secondary alcohol with a base such as sodium hydride in a solvent such as THF or DMF generates an alkoxide which is then reacted with an electrophilic reagent such as an alkyliodide (e.g. methyl iodide or ethyl iodide) (process 6). The ester thus produced is optionally hydrolysed in a solvent such as ethanol or methanol to the carboxylic acid derivative by addition of a base such as lithium/sodium hydroxide in water at a temperature between 15 and 80°C (process 7).

Method 3:

The alpha hydroxyl esters produced in Method 2 above are useful intermediates for the introduction of other functional groups in the $\alpha$-position. For example, the hydroxyl function can be activated by conversion to a halide or tosylate prior to reaction with different nucleophiles such as ammonia, amines, thiols, etc. The Mitsunobu reaction may also be used to convert the hydroxyl group into other functional groups.

Method 4:

Compounds represented by the general formula (II) where $R^3$ is a hydrogen atom and $R^2$ denotes an alkyl, carboxyl, hydroxyl, or alkoxy group can be prepared by reacting a long chain polyunsaturated tosylate, mesylate or halide with a substituted dialkylmalonate. Hydrolysis and decarboxylation gives the desired alpha-substituted products.

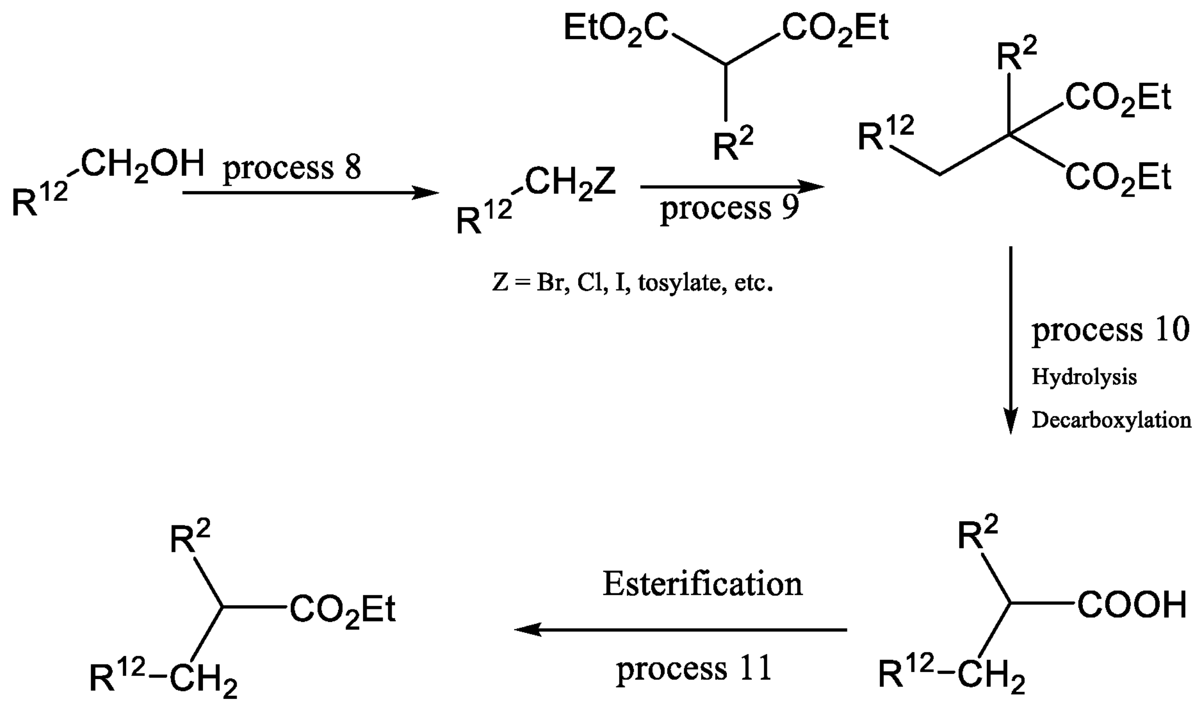


The long chain polyunsaturated tosylates used in Method 4 can be prepared from the corresponding long chain omega-6 polyunsaturated alcohol.

Compounds of general formula (II) where X is oxygen may be prepared by Methods 5 to 7 as detailed below wherein "LG" denotes a leaving group, suitably a halogen, or mesylate or tosylate group:

Method 5:

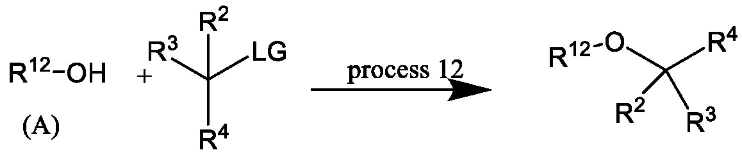


(A)

(B)

Method 6:

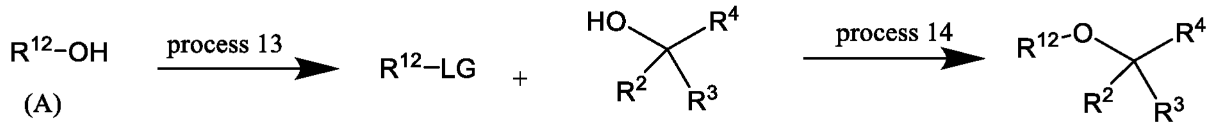


(A)

(C)

Method 7:

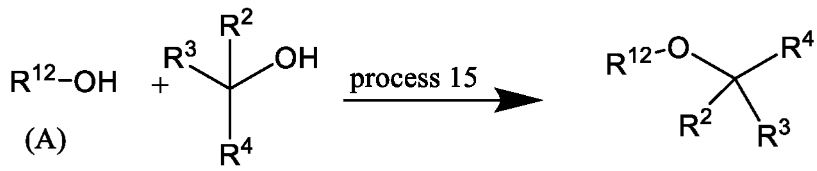


(A)

(C)

Alcohols of formula (A) used in Methods 5, 6 and 7 may be prepared directly from the carboxylic esters of, for example, naturally occurring omega-6 fatty acids by reduction with a reducing agent such as lithium aluminum hydride (LAH) or diisobutyl aluminum hydride (DIBAL-H) at -10° C to 0° C. The alcohol can also be prepared by reduction of (all-Z)-pentadeca-3,6,9-trienal made by degradation of arachidonic acid as described, for example, by Corey et al. (Tetrahedron Letters, 1983, Vol. 24, 265-268). The alcohols of formula (A) may be prepared starting from a purified omega-6 fatty acid, but it is also possible to start with a natural fatty acid mixture containing the omega-6 fatty acid. Such mixtures can come from different algae. The omega-6 alcohols can also be made by standard synthetic methods such as acetylene chemistry followed by selective hydrogenation. Several such methods are described by S. Durand et al. (J. Chem. Soc., Perkin Trans. 1, 2000, 253-273). Viala et al. (J. Org. Chem, 1988, 53, 6121) have also developed several syntheses for PUFAs using either a 3-carbon or a 6-carbon homologating agent to build up the polyene system with Wittig reactions.

Compounds of formulae (B) and (C) can be prepared by standard processes known in the art. The leaving group (LG) present in the compounds of formula (B) may, for example, be mesylate, tosylate or a suitable halogen, such as bromine, chlorine or iodine. Other suitable leaving groups will be apparent to the skilled person.

In Method 5 the alcohols of formula (A) are reacted in a substitution reaction with a compound of formula (B) in the presence of a base such as an alkali metal hydroxide, for example NaOH in an appropriate solvent system. Suitable solvent systems include a two-phase mixture of toluene and water.

In Method 6 the alcohols of formula (A) can be converted using functional group interconversion using methods familiar to a person skilled in the art to produce compounds where the terminal hydroxy group has been transformed into a suitable leaving group (process 13). Suitable leaving groups include bromine, mesylate, and tosylate, or others that will be apparent to the skilled person. These compounds can be reacted further (process 14) in a substitution reaction with the appropriately substituted hydroxy acetic acid derivatives (compounds of formula (C)), in the presence of a base in an appropriate solvent system.

In Method 7 an alcohol of formula (A) can be reacted with an appropriately substituted hydroxy acetic acid derivative (compound of formula (C)), under classic or non-classic Mitsunobu conditions, using methods familiar to persons skilled in the art.

Compounds of general formula (II) where X is sulfur may be prepared by Method 8 or Method 9 as described below wherein "LG" denotes a leaving group, suitably a halogen, or mesylate or tosylate group:

Method 8:

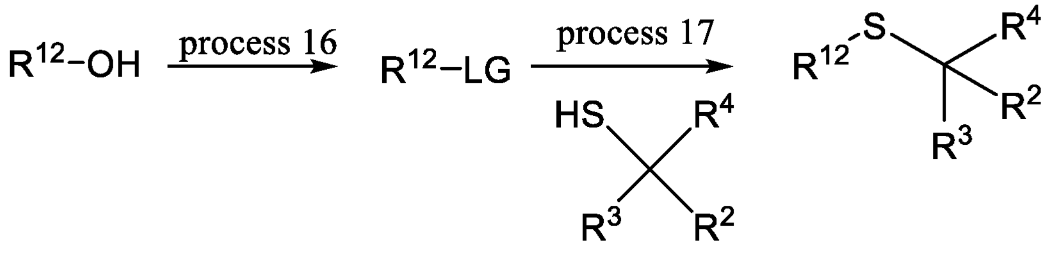


(D)

Method 9:

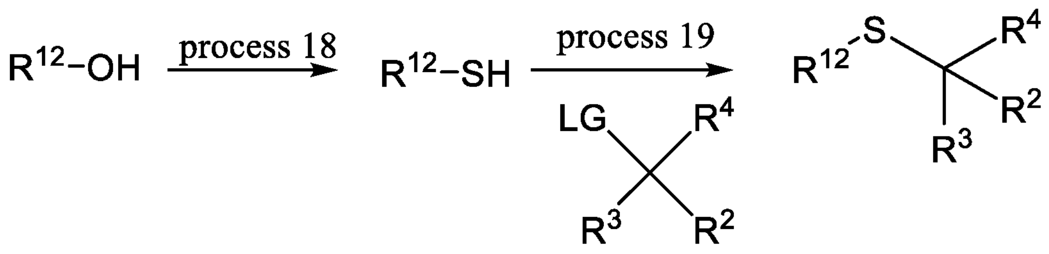


(E)

Compounds (D) and (E) are commercially available, or they are known in the literature, or they are prepared by standard processes known in the art. The leaving group (LG) present in the compounds of formula (E) may, for example, be mesylate, tosylate or a suitable halogen, such as bromine.

In Method 8 the starting alcohols $R^{12}$-OH can be converted, using functional group interconversion, by methods familiar to persons skilled in the art (process 16), to produce compounds where the terminal hydroxyl group has been transformed into a suitable leaving group (LG). Suitable leaving groups include bromine, mesylate and tosylate. These compounds can be reacted further (process 17) in a substitution reaction with the appropriate substituted thiol acetic acid derivative (D), in the presence of base.

Using Method 9, the alcohols $R^{12}$-OH can be converted to the corresponding thiols by methods familiar to persons skilled in the art (process 18). The thiols can then be reacted further (process 19) in a substitution reaction with compounds of formula (E) in the presence of base in an appropriate solvent system.

If the acid derivatives prepared in any of the methods herein described are carboxylic esters, hydrolysis can be performed to obtain the free fatty acids. An esterifying group such as a methyl or an ethyl group may be removed, for example, by alkaline hydrolysis using a base such as an alkali metal hydroxide, for example LiOH, NaOH or KOH, or by using an organic base, for example $Et_3N$ together with an inorganic salt, for example LiCl in an appropriate solvent system. A tert-butyl group may be removed, for example, by treatment with an acid, for example an organic acid such as trifluoroacetic acid or formic acid in an appropriate solvent system. Suitable solvent systems may comprise dichloromethane.

Conversion of a compound of formula (II) in the form of a carboxylic acid to a corresponding salt can be performed by suitable methods known in the art, for example, by treating it with a suitable base in an appropriate solvent system. Removal of the solvent will give the resulting salt.

As will be understood, the preparation of compounds of formula (II) according to Methods 1 to 8 may in some cases result in mixtures of stereoisomers. If required, these isomers may be separated by means of chiral resolving agents and/or by chiral column chromatography using methods known to the person skilled in the art.

As described herein, the omega-6 lipid compounds of formula (II) can also be provided in the form of derivatives,

e.g. as phospholipids, mono-, di- or tri-glycerides. Such derivatives can be prepared according to methods known in the art. Suitable methods include those described in Methods 10 to 13 below:

Method 10:

The compounds of formula (II) wherein $R^4$ is a carboxylic acid derivative in the form of a phospholipid can be prepared via this process. Acylation of sn-glycero-3-phosphocholine (GPC) with an activated fatty acid, such as fatty acid imidazolides, is a standard procedure in phosphatidylcholine synthesis. It is usually carried out in the presence of DMSO anion with DMSO as a solvent (see e.g. Hermetter, Chemistry and Physics of lipids, (1981) 28, 111).

Sn-Glycero-3-phosphocholine as a cadmium (II) adduct can also be reacted with the imidazolide activated fatty acid in the presence of DBU (1,8-diazabicyclo[5.4.0]undec-7-ene) to prepare the phosphatidylcholine of the respective fatty acid (see, for example, PCT/GB2003/002582). Enzymatic transphosphatidylation can effect the transformation of phosphatidylcholine to phosphatidyletanolamine (see e.g. Wang et al, J. Am. Chem. Soc, (1993) 115, 10487).

Phospholipids may also be prepared by enzymatic esterification and transesterification of phospholipids or enzymatic transphosphatidylation of phospholipids (see e.g. Hosokawa, J. Am. Oil Chem. Soc. 1995, 1287, and Lilja-Hallberg, Biocatalysis, (1994) 195).

Method 11:

The compounds of formula (II) wherein $R^4$ is a carboxylic acid derivative in the form of a triglyceride can be prepared through the following process. Excess of the fatty acid can be coupled to glycerol using dimethylaminopyridine (DMAP) and 2-(1H-benzotriazol-1-yl)-N,N,N',N'- tetramethyluroniumhexafluorophosphate (HBTU).

Method 12:

The compounds of formula (II) wherein $R^4$ is a carboxylic acid derivative in the form of a diglyceride can be prepared by reaction of the fatty acid (2 equivalents) with glycerol (1 equivalent) in the presence of 1,3-dicyclohexylcarbondiimide (DCC) and 4-dimethylaminopyridine (DMAP).

Method 13:

The compounds of formula (II) wherein $R^4$ is a carboxylic acid derivative in the form of a monoglyceride can be prepared through the following process. Acylation of 1,2-0-isopropylidene-sn-glycerol with a fatty acid using DCC and DMAP in chloroform gives a monodienoylglycerol. Deprotection of the isopropylidene group can be effected by treating the protected glycerol with an acid (e.g. HCl, acetic acid, etc.) (see e.g. O'Brian, J. Org. Chem., (1996) 5914).

There are several synthetic methods for the preparation of monoglycerides with the fatty acid in the 2-position. One method involves esterification of the fatty acid with glycidol in the presence of 1-(3- dimethylaminopropyl)-3-ethylcarbodiimidehydrochloride (EDC) and 4-dimethylaminopyridine (DMAP) to produce a glycidyl derivative. Treatment of the glycidyl derivative with trifluoroacetic anhydride (TFAA) prior to trans-esterification gives rise to the monoglyceride (see e.g. Parkkari et al, Bioorg. Med. Chem. Lett. (2006) 2437).

It is also possible to use enzymatic processes (lipase reactions) for the transformation of a fatty acid to a mono-, di-, tri-glyceride. A 1,3-regiospecific lipase from the fungus *Mucor miehei* can, for example, be used to produce triglycerides or diglycerides from polyunsaturated fatty acids and glycerol. The non-regiospecific yeast lipase from *Candida antartica* is also highly efficient in generating triglycerides from polyunsaturated fatty acids. (see e.g. Haraldsson, Pharmazie, (2000), 3).

The invention will now be described in more detail by way of the following non-limiting Examples and with reference to the accompanying figures, in which:

Figure 1 - illustrates the principle of cell based M1H assays in agonist mode used in Example 6;
Figure 2 - illustrates the principle of cell based M1H assays in antagonist mode used in Example 6;
Figure 3 - shows the results of the mitochondrial DNA study in Example 7;
Figure 4 - shows the effects of compounds on the activity of the NF-κB pathway using the NF-κB reporter assay in Example 22;
Figure 5 - shows the dose dependence of the inhibitory effect of dexamethasone, compound BIZ-110 and compound BIZ-101 on the NF-κB pathway;
Figure 6 - shows the PPARα activation of compounds at 5 μM concentration;
Figure 7 - shows the PPARγ activation of compounds at 5 μM concentration;
Figure 8 - shows kinetic monitoring of cytolysis in ARPE-19 cells treated with 0.1% DMSO (8A), Fenofibric acid (8B),

or BIZ-101 (8C). Figure 8D shows the dose response analysis of the tBHP cytotoxicity after 6, 24 and 96 hours; and

Figure 9 - shows kinetic monitoring of cytolysis in ARPE-19 cells treated with compound BIZ-102. Cytotoxicity is induced by 10 μM t-BHP (9A), 30 μM t-BHP (9B), and 100 μM t-BHP (9C). In Figure 9D the effects of BIZ-102 12 hrs after addition of tBHP is shown.

The NMR-spectra were recorded in $CDCl_3$, with a Bruker Avance DPX 200 or DPX 300 or DPX 400 instrument. Mass spectra were recorded at 70 eV with a Fision VG Pro spectrometer. All reactions were performed under nitrogen or argon atmosphere.

Example 1 - Preparation of ethyl (all-Z) 2-ethyl-5,8,11,14-eicosatetraenoate

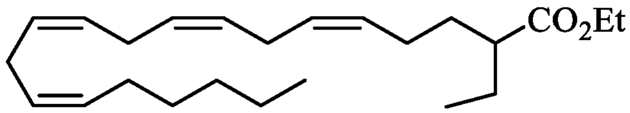

Butyl lithium (0.96 ml, 1.54 mmol in 1.6 M in hexane) was added dropwise to a stirred solution of diisopropylamine (0.23 ml, 1.6 mmol) in dry THF (5 ml) under a nitrogen atmosphere at 0°C. The resulting solution was stirred at 0°C for 20 minutes, cooled to -78°C and stirred an additional 10 minutes before dropwise addition of ethyl (all-Z)-5,8,11,14-eicosatetraenoate (466 mg, 1.4 mmol) in dry THF (5 ml). The mixture was stirred at -78°C for 10 minutes before addition of ethyl iodide (170 μl, 2.09 mmol). The mixture was allowed to warm to room temperature over 1 hour. The mixture was then poured into water, and extracted with heptane. The combined organic phase was washed with 1M HCl and then dried ($Na_2SO_4$). Filtration and evaporation under reduced pressure followed by flash chromatography (2% EtOAc in hexane) gave compound (1) as a clear oil (450 mg, 89% yield).

Example 2 - Preparation of (all-Z) 2-ethyl-5,8,11,14-eicosatetraenoic acid (BIZ 102)

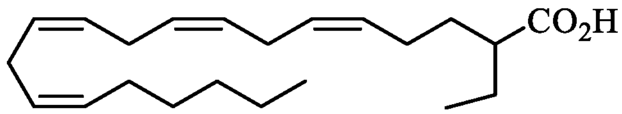

Ethyl (all-Z) 2-ethyl-5,8,12,15-eicosatetraenoate produced in Example 1 (450 mg, 1.25 mmol) was dissolved in 30 ml ethanol and a solution of LiOH (420 mg) in water (10 ml) was added. The mixture was left stirring at 80°C under an argon atmosphere for 18 hours. The mixture was cooled, then a 1M HCl solution (15 ml) was added and the mixture extracted with ether. The organic phase was washed with brine and dried ($MgSO_4$). Filtration and evaporation gave the acid as a light yellow oil (416 mg) in 100% yield.

$\delta_H$ (400 MHz, $CDCl_3$) δ 0.89 (t, 3H , J = 7.0,), 0.95 (t, 3H, J=7.4,), 1.16-1.45 (m, 6H), 1.45-1.83 (m, 4 H), 1.87-2.20 (m, 4H), 2.34 (tt, 1H, J = 8.4, 5.5), 2.62-2.96 (m, 6H ) 5.56-5.13 (m, 8H), $\delta_C$ (101 MHz, $CDCl_3$) 11.83, 14.23, 22.74, 25.21, 25.31, 25.77, 25.80, 27.38, 29.49, 31.68, 46.37, 127.72, 128.05, 128.32, 128.37, 128.72, 128.86, 129.17, 130.66, 180.83

Example 3 - Preparation of 2-ethyleicosa-(all-*Z*)-5,8,11,14,17-pentaenoic acid (α-ethyl EPA) (BIZ-101)

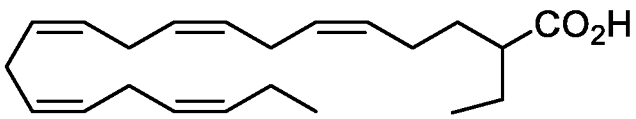

BIZ-101 was prepared based on the procedure described by Larsen et al., Biochemical Pharmacology, 1998, 405.

Butyl lithium (2.25 ml, 3.6 mmol in 1.6 M in hexane) was added dropwise to a stirred solution of diisopropylamine (594 μl, 4.2 mmol) in dry THF (5 ml) under a nitrogen atmosphere at -20°C. The resulting solution was stirred at -78°C for 45 min before dropwise addition of ethyl (all-Z)-5,8,11,14,17-eicosapentaenoate (1.0g, 3.0 mmol) in dry THF (20 ml). The mixture was stirred at -78°C for 30 minutes before addition of ethyl iodide (388μl, 4.8 mmol). The mixture was stirred at 0°C for 30 min before being poured into water (5 ml). The water phase was separated and extracted with hexane (2 × 10 ml). The combined organic phase was washed with 2M HCl (5 ml), water (2 × 5 ml) and then dried ($MgSO_4$). Filtration and evaporation under reduced pressure followed by flash chromatography (2% EtOAc in hexane) gave ethyl (all-Z)-2-ethyl-5,8,11,14,17-eicosapentaenoate as a clear oil (670 mg, 63% yield).

Ethyl (all-Z)-2-ethyl-5,8,11,14,17-eicosapentaenoate (670 mg, 1.9 mmol) was dissolved in a mixture of ethanol/THF (15 ml, 1:1) and a solution of LiOH (550 mg) in water (7.5 ml) was added. The mixture was left stirring at room temperature for 18 hours. Water and hexane were added and the organic phase was collected. The water phase was acidified with 5% HCl to pH 2 and extracted three times with hexane:ethylacetate (7:3). The organic phase was washed with water and brine and dried ($MgSO_4$). Filtration and evaporation gave the acid as a light yellow oil (429 mg) in 68% yield.

$\delta_H$ (200 MHz) δ 0.93 (t, 3H, *J* = 7.3), 0.96 (t, 3H, *J*=7.5), 1.39-1.85 (m, 4H), 1.95-2.19 (m, 4H), 2.22-2.42 (m, 1H), 2.68-2,95 (m, 8H), 5.21-5.52 (m, 10H), $\delta_C$(50 MHz):

δ 12.4, 15.0, 21.2, 25.6, 25.7, 26.1, 26.2, 31.9, 46.8, 126.4, 127.2, 127.5, 127.6, 127.9, 128.0, 128.4, 131.3, 181.6

Example 4 - Preparation of 2-methyldocosa-(all-Z)-4,7,10,13,16,19-hexaenoic acid (α-methyl DHA) (BIZ-105)

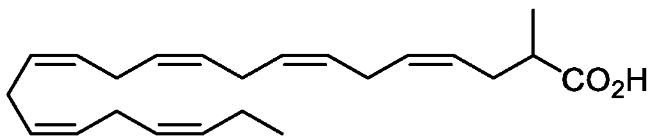

BIZ-105 was prepared based on the procedure described by Larsen et al., Lipids, Vol. 40, 2005.

Butyl lithium (1.12 ml, 1.7 mmol in 1.5 M in hexane) was added dropwise to a stirred solution of diisopropylamine (283 μl, 2.0 mmol) in dry THF (4.2 ml) under nitrogen atmosphere at -20°C. The resulting solution was stirred at -78°C for 45 min before dropwise addition of ethyl (all-Z)-4,7,10,13,16,19-docosahexaenoate (500 mg, 1.4 mmol) in dry THF (8.4 ml). The mixture was stirred at -78°C for 30 minutes before addition of methyl iodide (140μl, 4.8 mmol). The mixture was stirred at 0°C for 30 min before being poured into water (5 ml). The water phase was separated and extracted with hexane (2 × 10 ml). The combined organic phase was washed with 2M HCl (5 ml), water (2 × 5 ml) and then dried ($MgSO_4$). Filtration and evaporation under reduced pressure gave ethyl (all-Z)-2-methyl-4,7,10,13,16,19-docosahexaenoate as a clear oil (520 mg, 100% yield).

Ethyl (all-Z)-2-methyl-4,7,10,13,16,19-docosahexaenoate (520 mg, 1.4 mmol) was dissolved in a mixture of ethanol/THF (9 ml, 2:1) and a solution of LiOH (437 mg) in water (6 ml) was added. The mixture was left stirring at room temperature for 18 hours. Water and hexane were added and the organic phase was collected. The water phase was acidified with 5% HCl to pH 2 and extracted three times with hexane:ethylacetate (7:3). The organic phase was washed with water and brine and dried ($MgSO_4$). Filtration and evaporation gave the acid as a light yellow oil (390 mg) in 81% yield.

$\delta_H$ (300 MHz): 0.96 (t, 3H, J = 7.5 Hz), 1.18 (d, 3H, J = 6.8 Hz), 2.06 (m, 2H), 2.20-2.30 (m, 1H), 2.35-2.55 (m, 2H), 2.75-2.95 (m, 10 H), 5.25-5.55 (m, 12 H); δc (75 MHz) 14.25, 16.34, 20.55, 25.53, 26.63, 30.90, 39.41, 126.32, 127.01, 127.87, 127.98, 128.08, 128.11, 128.23, 128.56, 130.26, 132.03, 128.27, 182.37; m/z (CI) 343 (M+1, 1.65%), 215, 93, (100), HRMS: found M + 1 343.262563.

Example 5 - Preparation of 2-methyl-tetradecylthioacetic acid (a-methyl TTA) (BIZ-103)

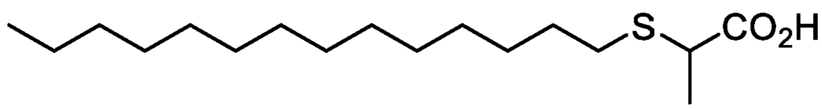

BIZ-103 was prepared based on the procedure described in EP-A-0345038 and US 2004/0192908.

Potassium hydroxide (34.30 g, 0.611 mol), 2-mercapto propionic acid (31.2 g, 0.294 mol) and 1-bromotetradecane (50 ml, 0.184 mol) were added in that order to methanol (400 ml) and stirred overnight at room temperature. A

concentrated hydrochloric acid solution (60 ml) dissolved in water (800 ml) was then added to the reaction mixture. Precipitation of 2-methyl-tetradecylthioacetic acid occurred. The mixture was stirred overnight at room temperature. The precipitate was then filtered, washed five times with water and dried. The product was recrystallized from methanol and isolated as white flakes by filtration (yield 90%). TLC gave only one spot with iodine vapor.

$\delta_H$ (400 MHz, $CDCl_3$): 0. 89 (t, 3H, J = 6.8 Hz), 1.2-1.3 (m, 20 H), 1.3-1.4 (m, 2 H), 1.46 (d, 3H, J = 7.2 Hz), 1.5-1.7 (m, 2H), 2.6-2.7 (m, 2H), 3.41 (q, 1 H, J = 7.2 Hz); $\delta$c (101 MHz, $CDCl_3$): 14.13, 16.90, 22.70, 28.88, 29.19, 29.21, 29.37, 29.50, 29.60, 29.66, 29.68, 29.70, 31.67, 31.93, 40.88, 179.27

Example 6 - PPAR$\alpha$ activity of compounds

The compounds BIZ-102 (Example 2), BIZ-103 (Example 5) and BIZ-106 were tested at the human PPARalpha receptor in a cellular GAL4 Reporter gene assay. The assay was run in agonist and antagonist mode to detect agonistic as well as antagonistic activities of the tested compounds.

*Materials and Methods:*

Compounds tested:

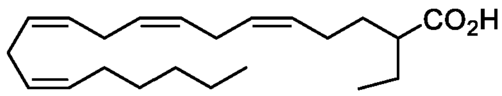

BIZ-102

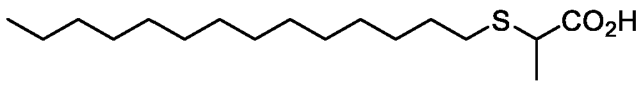

BIZ-103

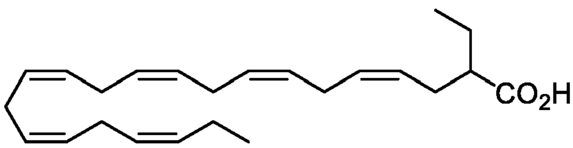

BIZ-106 ($\alpha$-ethyl DHA)

Reference compounds:

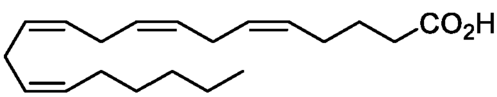

BIZ-107 (arachidonic acid)

GW7647 (a known PPAR$\alpha$ agonist)

Fenofibric acid (a known PPAR$\alpha$ agonist)

GAL4 Transactivation Assays:

Figure 1 shows the principle of the cell based M1H assays in agonist mode. Figure 2 shows the principle of the cell based M1H assays in antagonist mode.

Performance of the GAL4 cellular reporter assay:

Day 1: Cells were seeded in 96 well plates in plating medium (MEM with serum), incubation overnight at 37°C, 5% $CO_2$.

Day 2: Removal of plating medium

Addition of PEI-based transfection agent

Incubation for 4-6 hours at 37°C, 5% $CO_2$
Addition of assay medium (MEM with serum)
Addition of compounds (dilution series was generated in MEM with serum)
Incubation overnight at 37°C, 5% $CO_2$
Day 3: Removal of assay medium (16-20 hours after compound addition)
Addition of Passive Lysis Buffer (Promega)
15 mins incubation at room temperature
Addition of luciferase buffers and measurement in a dual-flash procedure
The assays were done in HEK293 cells (DSMZ ACC 305). The plasmids used in the GAL4 assay system were derivatives of Stratagene's M2H plasmids: the reporter plasmid pFR-Luc (containing a synthetic promoter with five tandem repeats of the yeast GAL4 binding sites that control expression of the Photinus pyralis (American firefly) luciferase gene), and pCMV-BD (for fusions of nuclear receptor ligand binding domains to the DNA-binding domain of the yeast protein GAL4). In order to improve experimental accuracy, a second reporter - *Renilla reniformis* luciferase, driven by a constitutive promoter - was included as an internal control. Using the control reporter (Renilla Luciferase) allowed corrections for variations in experimental handling, e.g. transfection efficacy, cell viability, pipetting errors, cell lysis efficiency and assay efficiency. For the antagonist mode experiments, the medium added after transfection contained intermediate concentrations of the reference compound GW7647 (2.5nM).

*Data Evaluation:*

Primary read out of the assays was loaded into PhAST (Phenex Assay and Screening Tool) and checked for assay quality (generation of S/B and Zprime values). These data were then loaded into the Analysis tool of PhAST to generate graphs and dose response curves. Within PhAST there are two measured and one calculated data layers:
LAYER1 (measured) - contains the activity values of the firefly luciferase activities and is a direct measure for modulation of the cofactor binding properties of the Nuclear Receptors by the tested compounds.
LAYER2 (measured) - contains the activity values of the renilla luciferase activities and is used as normalisation layer. As the renilla luciferase is expressed under control of the constitutively active CMV promoter, moderate well-to-well differences can be used to correct for variations in experimental handling.
LAYER3 (calculated) - is calculated according to the following equation:

$$1000 * \text{Firefly luciferase value} / \text{Renilla luciferase value}$$

These normalised values are, as well as LAYER1, a measure for the modulation of the cofactor binding properties of the nuclear receptors by the tested compounds.

*Results and discussion:*

Results were obtained for the 10 compound concentration triplicate assays in direct Firefly and Renilla normalized measurement mode. Dose response curves were used to determine the $EC_{50}$ values for BIZ-102 and BIZ-103 as 350 nM and 154 nM. Compared with the reference compound GW7647 the tested compounds showed an efficacy of ~150 and ~180%. Compared with fenofibric acid, the efficacies of the tested compounds are ~45 and ~55%, respectively.
The $EC_{50}$ values for BIZ-106 and BIZ-107 were determined from dose response curves to be ~5μM and ~26μM, respectively. Compared with the reference compound GW7647 the tested compounds show an efficacy of ~80 and ~100%, respectively. Compared with fenofibric acid, the efficacies of the tested compounds are -25 and ~30%, respectively.
All tested compounds showed agonistic effects at PPARα in antagonist mode. For evaluation of agonistic effects it is better to use agonist mode data as they are only influenced by the test compound. The table below shows the obtained potencies and efficacies in detail.

| Compound tested | $EC_{50}$ value | Efficacy vs. GW7647 | Efficacy vs. Fenofibric Acid |
|---|---|---|---|
| BIZ-102 | 350nM | 150% | 45% |
| BIZ-103 | 154nM | 180% | 55% |
| BIZ-106 | 5μM | 80% | 25% |
| BIZ-107 | 26 μM | 100% | 30% |

It can be seen that BIZ-102 and BIZ-103 activated PPARα at nanomolar concentrations and their efficacy was significantly better than the reference compound GW7647. Although their efficacy is not as high as fenofibric acid, this compound is not active on PPARα at nanomolar concentrations ($EC_{50}$ fenofibric acid measured as 10-18 μM).

Example 7 - PPARβ, γ and δ activity of compounds

The compounds BIZ-102 and BIZ-103 were tested at the human PPARbeta/delta and PPARgamma receptors using the same cellular GAL4 Reporter gene assays as in Example 6. The assays were run in agonist mode to detect agonistic activities of the tested compounds. Both tested compounds showed weak agonistic effects at PPARbeta/delta and PPARgamma.

The table below shows the obtained potencies and efficacies in detail.

| | **PPARbeta/delta** | | **PPARgamma** | |
|---|---|---|---|---|
| | **$EC_{50}$ value** | **efficacy vs. GW501516** | **$EC_{50}$ value** | **efficacy vs. Rosiglitazone** |
| **BIZ-102** | 4.3μM | 20% | 1.5μM | 23% |
| **BIZ-103** | 8.9μM | 18% | 1.6μM | 20% |

Example 8 - Effect on mitochondrial DNA (mtDNA)

Mitochondrial DNA damage is a useful biomarker to evaluate the potential therapeutic effect of the compounds in relation to the treatment of retinal degenerative diseases.

*Method:*

In this study, neuroblastoma cells were cultivated at near confluency (50-75%) in DMEM/F12/10% serum high glucose (20mM) supplied with 10 μM BIZ-101 or BIZ-105 for 24 hours prior to analyses. These conditions readily induce mtDNA damage that is representative of that accumulating during aging as the result of age-associated oxidative stress.

For DNA damage analyses, cells were washed, and DNA isolated using Qiagen Blood&Tissue DNA isolation kit. DNA damage level was analyzed by a RT-qPCR method based on the ability of DNA lesions to inhibit restriction enzyme cleavage, as described previously (http://www.ncbi.nlm.nih.gov/pubmed/25631007) using primers 5'-aaactgctcgccagaacact-3' and 5'-catgggctacaccttgacct-3' (sense and antisense, respectively). Briefly, genomic DNA (6 ng) was treated with 1 U TaqI for 15 min at 65°C. DNA damage frequency was calculated as 2exp-(ctTaq1-ctnt), where ctTaq1 and ctnt represent CT values of TaqI-treated and non-treated genomic DNA, respectively.

*Results:*

The results from the experiment demonstrated reduced mtDNA damage in *in vitro* cultured neuroblastoma cells upon BIZ-101 administration and a slightly reduced level of mtDNA damage by BIZ-105 (see Fig. 3; NT = no treatment). The results indicate at least the potential for BIZ-101 to be used for treatment of retinal degenerative diseases like AMD.

Example 9 - Preparation of 2-ethyl (4Z,7Z, 10Z, 13Z, 16Z, 19Z)-docosa-4,11,10,13,16,19-hexaenoic acid (BIZ 106)

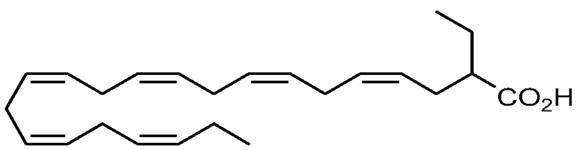

Butyl lithium (0.96 ml, 1.54 mmol in 1.6 M in hexane) was added dropwise to a stirred solution of diisopropylamine (0.23 ml, 1.6 mmol) in dry THF (5 ml) under a nitrogen atmosphere at 0°C. The resulting solution was stirred at 0°C for 20 minutes, cooled to -78°C and stirred an additional 10 minutes before dropwise addition of ethyl ethyl (4Z,7Z,10Z, 13Z,16Z,19Z)-docosa-4,11,10,13,16,19-hexaenoate (499 mg, 1.4 mmol) in dry THF (5 ml). The mixture was stirred at -78°C for 10 minutes before addition of ethyl iodide (0.16 ml, 2.09 mmol). The mixture was allowed to warm to room

temperature over 1 hour. The mixture was then poured into water, and extracted with heptane. The combined organic phase was washed with 1M HCl and then dried ($Na_2SO_4$). Filtration and evaporation under reduced pressure followed by filtration through a silica plug (hexane:EtOAC 98:2) gave the ethyl ester (330 mg):

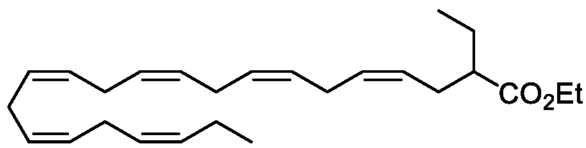

The ester (330 mg, 0.9 mmol) was dissolved in 20 ml ethanol and a solution of LiOH (320 mg) in water (10 ml) was added. The mixture was left stirring at 80°C under an argon atmosphere for 18 hours. The mixture was cooled, then a 1M HCl solution (15 ml) was added and the mixture extracted with ether and dried ($MgSO_4$). Filtration, evaporation followed by flash chromatography on silica gel (98:2 to 9:1 hexaene/ethylacetate) gave the acid as a light yellow oil (200 mg).

$\delta_H$ (400 MHz):0.97 (m, J=7.4, 6H), 1.55-1.75(m, 2H), 2.06 (m, 2H), 2.25-2.50 (m, 2H), 2.75-2.95 (m, 10H), 5.25-5.50 (m, 12 H); $\delta_C$ (100 MHz) 11.9, 14.4, 20.7, 24.8, 25.7, 25.79, 25.80, 29.5, 47.1, 126.6, 127.2, 128.0, 128.2, 128.25, 128.29, 128.38, 128.41, 128.42, 128.7, 130.2, 132.2, 181.1

Example 10 - Preparation of (5Z,8Z,11Z,14Z)-icosa-5,8,11,14-tetraenol

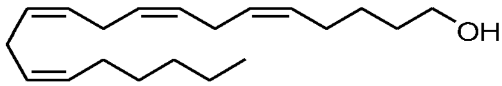

A solution of arachidonic acid ethyl ester (3.0 g, 9,0 mmol) in MTBE (15 ml) was added dropwise to a suspension of lithium aluminium hydride (0.68 g, 18 mmol) in MTBE (6 ml) at 0°C. The solution was stirred for an additional 1 hour at 0°C. Water and HCl (2M) were added and the mixture was extracted with diethylether. The combined ether extract was washed with brine and dried ($MgSO_4$). Evaporation under reduced pressure followed by dry flash on silica gel (50:50 diethyleter/EtOAC) gave the pure alcohol (2.9 g) as an oil.

$\delta_H$ (400 MHz): 0.86 (t, 3H, J = 6.9 Hz), 1.2-1.5 (m, 9H), 1.5-1.6 (m, 2H), 1.95-2.15 (m, 2H), 2.75-2.85 (m, 6H), 3.62 (t, 2 H, J=6.4), 5.2-5.5 (m, 8 H);

$\delta_C$ (100 MHz) 14.0 (2C), 22.5, 25.6 (2C), 25.7, 26.9, 27.2, 29.3, 31.5, 32.3, 62.8, 127.5, 127.9, 128.0 (2C), 128.3, 128.5, 129.8, 130.4

Example 11 - Preparation of 2-((5Z,8Z,11Z,14Z)-icosa-5,8,11,14-tetraen-1-yloxy) butanoic acid (BIZ 114)

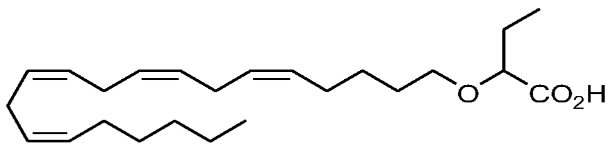

An aqueous solution of sodium hydroxide (50%, w/w, 3.5 ml) was added to a stirred solution of tetrabutylammonium bromide (133 mg, 0.41 mmol), t-butyl 2-bromobutyrate (930 mg, 4.1 mmol) and (5Z,8Z,11Z,14Z)-icosa-5,8,11,14-tetraenol (566 mg, 1.8 mmol) in toluene (5 ml) at room temperature. The resulting mixture was heated to 30-40°C and stirred for 3 hours. After cooling to room temperature, a saturated ammonium chloride ($NH_4Cl$) solution was added and the organic phase was separated. The aqueous phase was extracted with hexane (3x25ml). The combined organic layers were washed with $NH_4Cl$ solution, brine and dried ($MgSO_4$). Filtration and evaporation under reduced pressure followed by flash chromatography on silica gel (98:2 to 95:5 hexaene/ethylacetate) gave the t-butylester as a light yellow oil:

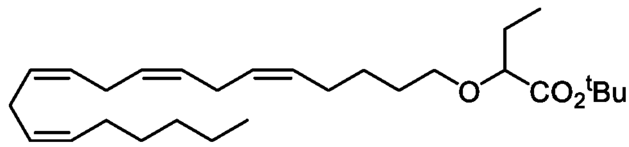

Tert-butyl 2-((5Z,8Z,11Z,14Z)-icosa-5,8,11,14-tetraen-1-yloxy)butanoate was dissolved in formic acid (95%, 6 ml) and stirred at room temperature under nitrogen atmosphere for 2.5 hours. The mixture was concentrated under vacuum and the residue purified by flash chromatography on silica gel (95:5 to 9:1 hexane/EtOAc containing 1% formic acid). Evaporation under reduced pressure gave the fatty acid (87 mg, 9%) as a light yellow oil.

$\delta_H$ (400 MHz): 0.85 (t, 3H, J = 6.9 Hz), 0.97 (t, 3H, J=7.4), 1.2-1.35 (m, 6H), 1.35-1.5 (m, 2H), 1.55-1.65 (m, 2H), 1.7-1.8 (m, 2H), 2.0-2.1 (m, 4H), 2.75-2.85 (m, 6H), 2.3-2.4 (m, 1H), 3.55-3.65 (m, 1H), 3.75-3.85 (m, 1H), 5.2-5.5 (m, 8H)

$\delta_C$ (100 MHz) 9.5, 14.1, 22.6, 25.5, 25.7, 25.9, 26.9, 27.1, 29.2, 29.3, 31.5 ,79.5, 127.4, 127.8, 128.0 (2C), 128.2, 128.4, 129.7, 130.4, 178.1.

Example 12 - Preparation of (3Z,6Z,9Z)-pentadeca-3,6,9-tetrien-1-ol

The alcohol was prepared from an algea oil containing arachidonic acid (40%) from Huatai Biopharm

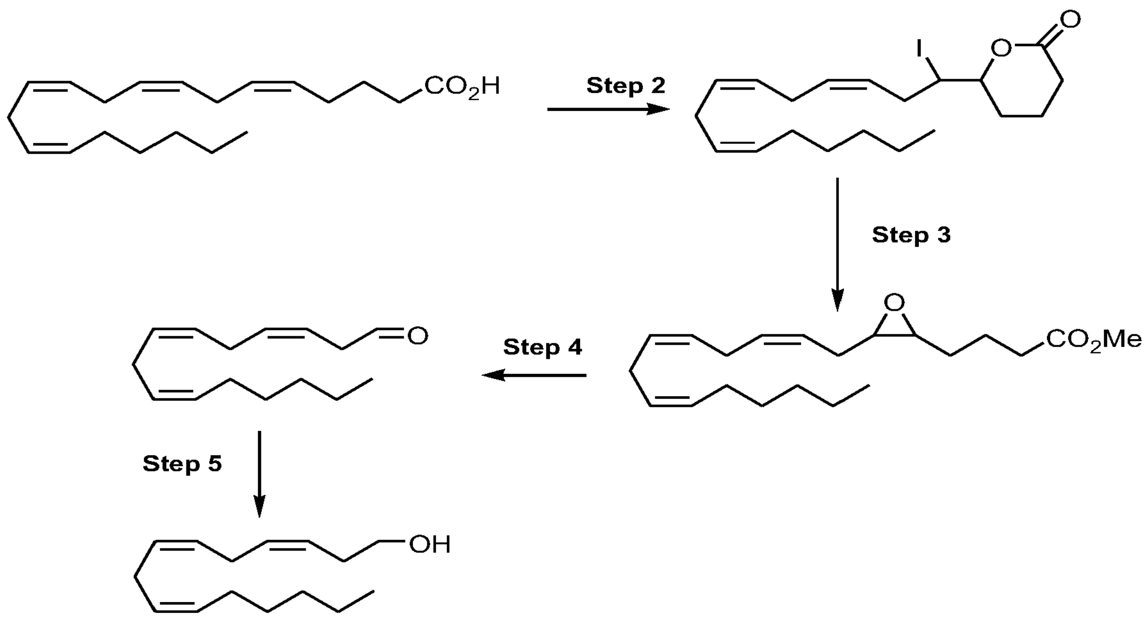


Step 1: Hydrolysis of algea oil

Algea oil (20 g) dissolved in ethanol (100 ml) was added to a stirred solution of NaOH (16 g) in water (100 ml). The mixture was heated to 60°C for 2 hrs and left stirring overnight at room temperature. Acetone (200 ml) was added to the mixture and the resulting slurry was filtrated. The filter cake was washed with acetone (2x150 ml) and the filtrate evaporated under reduced pressure. Acidification with HCl solution (5%) and extraction with a mixture of hexane:EtOAc (1:1) gave a crude arachidonic acid product (10.5 g) as an oil. The oil was dissolved in hexane and filtered through a silica plug. Evaporation of solvents under reduced pressure yielded the arachidonic acid in a mixture (approx. 40%) of a light yellow oil (5.7 g) which was used without further purification.

Step 2: Iodolactonisation

The crude arachidonic acid produced in step 1 was dissolved in ethanol (35 ml, 80%) and added to a saturated aqueous solution of $NaHCO_3$ (15 ml). An ethanoic solution (80 ml, 95%) of iodine (2.59 g ) was added dropwise under vigorous stirring within an hour. Additional iodine (2g) was added after 1.5 hrs reaction time. The mixture was left stirring overnight and $Na_2S_2O_3$ was added until the solution was colorless. The mixture was extracted with hexane (3x100 ml) and the combined organic layer washed with water, brine and dried ($MgSO_4$). Filtration and evaporation gave the crude

iodolactone (5g).

Step 3: Epoxidation

The crude iodolactone produced in step 3 was dissolved in methanol (100 ml), added to $K_2CO_3$ (2.7 g) and stirred for 4 hrs. Water was added to the mixture. The mixture was extracted with diethylether and the combined organic layers were washed with a saturated $NH_4Cl$ solution, dried ($MgSO_4$), filtrated and evaporated under reduced pressure to give the crude epoxide (4 g).

Step 4: Cleavage of epoxide [Procedure from Holmeide & Skattebøl, Journal Chemical Society, Perkin Transactions 1, 2000, 2271]

A solution of crude epoxide (2.6 g) in formic acid (50 ml) and acetic anhydride (5ml) was stirred at room temperature overnight. Volatile compounds were evaporated under reduced pressure, the residue was dissolved in methanol (65 ml) and $K_2CO_3$ (1.6 g) was added. After stirring for 3 hrs at ambient temperature water was added and the product extracted with ether. The extract was washed with water and the ether evaporated. The residue was dissolved in methanol (60 ml), cooled to 0°C and a solution of sodium periodate (2.6 g) in water (20 ml) was added. The mixture was stirred for 1.5 hrs, diluted with water and the product extracted with hexane. The extract was washed with water, dried ($MgSO_4$) and the solvents evaporated under reduced pressure giving the crude aldehyde (1.9 g).

Step 5: Reduction of aldehyde

An ice-cooled solution of crude aldehyde (1.9 g) in methanol (100 ml) was added to a solution of $NaBH_4$ (1.1 g) in methanol (30 ml). The reaction was stirred for 30 minutes before extraction with hexane (3x150 ml). The extract was washed with a saturated aqueous $NH_4Cl$ solution and water. The extract was filtered through a plug of silica using a mixture of hexane:EtOAc (95:5). The filtrate was concentrated under reduced pressure to give the alcohol (910 mg) as a colorless oil.

$\delta_H$ (400 MHz): 0.86 (t, J=6.9, 3H), 1.25-1.35 (m, 6H), 1.40 (s, 1H), 2.03 (q, J=6.8, 2H), 2.30-2.38 (m, 2H), 2.76-2.86 (m, 4H), 6.36 (bt, J=6.3, 2H), 5.23-5.43 (m, 5H), 5.49-5.57 (m, 1H) $\delta_C$ (100 MHz): 14.1, 22.6, 25.6, 25.7, 27.2, 29.3, 30.8, 31.5, 62.2, 125.6, 127.5, 127.7, 128.7, 130.5, 131.2.

Example 13 - Preparation of 2-((3Z,6Z,9Z)-pentadeca-3,6,9-tetrien-1-yloxy) butanoic acid (BIZ 111)

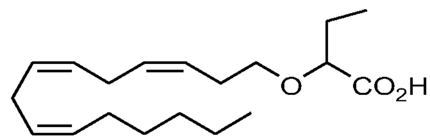

An aqueous solution of sodium hydroxide (50%, w/w, 2 ml) was added to a stirred solution of tetrabutylammonium bromide (131 mg, 0.41 mmol), t-butyl 2-bromobutyrate (923 mg, 4.1 mmol) and (3Z,6Z,9Z)-pentadeca-3,6,9-trienol as prepared in Example 12 (400 mg, 1.8 mmol) in toluene (5 ml) at 30°C. The resulting mixture was stirred for 2 hours at 30°C. After cooling to room temperature, a saturated ammonium chloride ($NH_4Cl$) solution was added and the organic phase was separated. The aqueous phase was extracted with hexane (3x25ml). The combined organic layers were washed with $NH_4Cl$ solution, brine and dried ($MgSO_4$). Filtration and evaporation under reduced pressure followed by flash chromatography on silica gel (98:2 to 9:1 hexaene/ethylacetate) afforded the ester (160 mg) containing small amounts of t-butyl-2-bromobutyrate and a pure ester (200 mg) in addition to recovery of the (3Z,6Z,9Z)-pentadeca-3,6,9-trienol (100 mg):

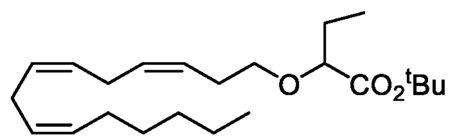

The pure fraction of the ester (200 mg) was dissolved in formic acid (95%, 3.0 mL). The reaction mixture was

left stirring at room temperature for 2 hours. The mixture was concentrated under vacuum, dissolved in hexane (60 ml) and extracted with a saturated $NaCO_3$ solution. The aqueous phase was acidified using HCl solution and extracted with EtOAc (3x25 ml). The combined organic layers were washed with brine and dried ($MgSO_4$). Filtration and evaporation under reduced pressure followed by filtration through a short plug of silica (9:1 Hexane/EtOAc+1% formic acid) afforded the title compound (130 mg) as a light yellow oil.

$\delta_H$ (400 MHz): 0.86 (t, 3H, J = 6.9 Hz), 0.98 (t, 3H, J=7.4), 1.20-1.40 (m, 6H), 1.70-1.90 (m, 2H), 2.03 (q, J=6.8, 2H), 2.35-2.45 (m, 2H), 2.75-2.85 (m, 4H), 3.45-3.50 (m, 1H), 3.55-3.65 (m, 1H), 3.83 (dd, J=6.7, J=4.9) 5.2-5.5 (m, 6 H)

$\delta_C$ (100 MHz) 9.3, 14.0, 22.5, 25.6, 25.3, 25.7, 27.2, 27.9, 29.3, 31.5, 70.3, 77.3, 125.4, 127.4, 127.6, 128.7, 130.50, 130.51, 176.9

Example 14 - Preparation of (2E,6Z,9Z,12Z)-Pentadeca-2,6,9,12-tetraen-1-ol

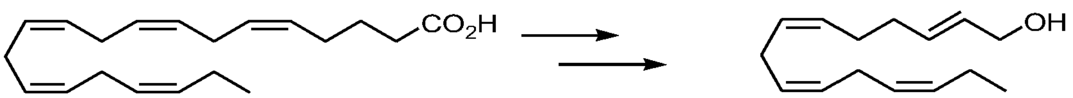

(2E,6Z,9Z,12Z)-Penradeca-2,6,9,12-tetraen-1-ol was prepared from eicosapentaenoic acid as described in the literature (see Flock et al., Acta Chemica Scandinavica, 1999, 53, 436 - Compound 24).

$\delta_H$ (400 MHz): 0.95 (t, J=7.5, 3H), 1.3 (bs,1H), 2.0-2.2 (m, 6H), 2.7-2.9 (m, 4H), 4.06 (bs, 2H), 5.20-5.45 (m, 6H), 5.6-5.7 (m, 2H)

$\delta_C$ (100 MHz): 14.2, 20.5, 25.5, 25.6, 26.8, 32.1, 63.7, 127.0, 128.0, 128.35, 128.41, 129.1, 129.4, 132.0, 132.5

Example 15 - Preparation of 2-((2E,6Z,9Z,12Z)-pentadeca-2,6,9,12-tetraen-1-yloxy) butanoic acid (BIZ 112)

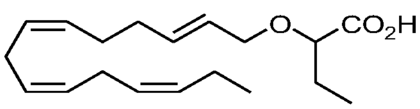

To a stirred solution of (2E,6Z,9Z,12Z)-Pentadeca-2,6,9,12-tetraen-1-ol (240 mg, 1.1 mmol), t-butyl-2-bromobutyrate (585 mg, 2.6 mmol) and tetrabutylammoniumbromide (71 mg, 0.22 mmol) in toluene (2.5 mL) at room temperature was added an aqueous solution of NaOH (1ml, 50% w/w). The mixture was stirred at room temperature for 2 hours before addition of a saturated $NH_4Cl$ solution. The organic phase was separated and the aqueous phase was extracted with hexane (3x25 ml). The combined organic phases were washed with brine water and dried ($MgSO_4$). Filtration and evaporation under reduced pressure followed by flash chromatography on silica (hexane/EtOAc, 95:5) afforded the ester (190 mg):

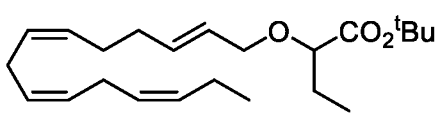

The ester (190 mg) was dissolved in formic acid (95%, 3.0 mL). The reaction mixture was left stirring at room temperature for 2 hours. The mixture was concentrated under vacuum and the residue purified by flash chromatography on silica gel (short coloumn) (95:5 to 9:1 hexane/EtOAc +1% formic acid) to give the title compound (160 mg) as a light yellow oil. $\delta_H$ (400 MHz) 0.92-1.0 (dt, J=7.5 J=7.6, 6H), 1.70-1.85 (m, 2H), 2.0-2.2 (m, 6H), 2.75-2.85 (m, 4H), 3.85-3.95 (m, 2H), 4.08 (dd, J= 6.1 J= 11.7, 1H), 5.2-5.4 (m, 6H), 5.50-5.60 (m, 1H), 5.65-5.75 (m,1H)

$\delta_C$ (100 MHz) 9.4, 14.2, 20.5, 25.5, 25.6,25.7, 26.6, 32.2, 71.3, 78.2, 125.7, 127.0, 127.9, 128.4, 128.4, 129.0, 132.0, 135.3, 177.57

Example 16 - Preparation of of 2-((5Z,8Z,11Z,14Z,17Z)-icosa-5,8,11,14,17-pentaen-1-yloxy) butanoic acid [corresponding to Example 1 in WO 2010/128401] (BIZ 110)

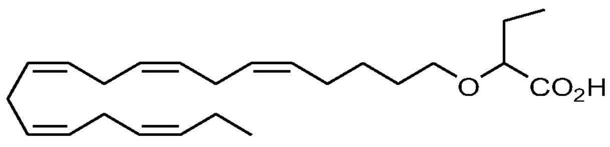

To a stirred solution of (5Z,7Z,11Z,14Z,17Z)-icosa-5,7,11,14,17-icosa-1-ol (500 mg, 1.7 mmol), t-butyl-2-bromobutyrate (758 mg, 3.4 mmol) and tetrabutylammoniumbromide (110 mg, 0.34 mmol) in toluene (5 mL) at 30°C was added an aqueous solution of NaOH (1.7 ml, 50% w/w). The mixture was stirred at 40-45°C for 2.5 hours before additional amounts of t-butyl bromobutyrate (800 mg, 3.5 mmol) and NaOH (0.8 ml, 50% w/w) were added. The mixture was stirred for an additional 1.5 hours at 40-45°C and cooled to room temperature before addition of a saturated $NH_4Cl$ solution. The organic phase was separated and the aqueous phase was extracted with hexane (3x25 ml). The combined organic phases were washed with $NH_4Cl$, brine water and dried ($MgSO_4$). Filtration and evaporation under reduced pressure followed by flash chromatography on silica (hexane/EtOAc, 97:3) afforded the ester (310 mg) containing small amounts of t-butyl-2-bromobutyrate and a pure ester (124 mg):

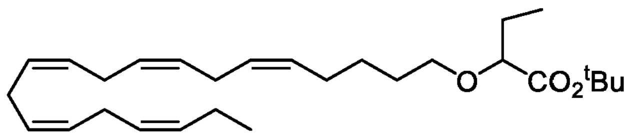

$\delta_H$ (400 MHz) 0.91-0.98 (dt, J= 7.4 J=7.5, 6H), 1.35-1.5 (m, 11 H), 1.55-1.75 (m, 4H), 2.0-2.1 (m, 4H), 2.75-2.85 (m, 8H), 3.25-3.35 (dt, J=6.6 and J=6.6, 1H), 3.55-3.65 (m, 2H), 3.75-3.85 (m, 1H) 5.2-5. 4 (m, 10H)

$\delta_C$ (100 MHz) 9.7, 14.3, 20.53, 25.51, 25.6, 26.2, 27.0, 28.1, 29.4, 70.2, 80.8, 81.0, 127.0, 127.86, 127.87, 127.9, 128.1, 128.2, 128.45, 128.52, 130.1, 132.0, 172.3

The tert-butylester (310 mg) was dissolved in formic acid (95%, 5.0 mL). The reaction mixture was left stirring at room temperature for 4 hours. The mixture was concentrated under vacuum and the residue purified by flash chromatography on silica gel (95:5 to 0:100 hexane/EtOAc) to give the title compound (120 mg) as a light yellow oil.

$\delta_H$ (400 MHz) 0.92-0.98 (dt, J= 7.4 J=7.5, 6H), 1.35-1.50 (m, 2 H), 1.55-1.70 (m, 2H), 1.70-1.90 (m, 2H), 2.0-2.15 (m, 4H), 2.75-2.85 (m, 8H), 3.41-3.48 (m, 1H), 3.54-3.62 (m, 2H), 3.82 (bt, 1H) 5.2-5. 4 (m, 10H)

$\delta_C$ (100 MHz) 9.2, 14.3, 20.5, 25.4, 25.5, 25.6, 26.0, 26.9, 29.3, 70.8, 79.7, 127.0, 127.9, 128.05, 128.10, 128.19, 128.23, 128.3, 128.6, 129.7, 132.0, 177.6

Example 17 - Preparation of 2-((4Z,7Z,10Z, 13Z,16Z,19Z)-docosa-4,7,10,13,16,19-hexaen-1-yloxy) butanoic acid [corresponding to Example 15 in WO 2010/128401] (BIZ 115)

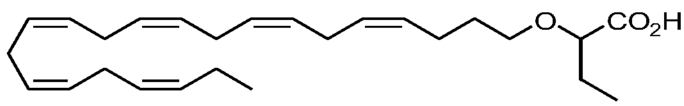

To a stirred solution of (4Z,7Z,10Z,13Z,16Z,19Z)-docosa-4,7,10,13,16,19-hexaen-1-ol (835 mg, 2.6 mmol) in toluene (7 mL) at room temperature, was added tetrabutylammonium bromide (193 mg, 0.6 mmol, 0.23 equiv.) and *t*-butyl-2-brombutyrate (1.34 g, 6.0 mmol, 2.3 equiv.). The reaction mixture was added to aq. 50% NaOH (2.45 mL). After 30 mins the mixture was added to aq. 50% NaOH (0.35 mL) dropwise. The reaction mixture was then heated to 30°C for 3 hours. After cooling to room temperature, a saturated ammonium chloride ($NH_4Cl$) solution was added and the organic phase was separated. The aqueous phase was extracted with hexane. The combined organic phases were washed with $NH_4Cl$, then brine and dried ($MgSO_4$). Filtration and evaporation under reduced pressure followed by flash chromatography on silica (hexane/EtOAc, 98:2) afforded tert-butyl-2-((4Z,7Z, 10Z, 13Z, 16Z, 19Z)-docosa-4,7, 10, 13, 16, 19-hexaen-1-yloxy)butanoate (552 mg) containing impurities of *t*-butyl-2-brombutyrate:

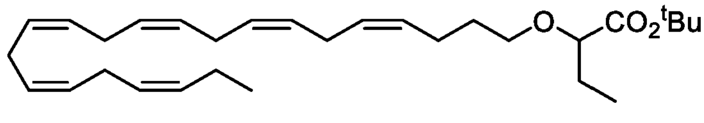

The tert-butylester (552 mg) was dissolved in formic acid (95%, 6.0 mL). The reaction mixture was left stirring at room temperature for 3 hours. The mixture was concentrated under vacuum and the residue purified by flash chromatography on silica gel (9:1 hexane/EtOAc containing 1% formic acid). Flash chromatography (hexane/EtOAc, 9:1, acidified with formic acid) eluted a mixture of product and the hydrolysed bromide. After evaporation the crude oil (291 mg) was dissolved in MTBE-ether (50 mL) and washed with sat. $NaHCO_3$ (3x25 mL), sat. $NH_4Cl$ (25 mL) and brine (25 mL) and dried ($MgSO_4$). Filtration and evaporation gave the pure acid as a colourless oil (202 mg) in 19% yield.

$\delta_H$ (400 MHz) 0.9-1.0 (m, 6H), 1.6-1.9 (m, 4H), 2.0-2.1 (m, 2H), 2.1-2.2 (m, 2H), 2.7-2.9 (m, 10H), 2.35-3.45 (m, 1H), 3.55-3.65 (m, 1H), 3.75-3.85 (m, 1H) 5.2-5.54 (m, 12H)

$\delta_C$ (100 MHz) 9.5, 14.3, 20.5, 23.6, 25.4, 25.5, 25.8, 29.4, 70.1, 79.6, 126.9, 127.7, 127.96, 127.97, 128.0, 128.07, 128.13, 128.2, 128.4, 128.5, 129.2, 132.0, 177.5.

Example 18 - Preparation of (3*Z*,6*Z*,9*Z*,12*Z*)-pentadeca-(3,6,9,12)-tetraen-1-thiol

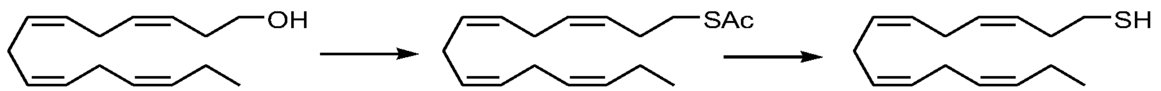

Diisopropyl azodicarboxylate (DIAD) (1.97 ml, 10.01 mmol) was added to a stirred solution of triphenylphosphine (2.75 g, 10.50 mmol) in THF (30 ml) at 0°C, and the mixture was stirred at this temperature for 30 mins. A solution of (3Z,6Z,9Z,12Z)-pentadeca-(3,6,9,12)-tetraen-1-ol (Flock et al., Acta chemical scandinavica, 1999, 53, 436) (1.8 g, 9.1 mmol) and thioacetic acid (715 µl, 10.01 mmol) in THF (10 ml) was added dropwise over 20 mins. The mixture was stirred for 1 hour at 0°C and for an additional hour at ambient temperature. The mixture was concentrated and purified by flash chromatography on silica gel (95:5 hexane:EtOAc) to give thioacetic ester (1.2 g, 47%) as an oil.

The thioester (710 mg, 2.6 mmol) was dissolved in methanol (30 ml) and added to $K_2CO_3$ (1.06 g, 7.65 mmol). The mixture was stirred at room temperature for 2 hours before addition of 1M HCl, water and diethylether. The organic phase was separated and the aqueous phase extracted with diethylether (3x30 ml). The combined organic layers were washed with brine and dried ($MgSO_4$). Filtration and evaporation gave the thiol as an oil (520 mg, 85% yield).

Example 19 - Preparation of 2-((3Z,6Z,9Z,12Z)-pentadeca-3,6,9,12-tetraenylthio)butanoic acid [corresponding to Example 9 in US 8,759,558] (BIZ 109)

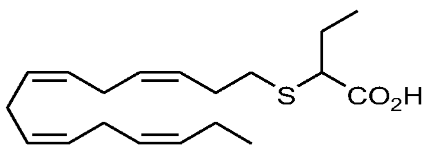

An ice-cooled solution of (3Z,6*Z*,9Z,12*Z*)-pentadeca-(3,6,9,12)-tetraen-1-thiolas prepared in Example 18 (480 mg, 2.03 mmol) in dry dimethylformamide (DMF) (10 ml) was added to NaH (89 mg, 60% in mineral oil). The mixture was stirred at 0°C for an additional 10 minutes before addition of ethyl bromobutyrate (330 µl, 2.3 mmol). The mixture was stirred at room temperature for 40 minutes, then the mixture was poured into a saturated $NH_4Cl$ solution and extracted with hexane. The extract was washed with a saturated $NH_4Cl$ solution, water and dried ($MgSO_4$). Filtration, evaporation under reduced pressure and purification by flash chromatography (hexane:EtOAC 98:2) afforded the ethyl ester (450 mg, 70%) as an oil:

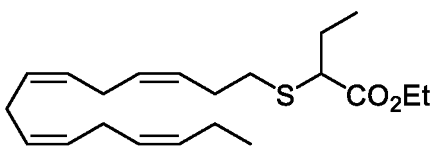

The ester (270 mg, 0.85 mmol) was dissolved in ethanol (10 ml) and added to a solution of LiOH (267 mg, 6.4 mmol) in water (10 ml). The mixture was heated at 45°C for 3 hours, cooled, added to water and 1M HCl until pH=2. The mixture was extracted with heptane (3x30 ml) and the extract was washed with brine, water and dried ($MgSO_4$).

Filtration and evaporation followed by purification by filtration through a short plug of silica gel (hexane:EtOAc 9:1) and concentration under reduced pressure afforded the acid (80 mg).

$\delta_H$ (400 MHz) 0.95 (t, J=7.5, 3H), 1.02 (t, J=7.4, 3H), 1.65-1.75 (m, 1H), 1.85-1.95 (m, 1H), 2.06 (quintett, J=7.3, 2H), 2.30-2.40 (m, 2H), 2.60-2.75 (m, 2H), 2.75-2.85 (m, 6H), 3.18 (t, J=7.5, 1H), 5.20-5.45 (m, 8H).

$\delta_C$ (100 MHz) 11.9, 14.3, 20.6, 24.48, 25.53, 25.6, 25.7, 27.1, 31.3, 48.2, 127.0, 127.5, 127.8, 127.9, 128.4, 128.6, 129.7, 132.0, 178.7

Example 20 - Preparation of 2-methyl-2-((3Z,6Z,9Z,12Z)-pentadeca-3,6,9,12-tetraenylthio)-propanoic acid (BIZ 113)

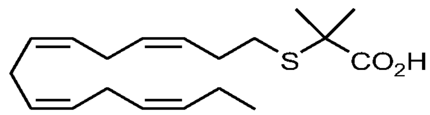

An ice-cooled solution of (3Z,6Z,9Z,12Z)-pentadeca-(3,6,9,12)-tetraen-1-thiol(520 mg, 2.2 mmol) in dry dimethylformamide (DMF) (10 ml) was added to NaH (97 mg, 60% in mineral oil). The mixture was stirred at 0°C for an additional 10 minutes before addition of 2-bromoisobutyrate (392 µl, 2.6 mmol). The mixture was stirred at room temperature for 40 minutes, then the mixture was poured into a saturated $NH_4Cl$ solution and extracted with hexane. The extract was washed with a saturated $NH_4Cl$ solution, water and dried ($MgSO_4$). Filtration, evaporation under reduced pressure and purification by flash chromatography (hexane:EtOAC 98:2) afforded the ester (270 mg) as an oil:

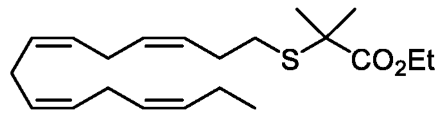

The ester (270 mg) was dissolved in ethanol (10 ml) and added to a solution of LiOH (270 mg, 6.4 mmol) in water (10 ml). The mixture was heated at 65°C for 4 hours, cooled, added to water and 1M HCl until pH=2. The mixture was extracted with hexane (3x30 ml) and the extract was washed with brine, water and dried ($MgSO_4$). Filtration and evaporation followed by purification by filtration through a short plug of silica gel (hexane:EtOAc 9:1) and concentration under reduced pressure afforded the acid (200 mg).

$\delta_H$ (400 MHz) 0.95 (t, J=7.5, 3H), 1.51 (s, 6H), 2.06 (quintet, J=7.3, 2H), 2.32 (quartet, J=7.0, 2H), 2.68 (t, J=7.3, 2H), 2.75-2.85 (m, 6H), 5.20-5-45 (m, 8H)

$\delta_C$ (100 MHz) 14.3, 20.5, 25.4, 25.5, 25.6, 25.7, 26.9, 27.7, 46.6, 127.0, 127.6, 127.8, 127.8, 127.9, 128.4, 128.6, 129.6, 132.0, 180.3

Example 21 - 3-oxa-(6Z,9Z,12Z,15Z,18Z)-heneicosa-(6,9,12,15,18)-pentaenoic acid (BIZ 108)

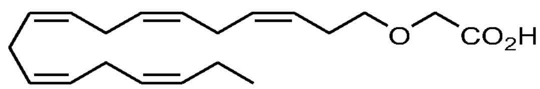

3-oxa-(6Z,9Z,12Z,15Z,18Z)-heneicosa-(6,9,12,15,18)-pentaenoic acid was prepared as described in the literature (see Flock et al., Acta Chemica Scandinavica, 1999, 53, 436 - Compound 21b)

$\delta_H$ (400 MHz) 0.95 (t, J=7.5, 3H), 2.05 (quintet, J=7.4, 2H), 2.40 (q, J=6.8, 2H), 2.7-2.9 (m, 8H), 3.56 (t, J=6.8, 2H), 4.14 (s, 2H), 5.20-5.60 (m, 10 H)

$\delta_C$ (100 MHz) 14.2, 20.5, 25.5, 25.6, 25.6, 25.7, 27.7, 67.7, 71.4, 125.2, 127.0, 127.8, 127.9, 128.0, 128.26, 128.31, 128.5, 130.4, 132.0, 175.0

Example 22 - Measuring the effects of compounds on the activity of NF-κB pathway using NF-kB reporter assay

**Background:** Corticosteroids like dexamethasone have for many years been used for treatment of a broad spectrum of inflammatory conditions of the eye due to their potent anti-inflammatory effect. However, steroids may cause severe side effects like cataract and raised intraocular pressure after prolonged use, which limits their therapeutic use

in chronic diseases. Corticosteroids exert their anti-inflammatory effects through influencing multiple signal transduction pathways. Inhibition of the NF-kB pathway is central in their anti-inflammatory effect.

NF-kB (Nuclear Factor-Kappa B, NF-kB) is a heterodimeric protein composed of different combinations of members of the Rel family of transcription factors. The NF-kB /Rel family of transcription factors (p50, p65, c-Rel, etc.) are involved in stress, immune, and inflammatory responses. In unstimulated cells, the NF-kB dimers are sequestered in the cytoplasm by inhibitory IkB proteins. Proinflammatory cytokines such as TNF-α, LPS, growth factors, and antigen receptors activate I B kinase (IKK), which phosphorylates the IkB proteins. Phosphorylation of IkB leads to its degradation, freeing NF-kB complexes to translocate to the nucleus, bind to NF-kB DNA response elements, and induce the transcription of the target genes.

**Description of assay:** The NF-kB reporter (luc)-HEK293 cell line is designed for monitoring the nuclear factor Kappa B (NF-kB) signal transduction pathways. It contains a firefly luciferase gene driven by four copies of NF-kB response element located upstream of the minimal TATA promoter. After activation by pro-inflammatory cytokines or stimulants of lymphokine receptors, endogenous NF-kB transcription factors bind to the DNA response elements, inducing transcription of the luciferase reporter gene.

**Cell Culture:** NF-kB Reporter-HEK293 cells were cultured in MEM medium with 10% FBS, 1% non-essential amino acids, 1mM Na-pyruvate, 1% Penn-strep, and 100 μg/ml of Hygromycin B.

**Assay Conditions:** To perform the NF-kB luciferase reporter assay, NF-kB Reporter-HEK293 cells were seeded at 40,000 cells per well into white clear-bottom 96-well microplate in 45 μl of growth medium without Hygromycin B. Cells were incubated at 37°C and 5% $CO_2$ overnight to allow them to recover and reattach. The following day a series of dilutions of compounds were made in assay medium (MEM medium with 0.5% FBS, 1% non-essential amino acids, 1mM Na-pyruvate, 1% Penn-strep). The medium was removed from the wells and 45 μl of diluted compound was added to the cells. Assay medium with DMSO was added to the untreated control wells and cell-free control wells. The final concentration of DMSO was 0.25%. The cells were incubated overnight at 37°C in CO2 incubator. The next day 5 μl of assay medium with TNFα was added to the wells. The final concentration of TNFa was 5ng/ml. Cells were treated for 5 hours at 37°C in a CO2 incubator.

After treatment, cells were then lysed and a luciferase assay was performed using the ONE-Step luciferase assay system. In brief, 50 μl of One-Step Luciferase reagent was added per well and the plate rocked at room temperature for 30 minutes. Luminescence was measured using a luminometer (BioTek Synergy™ 2 microplate reader).

**Data Analysis:** Reporter assays were performed in triplicate at each concentration. The luminescence intensity data were analyzed using the computer software, Graphpad Prism. In the absence of the compound, the luminescence intensity (Lt) in each data set was defined as 100%. In the absence of cells, the luminescence intensity ($L_b$) in each data set was defined as 0%. The percent luminescence in the presence of each compound was calculated according to the following equation: % Luminescence = $(L-L_b)/(L_t-L_b)$, where L= the luminescence intensity in the presence of the compound, $L_b$= the luminescence intensity in the absence of cells, and $L_t$ = the luminescence intensity in the absence of the compound. The values of % luminescence versus a series of compound concentrations were then plotted using non-linear regression analysis of a Sigmoidal dose-response curve generated with the equation $Y=B+(T-B)/1+10^{((LogEC50-X)\times \text{Hill Slope})}$, where Y=percent luminescence, B=minimum percent luminescence, T=maximum percent luminescence, X=logarithm of compound and Hill Slope=slope factor or Hill coefficient. The IC50 value was determined by the concentration causing a half-maximal percent activity.

**Results:** The results from the NF-kB assay clearly show that the tested compounds at 10 μM concentration have a surprisingly potent inhibitory effect of the TNF-α activated NF-κB pathway as shown in Figure 4. The compounds are more potent inhibitors of the NF-κB pathway than dexamethasone.

The inhibitory effect of the tested compounds BIZ-110 and BIZ-101 on the Nf-κB pathway is dose dependent as seen in Figure 5. In fact the Nf-κB pathway can be completely inhibited at high concentration using these two compounds which is not the case with dexamethasone.

Example 23 - Screening of PPAR activity

To screen the human PPARα, PPARδ and PPARγ ligand activity of the compounds a stable reporter cell line was used (HeLa cell line). The stable reporter cell line express respectively a chimeric protein containing the ligand binding domain (LBD) of human PPARα, human PPARδ and human PPARγ fused to the yeast transactivator GAL4 DNA binding domain (DBD). The luciferase (Luc) reporter gene is driven by a pentamer of the GAL4 recognition sequence in front of a β-globin promoter. The use of GAL4-PPARα, GAL4-PPARδ and GAL4-PPARγ chimeric receptors allows for elimination of background activity from endogenous receptors and quantification of relative activity across the three PPAR subtypes with the same reporter gene. The PPAR selectivity of the samples is determined by comparison to known drug references (GW7647) for PPARα, L-165041 for PPARδ and BRL49653 for PPARγ and a negative control (0.1 % DMSO). Luciferase activity was measured by a luminometer and luciferase activity was expressed as relative light units (RLU).

Day 1: Seed 96 - well plate with PPAR cells. Cell appearance was checked using optical microscopy. Cells were cultivated at confluency (80-100%).

Day 2: The test articles dissolved in DMSO were added to the cells. The controls (positive controls and the solvent control) were included in each individual plate. The cells were incubated for an additional 24 hrs after addition of test articles before analysis.

Day 3: To determine the PPAR subtype activity of the tested compounds, the percentage of PPAR ligand activity was calculated for each tested compound as follows:

Percentage of PPARα activity for the tested compound in 5 μM concentration = $(RLUX_{comp} \times 100)/RLU_{GW7647}$ where $RLU_{GW7647}$ is the luminescence measured from PPARα cells incubated with 1 μM GW7647 and expressed as Relative Light Units. The activity of 1 μM GW7647 is set to 100%

Percentage of PPARδ activity for the tested compound in 5 μM concentration = $(RLUX_{comp} \times 100)/RLU_{L165041}$ where $RLU_{L165041}$ is the luminescence measured from PPARδ cells incubated with 1 μM L and expressed as Relative Light Units. The activity of 1 μM L165041 is set to 100%

Percentage of PPARγ activity for the tested compound in 5 μM concentration = $(RLUX_{comp} \times 100)/RLU_{BRL49653}$ where $RLU_{BRL49653}$ is the luminescence measured from PPARγ cells incubated with 1 μM L and expressed as Relative Light Units. The activity of 1 μM BRL49653 is set to 100%

**Results:** The compounds tested in this assay were: BIZ-101, BIZ-102, BIZ-108, BIZ-109, BIZ-111, BIZ-112 and BIZ-113. The results showed that the tested compounds had no activity on PPARδ (results are not included). However, all compounds except BIZ-108 had potent activity on PPARα (Figure 6) with response at the same level as GW7647. The results also show that most of the compounds tested activated PPARγ except for the unsubstituted derivative BIZ-108 (Figure 7). The PPARγ activation is more potent in this stable reporter HeLA cell line than in the transient transfected HEK293 cell line described in Examples 6 and 7. Several differences in the assays can help to explain these results. BIZ-108 is unsubstituted in the α-position and was only added for comparison purposes. The results clearly show that a potent PPARα and PPARγ activation requires one or two substituents in the α-position of fatty acid derivatives.

Example 24 - Assay for measuring effects of compound BIZ-101 during conditions of oxidative stress in a retinal pigment epithelial cell line (ARPE-19)

In order to evaluate the potential of BIZ-101 to protective the eye from oxidative stress damage the compound was tested in a cell based assay. Oxidative stress was induced in an ARPE-19 cell line using tert-butyl hydroperoxide at different concentrations. The cellular viability was kinetically monitored and measured by analysing the plasma membrane integrity based on the incorporation of a non-permeant and fluorescent DNA intercalating agent that selectively stain cytolytic cells with comprised plasma membranes.

**Procedure:** The ARPE-19 cell line was seeded and cultured in DMEM-F12 medium + 10% SVF for 24 hrs in a 96 well plate. Cells were pre-treated or not with either fenofibric acid (25 μM) or compound BIZ-101 (10 μM) during this 24 hr period. After 24 hrs, the different concentrations of t-butyl hydroperoxide were added in the presence of a fluorescent DNA intercalating agent for the following monitoring. Live content time-lapse imaging was performed with a sampling rate of 1 image every 2 hr over a 96 hrs period. The number of fluorescent/cytolytic cells were counted and reported during the experiment. The treatment conditions were tested in one experiment session in a triplicate format.

**Handling and solubilisation of compounds:** Fenofibric acid was used at 25 μM and was ordered from Sigma Aldrich. The day of the pre-treatment, a 25mM stock solution of fenofibric acid was prepared in DMSO and diluted in the complete culture medium at 25 μM. 100 μL of this solution or 100 μL of complete culture medium + 0.1% DMSO replaced the 100 μL of the complete culture medium already present in the well. The day of the treatment with t-butyl hydroperoxide, a 2 times concentrated solution of fenofibric acid was freshly diluted in the complete culture medium and 50 μL of this solution or 50 μL of complete culture medium + 0.2% DMSO were added to the 100 μL of the culture medium already present in the well.

Compound BIZ-101 was tested at a final concentration of 10 μM. A 10 mM predilution was prepared in DMSO from a 10 mM stock solution. The 10 mM solution of BIZ 101 was diluted in the complete culture medium at 10 μM. 100 μL of this solution or 100 μL of the complete culture medium + 0.1% DMSO replaced the 100 μL of the complete culture medium already present in the well. The day of the treatment with t-butyl hydroperoxide, a 2 times concentrated solution of BIZ 101 was freshly diluted in the complete culture medium and 50 μL of this solution or 50 μL of complete culture medium + 0.2% DMSO were added to the 100 μL of the culture medium already present in the well.

Preparation of the tert-butyl hydroperoxide [tBHP] solution: 0, 0.01 mM, 0.03 mM, 0.1 mM, 0.3 mM, 1 mM, 3 mM and 10mM. A 4 times concentrated solution of tBHP was freshly diluted in the complete culture medium for each of the concentrations to be tested. 50 μL of the 4 times concentrated solution was added to the 150 μL of the cultured medium.

**Results:** The addition of tBHP induced a rapid, severe and dose dependent cytotoxic effect on the ARPE-19 cells as shown in Figure 8A. A dose-dependent effect could be observed and 24 hrs after addition of tBHP the complete cytolysis of ARPE-19 cells was induced even with the lowest concentration of tBHP (Figure 8A and 8D).

Pre-treatment with either fenofibric acid or BIZ-101 slightly decreased the dose dependent cytotoxicity of tBHP 6 hrs after addition of tBHP. The protective effects of fenofibric acid and BIZ-101 were more efficient 24 hrs after addition of t-BHP, and remained stable until the end of the monitoring time (Figure 8B, 8C, 8D).

The study clearly shows that BIZ-101 has a protective effect on retinal pigment epithelial cells (ARPE-19 cells) during conditions of oxidative stress. The effect is much more potent than the effect seen for fenofibric acid which is known to have therapeutic effects in treatment of diabetic retinopathy.

Example 25 - Assay for measuring effects of compound BIZ-102 during conditions of oxidative stress in a retinal pigment epithelial cell line (ARPE-19)

In order to evaluate the potential of BIZ-102 to protect the eye from oxidative stress damage the compound was tested in a cell based assay similar to Example 24 with the following changes. The oxidative stress in the ARPE-19 cell line was induced by using 3 different concentrations of tBHP: 10 μM, 30 μM and 100 μM and BIZ-102 was tested in 2 different concentrations: 1 μM and 10 μM.

**Procedure:** The ARPE-19 cell line was seeded and cultured in DMEM-F12 medium + 10% SVF for 24 hrs in a 96 well plate. Cells were pre-treated or not with compound BIZ-102 (1 μM and 10 μM) during this 24 hr period. After 24 hrs, the different concentrations of t-butyl hydroperoxide were added in the presence of a fluorescent DNA intercalating agent for the following monitoring. Live content time-lapse imaging was performed with a sampling rate of 1 image every 2 hr over a 96 hrs period. The number of fluorescent/cytolytic cells were counted and reported during the experiment. The treatment conditions were tested in one experiment session in a triplicate format.

Compound BIZ-102 was tested at a final concentration of 1 and 10 μM. A 10 mM predilution was prepared in DMSO from a 20 mM stock solution. The day of the pre-treatment with BIZ-102 and the day of the treatment with t-BHP, a 1000 times concentrated solution of BIZ-102 for each concentration to be tested was prepared in DMSO. The day of the pre-treatment, a one time concentrated solution for each concentration to be tested was prepared by diluting the 1000 times concentrated solution in complete culture medium. 100 μL of those solutions replaced the 100 μL of the culture medium already present in the well. The day of the treatment with t-BHP, for each concentration to be tested, a 2 times concentrated solution was freshly diluted in the complete culture medium and 50μL of this solution was added on the 100 μL of the complete culture medium already present in the well.

**Results:** The results show that BIZ-102 at the highest concentration (10 μM) exhibited a significant inhibition of the cytotoxicity induced by both 10 μM and 30 μM of t-BHP but not for the highest concentration of t-BHP (Figure 9A, 9B, 9C). The protective effect remained stable until the end of the monitoring time. In Figure 9D the effects of BIZ-102 12 hrs after addition of tBHP is shown. The study clearly shows that BIZ-102 has a protective effect on retinal pigment epithelial cells (ARPE-19 cells) during conditions of oxidative stress.

## Claims

1. A lipid compound of formula (I), or a pharmaceutically acceptable salt thereof, for the use in prevention and/or treatment of an ophthalmic disorder:

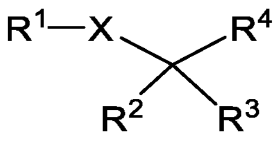

(I)

wherein

$R^1$ is a $C_9$ to $C_{22}$ alkenyl group having from 1 to 6 double bonds;
$R^2$ is selected from the group consisting of a halogen atom, a hydroxyl group, an alkyl group, an alkoxy group, an alkylthio group, a carboxy group, an acyl group, an amino group, and an alkylamino group;
$R^3$ is a hydrogen atom, or a group $R^2$;
$R^4$ is a carboxylic acid or a derivative thereof which is a carboxylic ester; and
X is methylene ($-CH_2-$), or an oxygen or sulfur atom.

**2.** A lipid compound for use as claimed in claim 1, wherein said ophthalmic disorder is:

(i) selected from cataracts, corneal neovascularization, dry eye syndrome, glaucoma, keratocornus, dry eye disease, and Sjøgren's syndrome;
(ii) an ocular inflammatory disease (OID), for example an ocular inflammatory disease which is allergic conjunctivitis, hayfever, uveitis (e.g. anterior uveitis, intermediate uveitis, posterior uveitis or panuveitis uveitis), scleritis, episcleritis, endophthalmitis, optic neuritis, keratitis, orbital pseudotumor, retinal vasculitis, or chronic conjunctivitis;
(iii) an inflammatory, autoimmune, vascular and/or infectious disease of the posterior segment of the eye;
(iv) a retinal degenerative disease, for example age-related macular degeneration (including dry-form AMD and wet-form AMD), diabetic retinopathy, diabetic macular edema, retinitis pigmentosa, Stargardt's disease, Usher syndrome, Bardet-Biedl syndrome, or retinopathy of prematurity, preferably diabetic retinopathy or age-related macular degeneration (AMD).

**3.** A lipid compound for use as claimed in claim 1 or claim 2, wherein in formula (I), one or more of the following apply:

(i) $R^2$ and/or $R^3$ is an alkyl group, preferably a straight-chained or branched $C_{1-6}$ alkyl (e.g. $C_{1-3}$ alkyl), e.g. methyl or ethyl;
(ii) $R^3$ is a hydrogen atom; and
(iii) $R^1$ is a straight-chained $C_9$ to $C_{22}$ alkenyl group.

**4.** A lipid compound for use as claimed in any one of claims 1 to 3, wherein

(i) $R^1$ is a $C_{10}$ to $C_{22}$ alkenyl group, more preferably a $C_{12}$ to $C_{20}$ alkenyl group, e.g. a $C_{20}$ alkenyl group, more preferably a $C_{14}$ to $C_{18}$ alkenyl group, preferably a $C_{15}$ or $C_{17}$ alkenyl group; or
(ii) $R^1$ is a straight-chained $\omega$-3 $C_9$ to $C_{22}$ alkenyl group having from 1 to 6 double bonds, or a straight-chained $\omega$-6 $C_9$ to $C_{22}$ alkenyl group having from 1 to 5 double bonds.

**5.** A lipid compound for use as claimed in any one of the preceding claims, wherein:

(i) at least two double bonds are present in group $R^1$, preferably wherein at least one pair of successive double bonds is interrupted by no more than one methylene group, e.g. wherein each pair of consecutive double bonds is interrupted by no more than one methylene group; and/or
(ii) all double bonds present in group $R^1$ are either in the E- or Z-configuration, preferably wherein all double bonds are in the Z-configuration.

**6.** A lipid compound for use as claimed in any one of the preceding claims, wherein in formula (I):

(i) X is $-CH_2-$ and $R^1$ is a $C_9$ to $C_{22}$ alkenyl having 2 to 6 double bonds, e.g. a $C_{19}$, $C_{17}$, or $C_{15}$ alkenyl having 6, 5, 4 or 3 double bonds; or
(ii) X is S or O and $R^1$ is a $C_9$ to $C_{22}$ alkenyl having 2 to 6 double bonds, e.g. a $C_{22}$, $C_{20}$, $C_{18}$, or $C_{15}$ alkenyl having 6, 5, 4 or 3 double bonds.

**7.** A lipid compound for use as claimed in any one of the preceding claims, wherein said compound is derived from an $\omega$-3 PUFA or an $\omega$-6 PUFA, e.g. derived from (all-Z)-4,7,10,13,16,19-docosahexaenoic acid, (all-Z)-5,8,11,14,17-eicosapentaenoic acid, (all-Z)-7,10,13,16,19-docosapentaenoic acid, (all-Z)-9,12,15-octadecatrienoic acid, (all-Z)-5,8,11,14-icosatetraenoic acid, (all-Z)-4,7,10,13,16-docosapentaenoic acid, (all-Z)-8,11,14-eicosatrienoic acid, or (all-Z)-9,12-octadecadienoic acid.

**8.** A lipid compound for use as claimed in claim 1 or claim 2, wherein said compound is selected from any of the following, or their pharmaceutically acceptable salts:

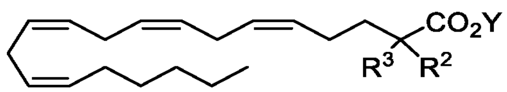

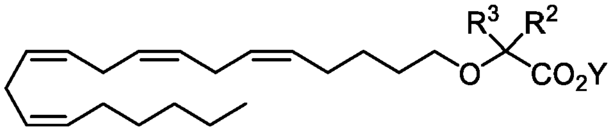

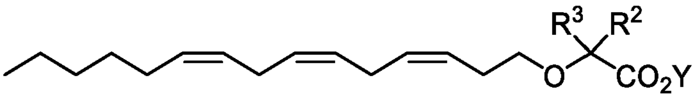

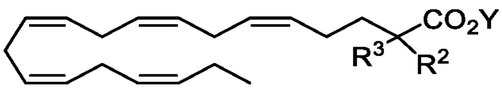

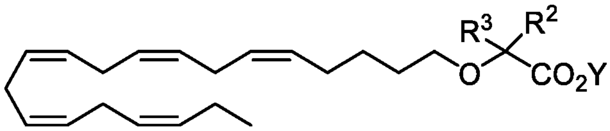

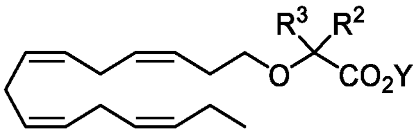

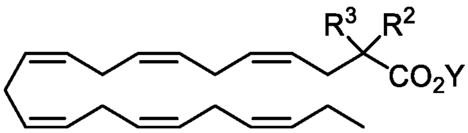

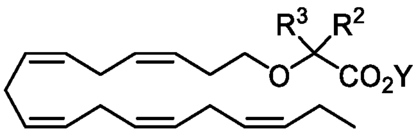

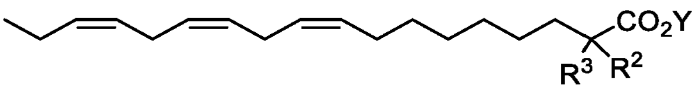

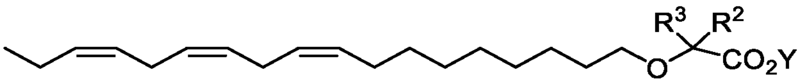

in which $R^2$ and $R^3$ are as defined in any one of claims 1 and 3; and
Y is either hydrogen or an alkyl group, e.g. $C_{1-6}$ alkyl, preferably wherein said compound is:

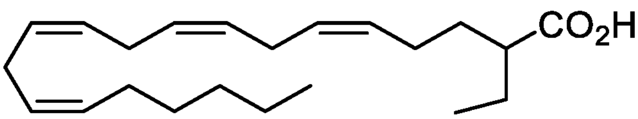

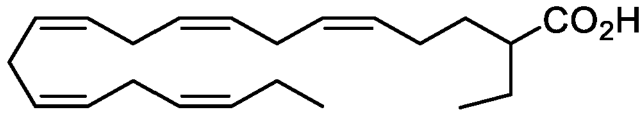

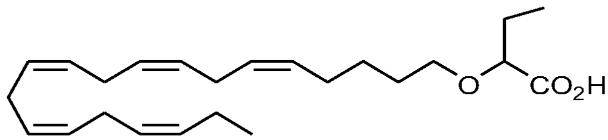

or

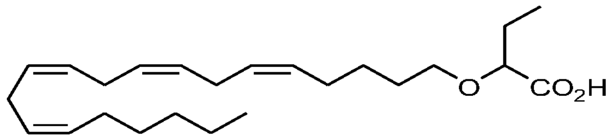

or a pharmaceutically acceptable salt, or ester (e.g. $C_{1-6}$ alkyl ester) thereof.

**9.** An ophthalmic composition for use in topical administration to at least one surface of the eye, said composition comprising a lipid compound of formula (I), or a pharmaceutically acceptable salt thereof as defined in any one of claims 1 and 3 to 8 together with at least one pharmaceutically acceptable carrier or excipient adapted for topical administration, preferably wherein said composition is provided in the form of eye drops or a gel, and/or in the form of an emulsion.

**10.** An omega-6 lipid compound of formula (II), or a pharmaceutically acceptable salt thereof:

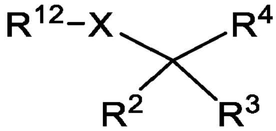

(II)

wherein

$R^{12}$ is a $C_9$ to $C_{22}$ alkenyl group having from 1 to 5 double bonds (e.g. 2 to 5 double bonds) in which:

- the first double bond counting from the ω-end is at carbon 6; and
- where two or more double bonds are present, at least one pair of consecutive double bonds is interrupted by a single methylene group;

$R^2$ is selected from the group consisting of a halogen atom, a hydroxy group, an alkyl group, an alkoxy group, an alkylthio group, a carboxy group, an acyl group, an amino group, and an alkylamino group, preferably $R^2$ is an alkyl group;
$R^3$ is a hydrogen atom, or a group $R^2$;
$R^4$ is a carboxylic acid or a derivative thereof which is a carboxylic ester; and
X is an oxygen or sulfur atom.

**11.** An omega-6 lipid compound as claimed in claim 10, wherein in formula (II), one or more of the following apply:

(i) $R^2$ and/or $R^3$ is an alkyl group, preferably an unsubstituted, straight-chained or branched $C_{1-6}$ alkyl (e.g. $C_{1-3}$ alkyl), e.g. methyl or ethyl;
(ii) $R^3$ is a hydrogen atom; and
(iii) $R^1$ is a straight-chained $C_9$ to $C_{22}$ alkenyl group, preferably a $C_{10}$ to $C_{22}$ alkenyl group, more preferably a $C_{12}$ to $C_{20}$ alkenyl group, e.g. a $C_{20}$ alkenyl group, more preferably a $C_{14}$ to $C_{18}$ alkenyl group, e.g. wherein $R^1$ is a $C_{15}$ or $C_{18}$ alkenyl group.

**12.** An omega-6 lipid compound as claimed in claim 10 or claim 11, wherein at least two double bonds are present in group $R^1$, preferably wherein at least one pair of successive double bonds is interrupted by no more than one methylene group, e.g. wherein each pair of consecutive double bonds is interrupted by no more than one methylene group; and/or wherein all double bonds present in group $R^1$ are either in the E- or Z-configuration, e.g. wherein all double bonds are in the Z-configuration.

**13.** An omega-6 lipid compound as claimed in any one of claims 10 to 12, wherein said compound is derived from an ω-6 PUFA, e.g. derived from (all-Z)-5,8,11,14-icosatetraenoic acid, (all-Z)-4,7,10,13,16-docosapentaenoic acid, (all-Z)-8,11,14-eicosatrienoic acid, or (all-Z)-9,12-octadecadienoic acid.

**14.** An omega-6 lipid compound as claimed in claim 10 which is

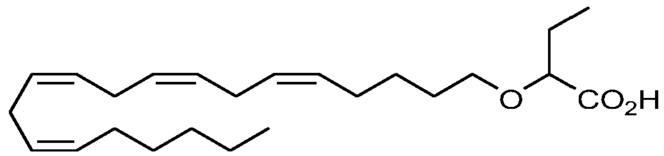

or a pharmaceutically acceptable salt, or ester (e.g. a $C_{1-6}$ alkyl ester) thereof.

**15.** An omega-6 lipid compound as claimed in any one of claims 10 to 14 for use as a medicament.

**16.** A pharmaceutical composition comprising an omega-6 lipid compound as claimed in any one of claims 10 to 14, together with one or more pharmaceutically acceptable carriers, excipients or diluents.

**17.** An oil-in-water emulsion, e.g. an oil-in-water microemulsion, comprising an omega-6 lipid compound of formula (II), or a pharmaceutically acceptable salt thereof, as claimed in any one of claims 10 to 14.

## Patentansprüche

**1.** Lipidverbindung der Formel (I) oder ein pharmazeutisch verträgliches Salz davon zur Verwendung in der Prävention und/oder Behandlung einer Augenerkrankung:

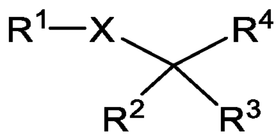

(I)

wobei

$R^1$ eine $C_9$- bis $C_{22}$-Alkenylgruppe mit 1 bis 6 Doppelbindungen ist; $R^2$ aus der Gruppe bestehend aus einem Halogenatom, einer Hydroxylgruppe, einer Alkylgruppe, einer Alkoxygruppe, einer Alkylthiogruppe, einer Carboxygruppe, einer Acylgruppe, einer Aminogruppe und einer Alkylaminogruppe ausgewählt ist;
$R^3$ ein Wasserstoffatom oder eine Gruppe $R^2$ ist;
$R^4$ eine Carbonsäure oder ein Derivat davon ist, das ein Carbonsäureester ist; und
X Methylen ($-CH_2-$) oder ein Sauerstoff- oder Schwefelatom ist.

**2.** Lipidverbindung zur Verwendung nach Anspruch 1, wobei die Augenerkrankung:

(i) aus Katarakt, Hornhautneovaskularisation, Syndrom des trockenen Auges, Glaukom, Keratocornus, Krankheit des trockenen Auges und Sjøgren-Syndrom ausgewählt ist;
(ii) eine entzündliche Augenerkrankung (OID), zum Beispiel eine entzündliche Augenerkrankung, die allergische Konjunktivitis, Heuschnupfen, Uveitis (z. B. vordere Uveitis, intermediäre Uveitis, hintere Uveitis oder Panuveitis-Uveitis), Skleritis, Episkleritis, Endophthalmitis, Optikusneuritis, Keratitis, orbitaler Pseudotumor, retinale Vaskulitis oder chronische Konjunktivitis ist;
(iii) eine entzündliche, autoimmune, vaskuläre und/oder Infektionskrankheit des hinteren Augenabschnitts ist;

(iv) eine netzhautdegenerative Erkrankung, zum Beispiel altersbedingte Makuladegeneration (einschließlich trockener AMD und feuchter AMD), diabetische Retinopathie, diabetisches Makulaödem, Retinitis pigmentosa, Morbus Stargardt, Usher-Syndrom, Bardet-Biedl-Syndrom oder Frühgeborenenretinopathie, vorzugsweise diabetische Retinopathie oder altersbedingte Makuladegeneration (AMD) ist.

**3.** Lipidverbindung zur Verwendung nach Anspruch 1 oder Anspruch 2, wobei in Formel (I) eines oder mehrere der folgenden gilt/gelten:

(i) $R^2$ und/oder $R^3$ ist eine Alkylgruppe, vorzugsweise ein geradkettiges oder verzweigtes $C_{1-6}$-Alkyl (z. B. $C_{1-3}$ -Alkyl), z. B. Methyl oder Ethyl;
(ii) $R^3$ ist ein Wasserstoffatom; und
(iii) $R^1$ ist eine geradkettige $C_9$- bis $C_{22}$-Alkenylgruppe.

**4.** Lipidverbindung zur Verwendung nach einem der Ansprüche 1 bis 3, wobei

(i) $R^1$ eine $C_{10}$- bis $C_{22}$-Alkenylgruppe, stärker bevorzugt eine $C_{12}$ - bis $C_{20}$-Alkenylgruppe, z. B. eine $C_{20}$-Alkenylgruppe, stärker bevorzugt eine $C_{14}$- bis $C_{18}$-Alkenylgruppe, vorzugsweise eine $C_{15}$-oder $C_{17}$-Alkenylgruppe ist; oder
(ii) $R^1$ eine geradkettige ω-3 $C_{9-}$ bis $C_{22}$-Alkenylgruppe mit 1 bis 6 Doppelbindungen oder eine geradkettige ω-6 $C_9$- bis $C_{22}$-Alkenylgruppe mit 1 bis 5 Doppelbindungen ist.

**5.** Lipidverbindung zur Verwendung nach einem der vorstehenden Ansprüche, wobei:

(i) mindestens zwei Doppelbindungen in der Gruppe $R^1$ vorhanden sind, vorzugsweise wobei mindestens ein Paar von aufeinanderfolgenden Doppelbindungen durch nicht mehr als eine Methylengruppe unterbrochen ist, z. B. wobei jedes Paar von aufeinanderfolgenden Doppelbindungen durch nicht mehr als eine Methylengruppe unterbrochen ist; und/oder
(ii) alle in der Gruppe $R^1$ vorhandenen Doppelbindungen entweder in der E- oder Z-Konfiguration vorliegen, wobei vorzugsweise alle Doppelbindungen in der Z-Konfiguration vorliegen.

**6.** Lipidverbindung zur Verwendung nach einem der vorstehenden Ansprüche, wobei in Formel (I):

(i) X $-CH_2-$ und $R^1$ ein $C_9$- bis $C_{22}$-Alkenyl mit 2 bis 6 Doppelbindungen ist, z. B. ein $C_{19}$-, $C_{17}$- oder $C_{15}$-Alkenyl mit 6, 5, 4 oder 3 Doppelbindungen; oder
(ii) X S oder O ist und $R^1$ ein $C_9$- bis $C_{22}$-Alkenyl mit 2 bis 6 Doppelbindungen ist, z. B. ein $C_{22-}$, $C_{20-}$, $C_{18}$- oder $C_{15}$-Alkenyl mit 6, 5, 4 oder 3 Doppelbindungen.

**7.** Lipidverbindung zur Verwendung nach einem der vorstehenden Ansprüche, wobei die Verbindung von einer ω-3-PUFA oder einer ω-6-PUFA abgeleitet ist, z. B. abgeleitet von (all-Z)-4,7,10,13,16,19-Docosahexaensäure, (all-Z)-5,8,11,14,17-Eicosapentaensäure, (all-Z)-7,10,13,16,19-Docosapentaensäure, (all-Z)-9,12,15-Octadecatriensäure, (all-Z)-5,8,11,14-Icosatetraensäure, (all-Z)-4,7,10,13,16-Docosapentaensäure, (all-Z )-8,11,14-Eicosatriensäure oder (all-Z)-9,12-Octadecadiensäure.

**8.** Lipidverbindung zur Verwendung nach Anspruch 1 oder Anspruch 2, wobei die Verbindung aus einem der folgenden oder deren pharmazeutisch verträglichen Salzen ausgewählt ist:

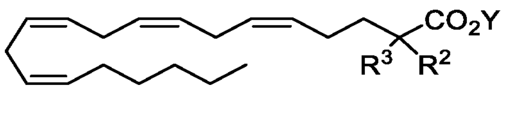

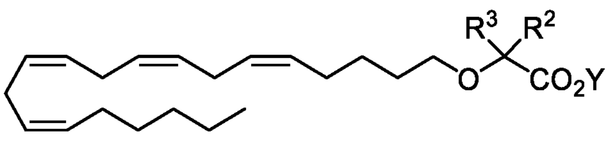

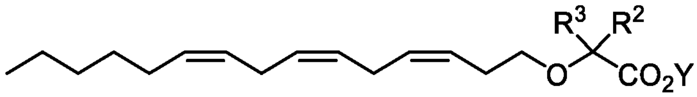

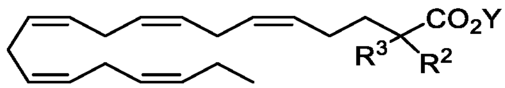

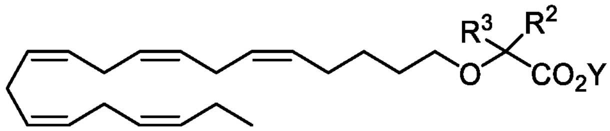

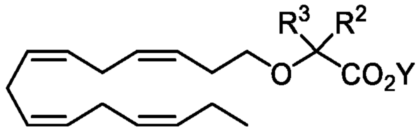

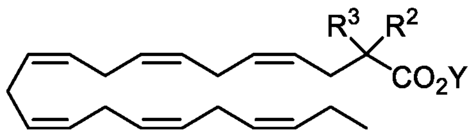

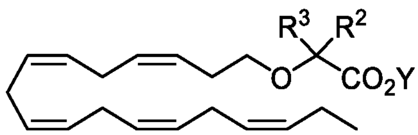

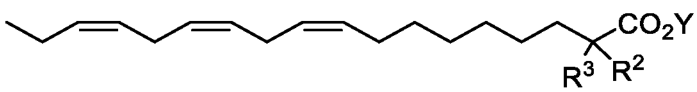

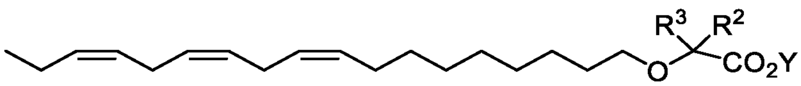

worin $R^2$ und $R^3$ wie in einem der Ansprüche 1 und 3 definiert sind; und Y entweder Wasserstoff oder eine Alkylgruppe ist, z. B. $C_{1-6}$-Alkyl, wobei die Verbindung vorzugsweise:

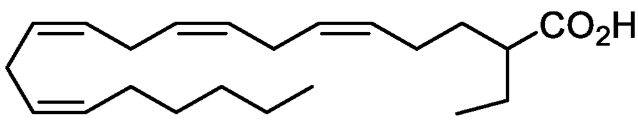

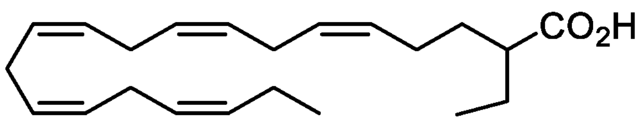

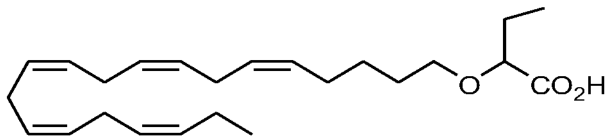

oder

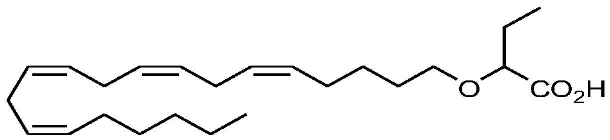

oder ein pharmazeutisch verträgliches Salz oder Ester (z. B. $C_{1-6}$-Alkylester) davon ist.

**9.** Ophthalmische Zusammensetzung zur Verwendung bei der topischen Verabreichung an mindestens eine Oberfläche des Auges, wobei die Zusammensetzung eine Lipidverbindung der Formel (I) oder ein pharmazeutisch verträgliches Salz davon umfasst, wie in einem der Ansprüche 1 und 3 bis 8 definiert, zusammen mit mindestens einem pharmazeutisch verträglichen Träger oder Hilfsstoff, der für die topische Verabreichung geeignet ist, wobei die Zusammensetzung vorzugsweise in Form von Augentropfen oder einem Gel und/oder in Form einer Emulsion bereitgestellt wird.

**10.** Omega-6-Lipidverbindung der Formel II oder ein pharmazeutisch verträgliches Salz davon:

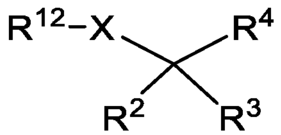

(II)

wobei

$R^{12}$ eine $C_9$- bis $C_{22}$ -Alkenylgruppe mit 1 bis 5 Doppelbindungen (z. B. 2 bis 5 Doppelbindungen) ist, worin:

- sich die erste Doppelbindung vom ω-Ende aus gezählt am Kohlenstoff 6 befindet; und
- bei Vorhandensein von zwei oder mehr Doppelbindungen mindestens ein Paar von aufeinanderfolgenden Doppelbindungen durch eine einzelne Methylengruppe unterbrochen ist;

$R^2$ aus der Gruppe bestehend aus einem Halogenatom, einer Hydroxylgruppe, einer Alkylgruppe, einer Alkoxygruppe, einer Alkylthiogruppe, einer Carboxygruppe, einer Acylgruppe, einer Aminogruppe und einer Alkylaminogruppe ausgewählt ist, vorzugsweise ist $R^2$ eine Alkylgruppe,;
$R^3$ ein Wasserstoffatom oder eine Gruppe $R^2$ ist;
$R^4$ eine Carbonsäure oder ein Derivat davon ist, das ein Carbonsäureester ist; und
X ein Sauerstoff- oder Schwefelatom ist.

**11.** Omega-6-Lipidverbindung nach Anspruch 10, wobei in Formel (II) eines oder mehrere der folgenden gilt/gelten:

(i) $R^2$ und/oder $R^3$ ist eine Alkylgruppe, vorzugsweise ein unsubstituiertes geradkettiges oder verzweigtes $C_{1-6}$-Alkyl (z. B. $C_{1-3}$-Alkyl), z. B. Methyl oder Ethyl;
(ii) $R^3$ ist ein Wasserstoffatom; und
(iii) $R^1$ ist eine geradkettige $C_9$- bis $C_{22}$-Alkenylgruppe, vorzugsweise eine $C_{10}$- bis $C_{22}$-Alkenylgruppe, stärker bevorzugt eine $C_{12}$ - bis $C_{20}$-Alkenylgruppe, z. B. eine $C_{20}$-Alkenylgruppe, stärker bevorzugt eine $C_{14}$- bis $C_{18}$-Alkenylgruppe, z. B. wobei $R^1$ eine $C_{15}$ - oder $C_{18}$ -Alkenylgruppe ist.

**12.** Omega-6-Lipidverbindung nach Anspruch 10 oder Anspruch 11, wobei mindestens zwei Doppelbindungen in der Gruppe $R^1$ vorhanden sind, wobei vorzugsweise mindestens ein Paar von aufeinanderfolgenden Doppelbindungen durch nicht mehr als eine Methylengruppe unterbrochen ist, z. B. wobei jedes Paar von aufeinanderfolgenden

Doppelbindungen durch nicht mehr als eine Methylengruppe unterbrochen ist; und/oder wobei alle in der Gruppe $R^1$ vorhandenen Doppelbindungen entweder in der E- oder Z-Konfiguration vorliegen, z. B. wobei alle Doppelbindungen in der Z-Konfiguration vorliegen.

**13.** Omega-6-Lipidverbindung nach einem der Ansprüche 10 bis 12, wobei die Verbindung von einer ω-6-PUFA abgeleitet ist, z. B. abgeleitet von (all-Z)-5,8,11,14-Icosatetraensäure, (all-Z)-4,7,10,13,16-Docosapentaensäure, (all-Z)-8,11,14-Eicosatriensäure oder (all-Z)-9,12- Octadecadiensäure.

**14.** Omega-6-Lipidverbindung nach Anspruch 10, die

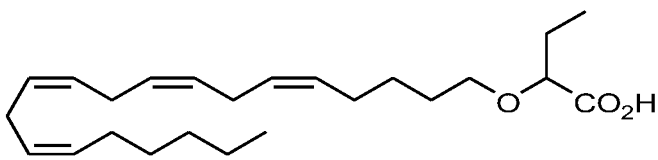

oder ein pharmazeutisch verträgliches Salz oder Ester (z. B. ein $C_{1-6}$-Alkylester) davon ist.

**15.** Omega-6-Lipidverbindung nach einem der Ansprüche 10 bis 14 zur Verwendung als Medikament.

**16.** Pharmazeutische Zusammensetzung, umfassend eine Omega-6-Lipidverbindung nach einem der Ansprüche 10 bis 14 zusammen mit einem oder mehreren pharmazeutisch verträglichen Trägern, Hilfsstoffen oder Verdünnungsmitteln.

**17.** Öl-in-Wasser-Emulsion, z. B. eine Öl-in-Wasser-Mikroemulsion, umfassend eine Omega-6-Lipidverbindung der Formel (II) oder ein pharmazeutisch verträgliches Salz davon nach einem der Ansprüche 10 bis 14.

## Revendications

**1.** Composé lipidique de formule (I), ou un sel pharmaceutiquement acceptable de celui-ci, destiné à être utilisé dans la prévention et/ou le traitement d'un trouble ophtalmique :

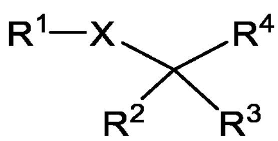

(I)

dans laquelle

$R^1$ est un groupe alcényle en $C_9$ à $C_{22}$ ayant de 1 à 6 doubles liaisons ;
$R^2$ est choisi dans le groupe constitué d'un atome d'halogène, d'un groupe hydroxyle, d'un groupe alkyle, d'un groupe alcoxy, d'un groupe alkylthio, d'un groupe carboxy, d'un groupe acyle, d'un groupe amino et d'un groupe alkylamino ;
$R^3$ est un atome d'hydrogène, ou un groupe $R^2$ ;
$R^4$ est un acide carboxylique ou un dérivé de celui-ci qui est un ester carboxylique ; et
X est un méthylène ($-CH_2-$), ou un atome d'oxygène ou de soufre.

**2.** Composé lipidique destiné à être utilisé selon la revendication 1, dans lequel ledit trouble ophtalmique est :

(i) choisi parmi les cataractes, la néovascularisation cornéenne, le syndrome de l'œil sec, le glaucome, le kératocorne, la maladie de l'œil sec et le syndrome de Sjøgren ;
(ii) une maladie inflammatoire oculaire (OID), par exemple une maladie inflammatoire oculaire qui est la conjonctivite allergique, le rhume des foins, l'uvéite (par exemple, l'uvéite antérieure, l'uvéite intermédiaire, l'uvéite postérieure ou l'uvéite panuvéite), la sclérite, l'épisclérite, l'endophtalmie, la névrite optique, la kératite, la pseudotumeur orbitaire, la vascularite rétinienne ou la conjonctivite chronique ;
(iii) une maladie inflammatoire, auto-immune, vasculaire et/ou infectieuse du segment postérieur de l'œil ;

(iv) une maladie dégénérative de la rétine, par exemple la dégénérescence maculaire liée à l'âge (y compris la forme sèche de la DMLA et la forme humide de la DMLA), la rétinopathie diabétique, l'œdème maculaire diabétique, la rétinite pigmentaire, la maladie de Stargardt, le syndrome d'Usher, le syndrome de Bardet-Biedl, ou la rétinopathie du prématuré, de préférence la rétinopathie diabétique ou la dégénérescence maculaire liée à l'âge (DMLA).

**3.** Composé lipidique destiné à être utilisé selon la revendication 1 ou la revendication 2, dans lequel, dans la formule (I), un ou plusieurs des éléments suivants s'appliquent :

(i) $R^2$ et/ou $R^3$ sont un groupe alkyle, de préférence un alkyle en $C_{1-6}$ linéaire ou ramifié (par exemple alkyle en $C_{1-3}$), par exemple méthyle ou éthyle ;
(ii) $R^3$ est un atome d'hydrogène ; et
(iii) $R^1$ est un groupe alcényle en $C_9$ à $C_{22}$ linéaire.

**4.** Composé lipidique destiné à être utilisé selon l'une quelconque des revendications 1 à 3, dans lequel

(i) $R^1$ est un groupe alcényle en $C_{10}$ à $C_{22}$, de manière davantage préférée un groupe alcényle en $C_{12}$ à $C_{20}$, par exemple un groupe alcényle en $C_{20}$, de manière davantage préférée un groupe alcényle en $C_{14}$ à $C_{18}$, de préférence un groupe alcényle en $C_{15}$ ou en $C_{17}$ ; ou
(ii) $R^1$ est un groupe alcényle ω-3 en $C_9$ à $C_{22}$ linéaire ayant de 1 à 6 doubles liaisons, ou un groupe alcényle ω-6 en $C_9$ à $C_{22}$ linéaire ayant de 1 à 5 doubles liaisons.

**5.** Composé lipidique destiné à être utilisé selon l'une quelconque des revendications précédentes, dans lequel :

(i) au moins deux doubles liaisons sont présentes dans le groupe $R^1$, de préférence dans lequel au moins une paire de doubles liaisons successives est interrompue par un groupe méthylène maximum, par exemple dans lequel chaque paire de doubles liaisons consécutives est interrompue par un groupe méthylène maximum ; et/ou
(ii) toutes les doubles liaisons présentes dans le groupe $R^1$ sont soit dans la configuration *E,* soit dans la configuration Z, de préférence dans lequel toutes les doubles liaisons sont dans la configuration Z.

**6.** Composé lipidique destiné à être utilisé selon l'une quelconque des revendications précédentes, dans lequel dans la formule (I) :

(i) X est $-CH_2-$ et $R^1$ est un alcényle en $C_9$ à $C_{22}$ ayant 2 à 6 doubles liaisons, par exemple un alcényle en $C_{19}$, $C_{17}$ ou $C_{15}$ ayant 6, 5, 4 ou 3 doubles liaisons ; ou
(ii) X est S ou O, et $R^1$ est un alcényle en $C_9$ à $C_{22}$ ayant 2 à 6 doubles liaisons, par exemple un alcényle en $C_{22}$, $C_{20}$, $C_{18}$ ou $C_{15}$ ayant 6, 5, 4 ou 3 doubles liaisons.

**7.** Composé lipidique destiné à être utilisé selon l'une quelconque des revendications précédentes, dans lequel ledit composé est dérivé d'un PUFA ω-3 ou d'un PUFA ω-6, par exemple dérivé de l'acide (tout-Z)-4,7,10,13,16,19-docosahexaénoïque, l'acide (tout-Z)-5,8,11,14,17-éicosapentaénoïque, l'acide (tout-Z)-7,10,13,16,19-docosapentaénoïque, l'acide (tout-Z)-9,12,15-octadécatriénoïque, l'acide (tout-Z)-5,8,11,14-icosatétraénoïque, l'acide (tout-Z)-4,7,10,13,16-docosapentaénoïque, l'acide (tout-Z )-8,11,14-éicosatriénoïque ou l'acide (tout-Z)-9,12-octadécadiénoïque.

**8.** Composé lipidique destiné à être utilisé selon la revendication 1 ou la revendication 2, dans lequel ledit composé est choisi parmi l'un quelconque des composés suivants, ou de leurs sels pharmaceutiquement acceptables :

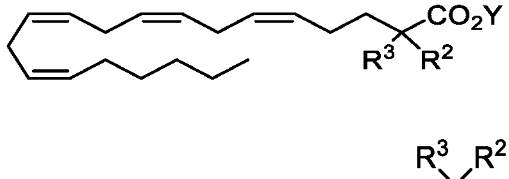

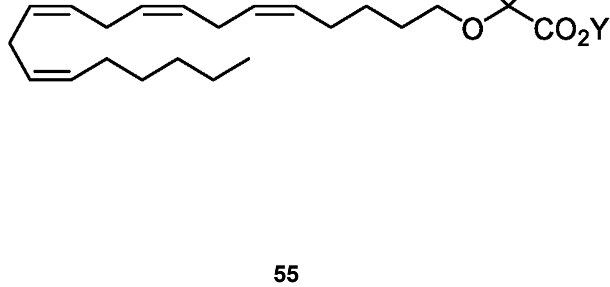

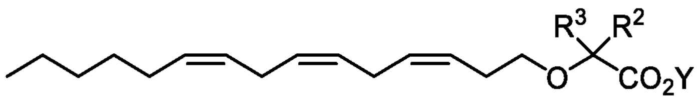

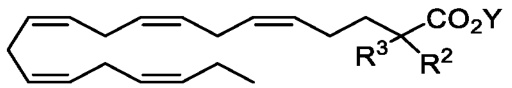

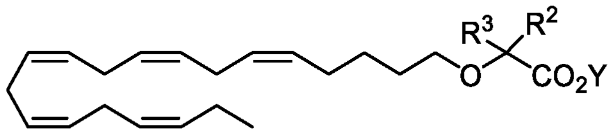

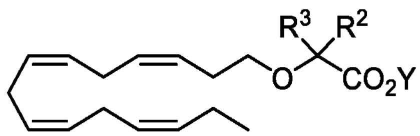

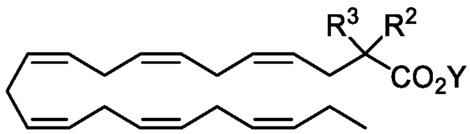

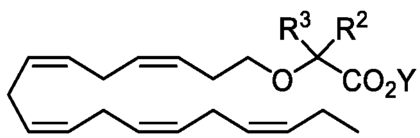

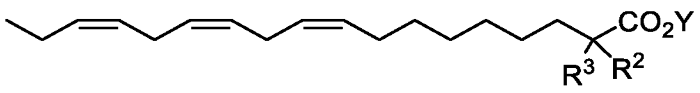

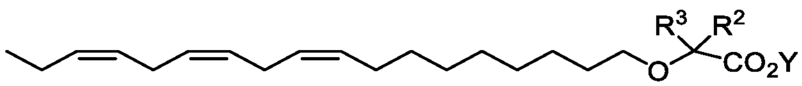

dans lequel $R^2$ et $R^3$ sont tels que définis dans l’une quelconque des revendications 1 et 3 ; et
Y est soit un atome d’hydrogène, soit un groupe alkyle, par exemple un alkyle en $C_{1-6}$, de préférence dans lequel ledit composé est :

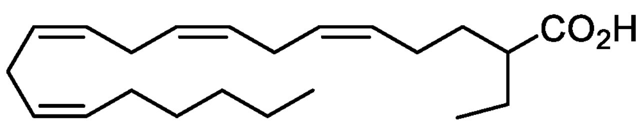

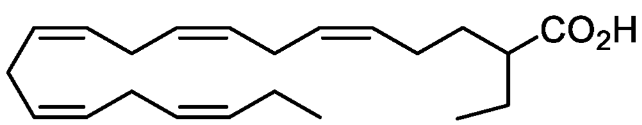

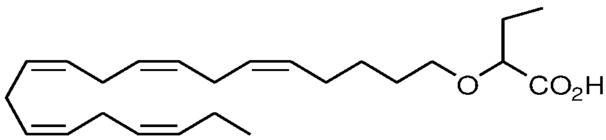

ou

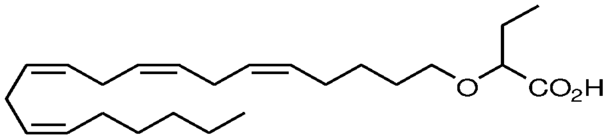

ou un sel pharmaceutiquement acceptable, ou un ester (par exemple un ester d'alkyle en $C_{1-6}$) de ceux-ci.

**9.** Composition ophtalmique destinée à être utilisée en administration topique sur au moins une surface de l'œil, ladite composition comprenant un composé lipidique de formule (I), ou un sel pharmaceutiquement acceptable de celui-ci tel que défini dans l'une quelconque des revendications 1 et 3 à 8 avec au moins au moins un support ou excipient pharmaceutiquement acceptable adapté à une administration topique, de préférence dans laquelle ladite composition se présente sous forme de collyre ou de gel, et/ou sous forme d'émulsion.

**10.** Composé lipidique oméga-6 de formule (II), ou sel pharmaceutiquement acceptable de celui-ci :

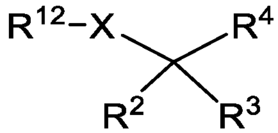

(II)

dans laquelle

$R^{12}$ est un groupe alcényle en $C_9$ à $C_{22}$ ayant de 1 à 5 doubles liaisons (par exemple 2 à 5 doubles liaisons) dans lequel :

- la première double liaison à partir de l'extrémité ω est au niveau du carbone 6 ; et
- lorsque deux doubles liaisons ou plus sont présentes, au moins une paire de doubles liaisons consécutives est interrompue par un seul groupe méthylène ;

$R^2$ est choisi dans le groupe constitué d'un atome d'halogène, d'un groupe hydroxy, d'un groupe alkyle, d'un groupe alcoxy, d'un groupe alkylthio, d'un groupe carboxy, d'un groupe acyle, d'un groupe amino et d'un groupe alkylamino, de préférence $R^2$ est un groupe alkyle ;
$R^3$ est un atome d'hydrogène, ou un groupe $R^2$ ;
$R^4$ est un acide carboxylique ou un dérivé de celui-ci qui est un ester carboxylique ; et
X est un atome d'oxygène ou de soufre.

**11.** Composé lipidique oméga-6 selon la revendication 10, dans lequel, dans la formule (II), un ou plusieurs des éléments suivants s'appliquent :

(i) $R^2$ et/ou $R^3$ sont un groupe alkyle, de préférence un alkyle en $C_{1-6}$, non substitué, linéaire ou ramifié (par exemple un alkyle en $C_{1-3}$), tel que le méthyle ou l'éthyle ;
(ii) $R^3$ est un atome d'hydrogène ; et
(iii) $R^1$ est un groupe alcényle en $C_9$ à $C_{22}$ linéaire, de préférence un groupe alcényle en $C_{10}$ à $C_{22}$, de manière davantage préférée un groupe alcényle en $C_{12}$ à $C_{20}$, par exemple un groupe alcényle en $C_{20}$, de manière davantage préférée un groupe alcényle en $C_{14}$ à $C_{18}$, par exemple dans lequel $R^1$ est un groupe alcényle en $C_{15}$ ou $C_{18}$ .

**12.** Composé lipidique oméga-6 selon la revendication 10 ou la revendication 11, dans lequel au moins deux doubles

liaisons sont présentes dans le groupe $R^1$, de préférence dans lequel au moins une paire de doubles liaisons successives est interrompue par un groupe méthylène maximum, par exemple dans lequel chaque paire de doubles liaisons consécutives est interrompue par un groupe méthylène maximum ; et/ou dans lequel toutes les doubles liaisons présentes dans le groupe $R^1$ sont soit dans la configuration E- soit dans la configuration Z-, par exemple dans lequel toutes les doubles liaisons sont dans la configuration Z-.

**13.** Composé lipidique oméga-6 selon l'une quelconque des revendications 10 à 12, dans lequel ledit composé est dérivé d'un PUFA ω-6, par exemple dérivé de l'acide (tout-Z)-5,8,11,14-icosatétraénoïque, l'acide (tout-Z)-4,7,10,13,16-docosapentaénoïque, l'acide (tout-Z)-8,11,14-éicosatriénoïque ou l'acide (tout-Z)-9,12-octadécadiénoïque.

**14.** Composé lipidique oméga-6 selon la revendication 10, qui est

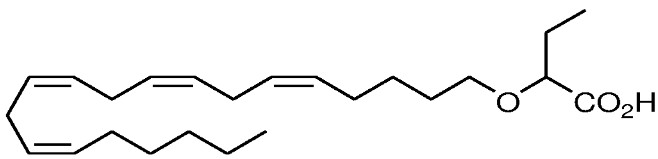

ou un sel pharmaceutiquement acceptable, ou un ester (par exemple un ester d'alkyle en $C_{1-6}$) de celui-ci.

**15.** Composé lipidique oméga-6 selon l'une quelconque des revendications 10 à 14 destiné à être utilisé comme médicament.

**16.** Composition pharmaceutique comprenant un composé lipidique oméga-6 selon l'une quelconque des revendications 10 à 14, conjointement avec un ou plusieurs supports, excipients ou diluants pharmaceutiquement acceptables.

**17.** Emulsion huile-dans-eau, par exemple une microémulsion huile-dans-eau, comprenant un composé lipidique oméga-6 de formule (II), ou un sel pharmaceutiquement acceptable de celui-ci, selon l'une quelconque des revendications 10 à 14.

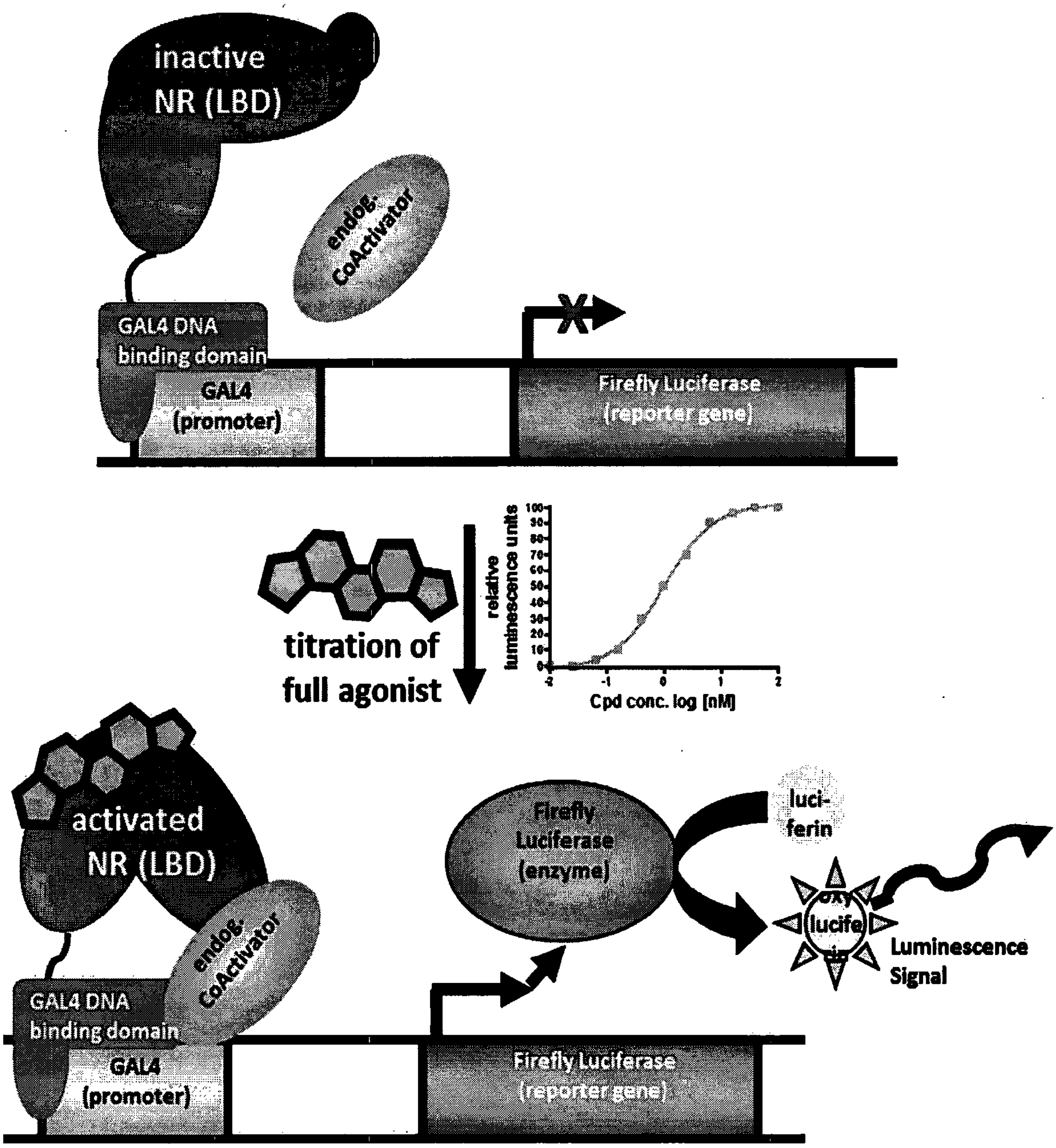


**Figure 1:** Principle of cell based M1H assays in agonist mode

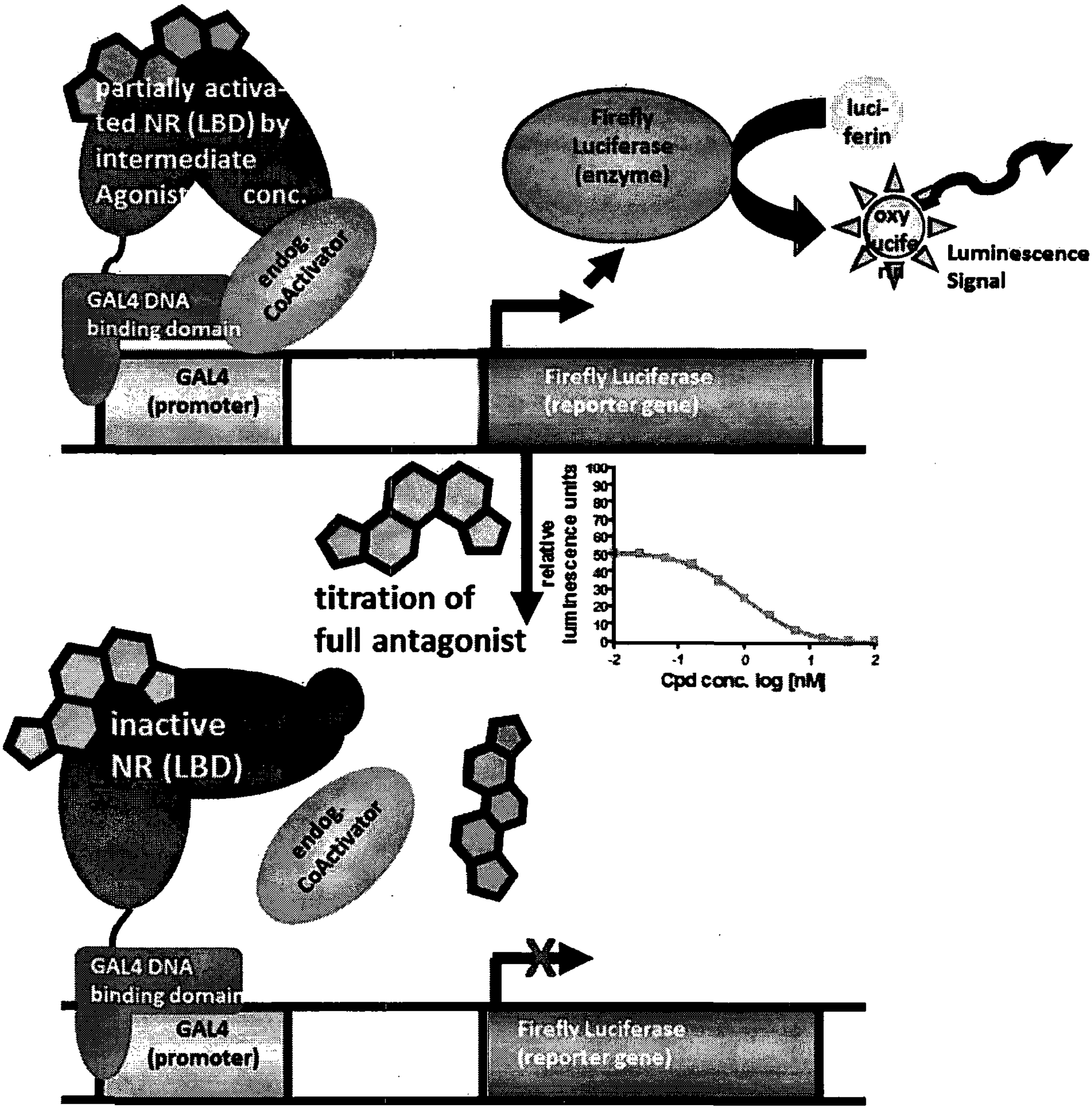


**Figure 2:** Principle of cell based M1H assays in antagonist mode

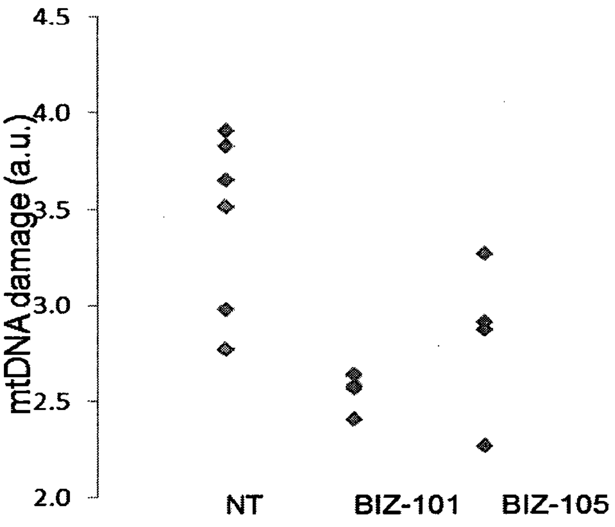


**Figure 3:** Results of mtDNA study in Example 7

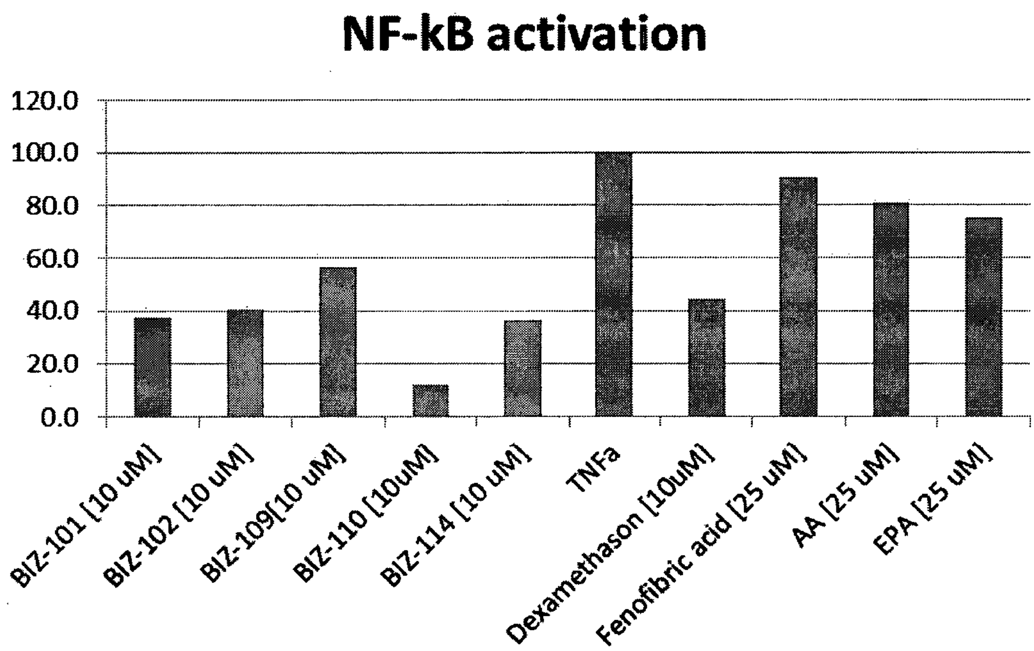


**Figure 4**

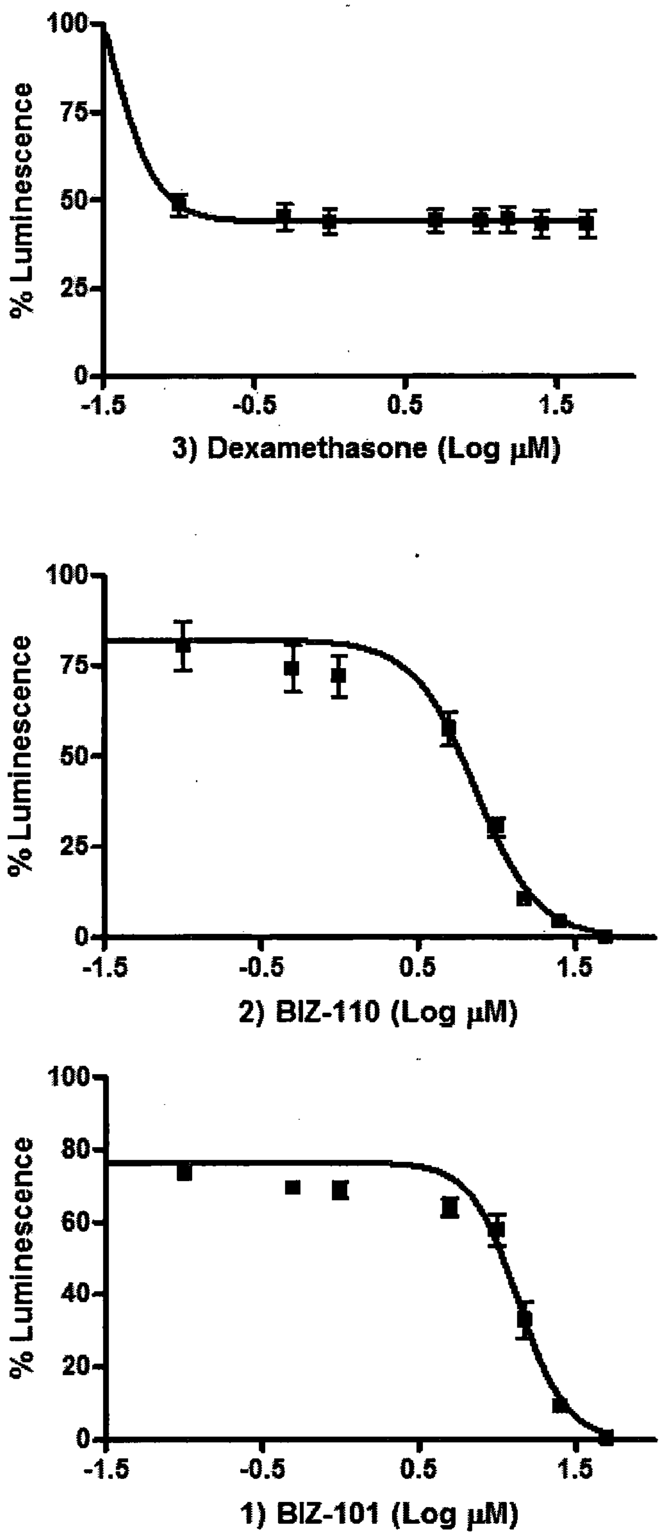
100
75
50
25
0
% Luminescence
-1.5
-0.5
0.5
1.5
3) Dexamethasone (Log µM)
100
75
50
25
0
% Luminescence
-1.5
-0.5
0.5
1.5
2) BIZ-110 (Log µM)
100
80
60
40
20
0
% Luminescence
-1.5
-0.5
0.5
1.5
1) BIZ-101 (Log µM)

**Figure 5**

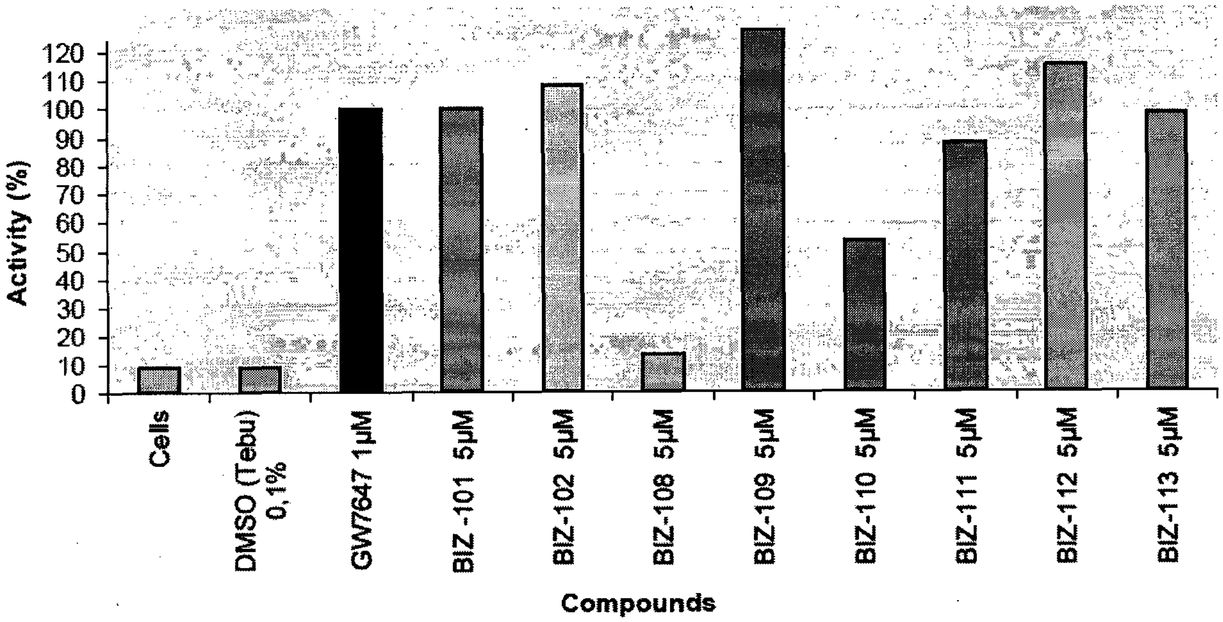


Figure 6

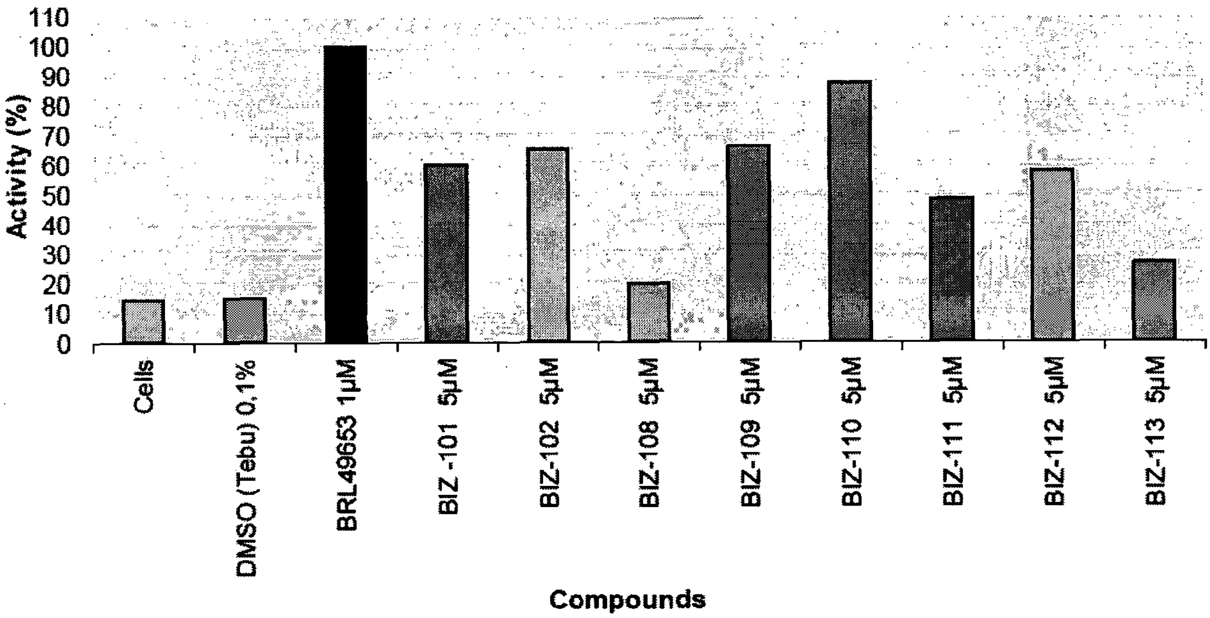


Figure 7

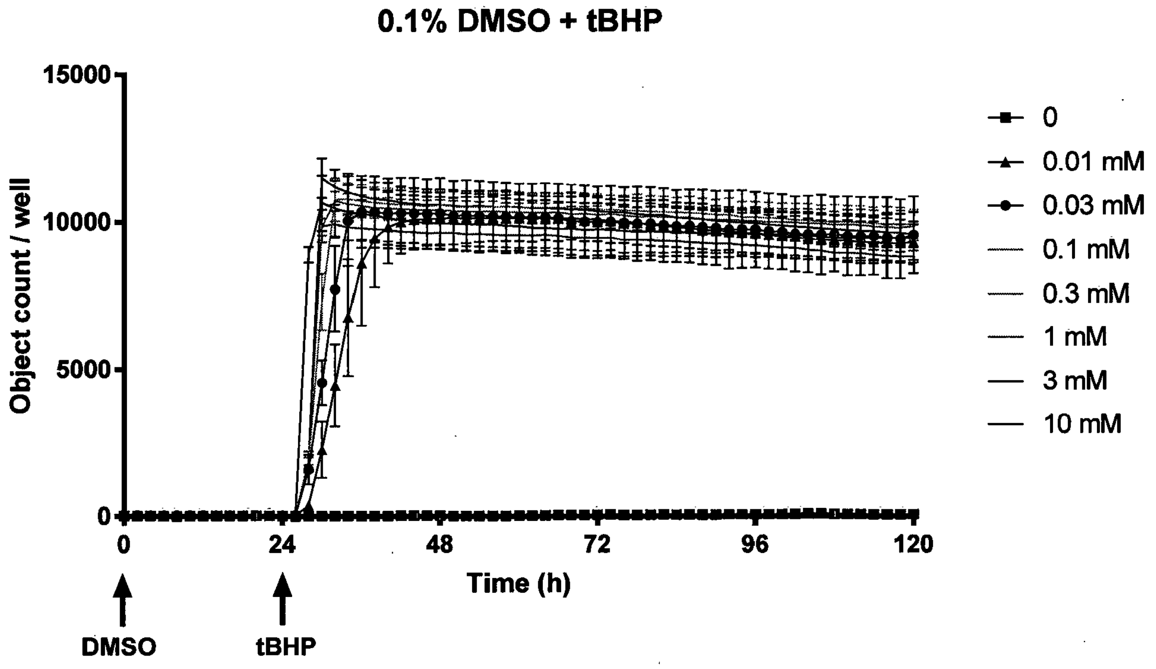


Figure 8A

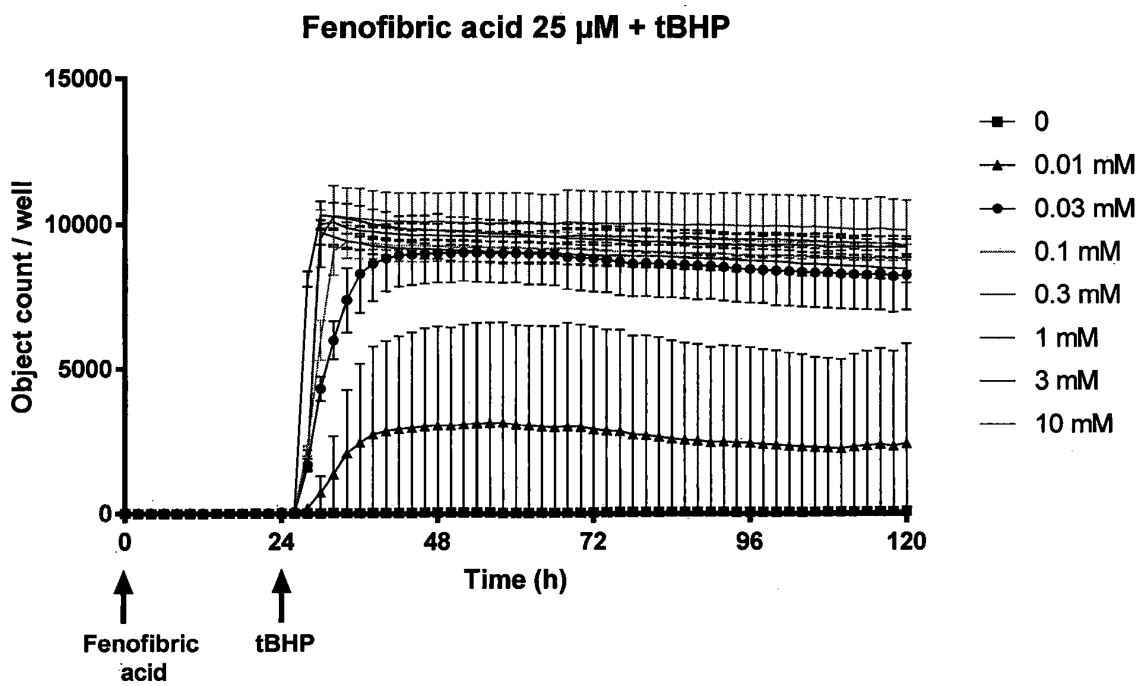


Figure 8B

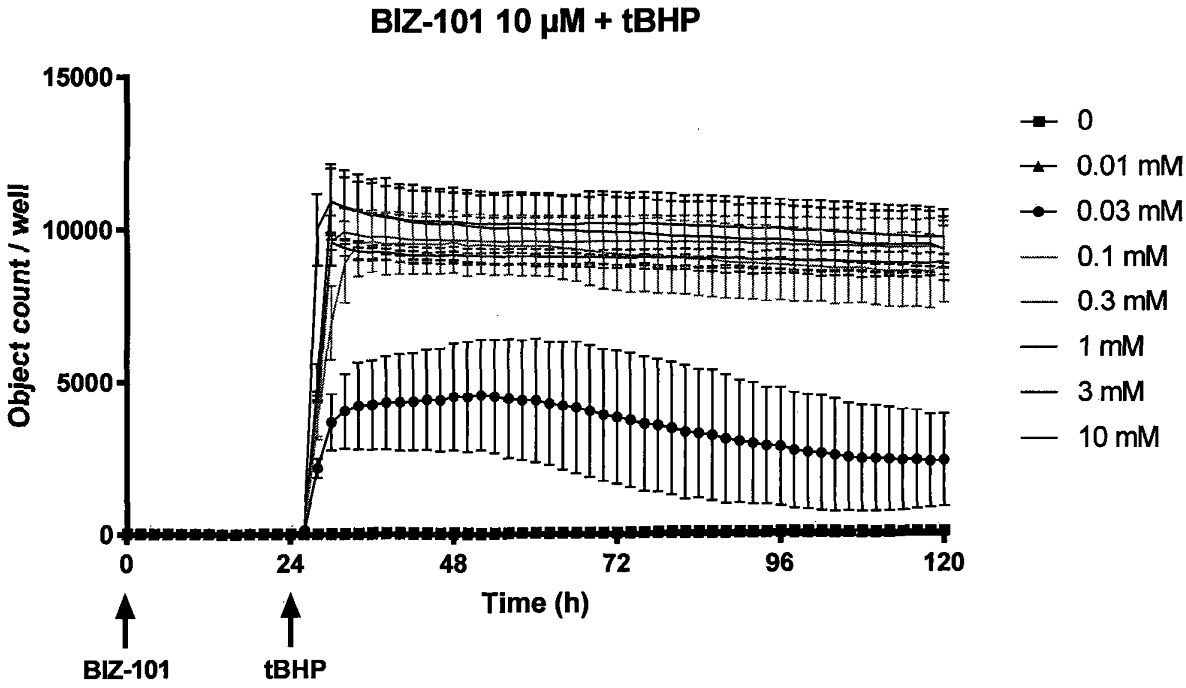
BIZ-101 10 µM + tBHP
Object count / well
15000
10000
5000
0
0
24
48
72
96
120
Time (h)
BIZ-101
tBHP
0
0.01 mM
0.03 mM
0.1 mM
0.3 mM
1 mM
3 mM
10 mM

Figure 8C

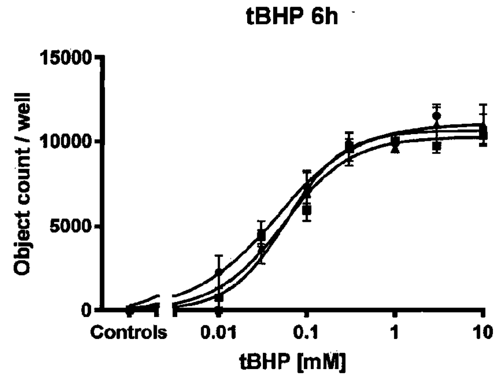


| | EC50 |
|---|---|
| 0.1% DMSO | 0.04434 |
| Fenofibric acid 25 µM | 0.05381 |
| BIZ-101 10 µM | 0.05824 |

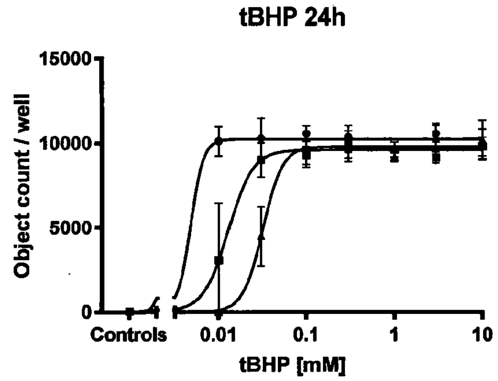


| | EC50 |
|---|---|
| 0.1% DMSO | ~ 0.004760 |
| Fenofibric acid 25 µM | 0.01276 |
| BIZ-101 10 µM | 0.03131 |

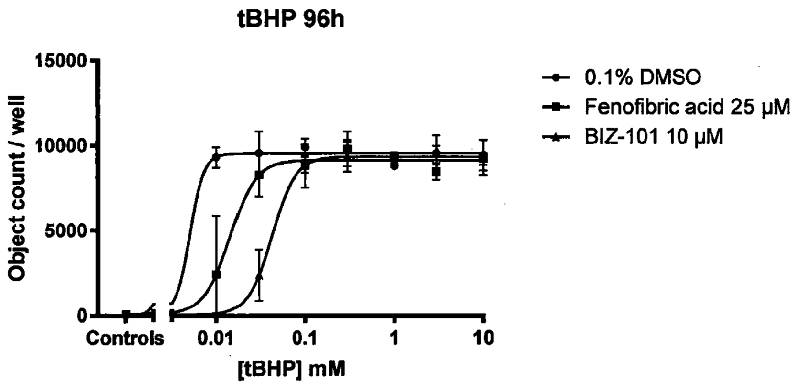


| | EC50 |
|---|---|
| 0.1% DMSO | ~ 0.005058 |
| Fenofibric acid 25 µM | 0.01419 |
| BIZ-101 10 µM | 0.04192 |

**Figure 8D**

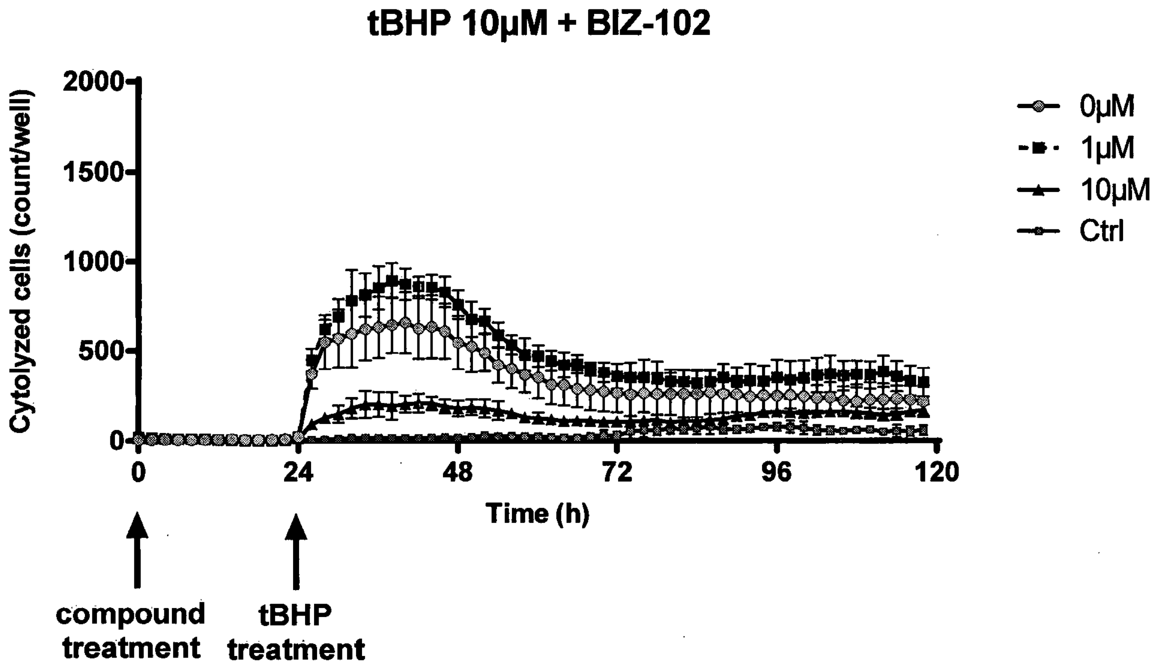


Figure 9A

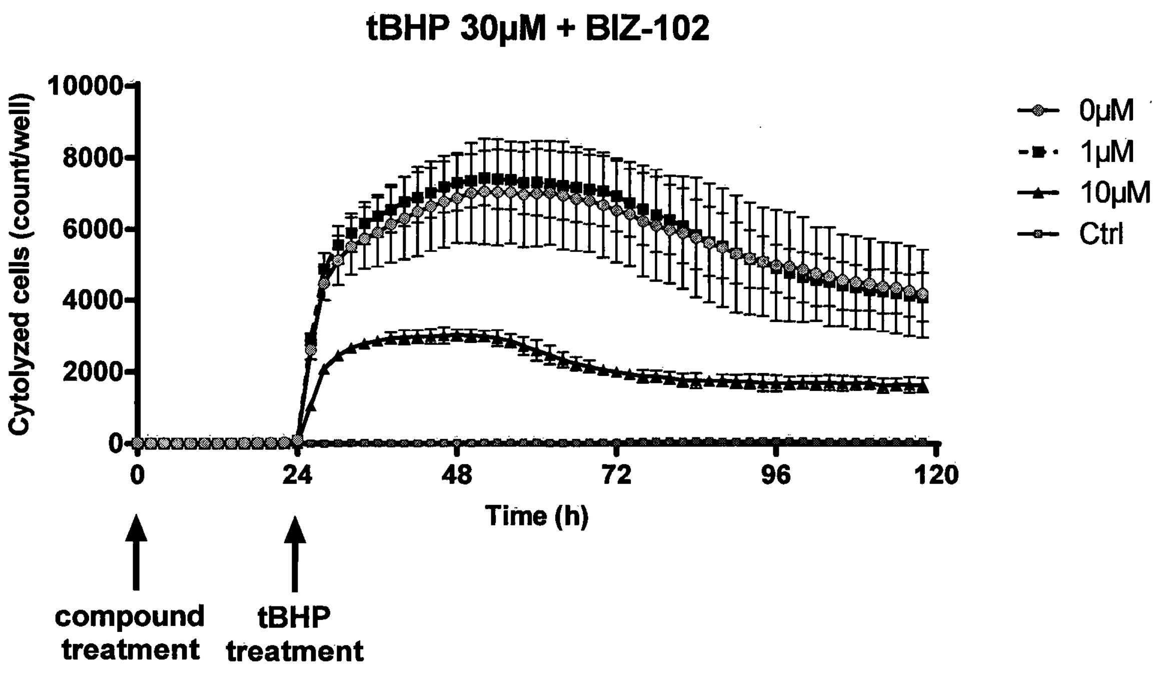


Figure 9B

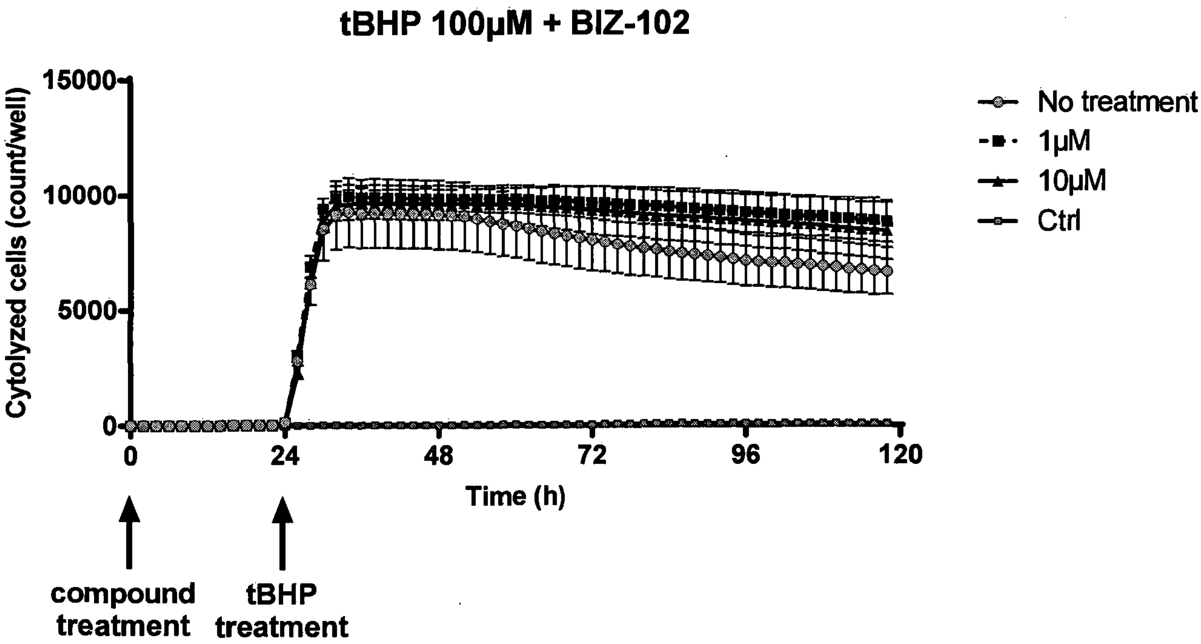
tBHP 100µM + BIZ-102
Cytolyzed cells (count/well)
15000
10000
5000
0
0
24
48
72
96
120
Time (h)
No treatment
1µM
10µM
Ctrl
compound treatment
tBHP treatment

Figure 9C

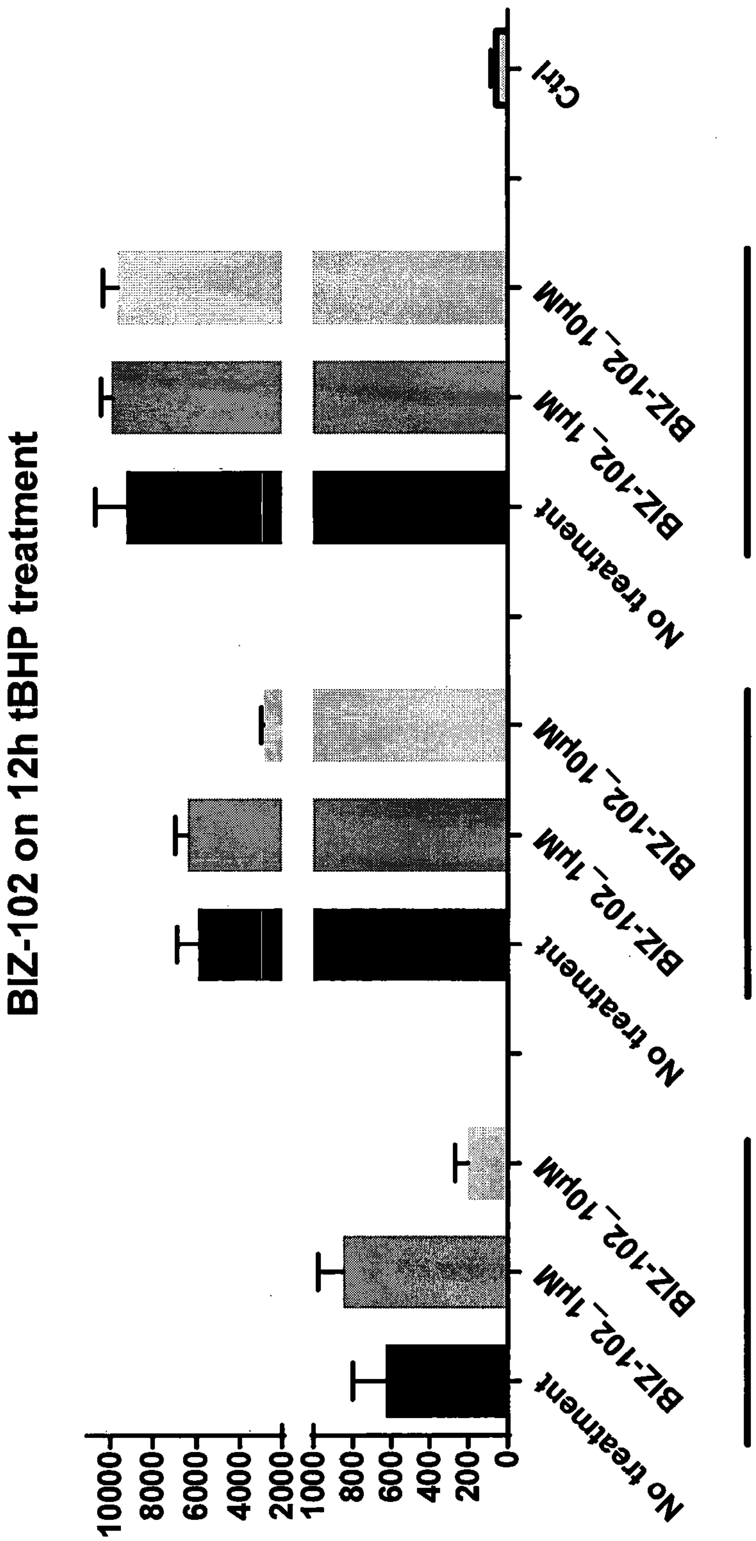

BIZ-102 on 12h tBHP treatment
Cytolyzed cells (count/well)
10000
8000
6000
4000
2000
1000
800
600
400
200
0
No treatment
BIZ-102_1uM
BIZ-102_10uM
Ctrl
tBHP 10μM
tBHP 30μM
tBHP 100μM


Figure 9D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006117664 A
- WO 2010128401 A
- WO 2010008299 A
- IL 113228
- WO 0130337 A
- US 2005137123 A
- WO 2005060542 A
- WO 2011064779 A
- WO 2006044391 A
- EP 2409963 A
- US 2006269495 A
- US 3972907 A
- EP 0413528 A
- WO 2013049621 A
- US 9241900 B
- EP 2444063 A
- US 5874469 A
- US 20140275263 A
- WO 2008053331 A
- WO 2008142482 A
- WO 2012059818 A
- GB 2003002582 W
- EP 0345038 A
- US 20040192908 A


### Non-patent literature cited in the description


- *The Journal of Neuroscience,* May 2015, 7304
- **R. SIMO et al.** *Diabetes Care,* 2009, 1556
- **A. CIUDIN et al.** *PPAR Res.,* 2013, 686525
- **Y. HU et al.** *Proceedings of the National Academy of Sciences of the United States of America,* 2013, vol. 110 (38), 15401-15406
- **DEL. V. CANO et al.** *PPAR Res.,* 2008, 821592
- *Toxicology and Applied Pharmacology,* 2007, vol. 223, 277-287
- **LARSEN et al.** *Lipids,* 2005, vol. 49
- **LIN et al.** *Invest. Ophthalmol. Vis. Sci.,* 2001, 3521
- **MUKHERJEE.** *PNAS,* 2004, vol. 101, 8491
- **LOPEZ et al.** *Plos One,* 27 August 2023, vol. 8 (8
- **PARKKARI et al.** *Bioorganic & Medicinal Chemistry Letters,* 2006, vol. 16 (9
- **RYAN et al.** *Bioorganic & Medicinal Chemistry Letters,* 1997, vol. 7 (20
- **HOWLET et al.** *Life Sciences,* 1999, vol. 65
- **LIANG et al.** *Advances in Prostaglandin and Throxane Research,* 1980, vol. 6
- **E.B. SUOTO et al.** *Current Eye Research,* 2010, vol. 35 (7), 537-552
- **SCHRIER ; FALK.** *Curr. Opin. Ophthalmol.,* September 2011, vol. 22 (5), 325-331
- **JARRETT et al.** *Ophthalmic Res.,* 2010, vol. 44, 179-190
- **COREY et al.** *Tetrahedron Letters,* 1983, vol. 24, 265-268
- **S. DURAND et al.** *J. Chem. Soc., Perkin Trans.,* 2000, vol. 1, 253-273
- **VIALA et al.** *J. Org. Chem,* 1988, vol. 53, 6121
- **HERMETTER.** *Chemistry and Physics of lipids,* 1981, vol. 28, 111
- **WANG et al.** *J. Am. Chem. Soc,* 1993, vol. 115, 10487
- **HOSOKAWA.** *J. Am. Oil Chem. Soc,* 1995, 1287
- **LILJA-HALLBERG.** *Biocatalysis,* 1994, 195
- **O'BRIAN.** *J. Org. Chem.,* 1996, 5914
- **PARKKARI et al.** *Bioorg. Med. Chem. Lett.,* 2006, 2437
- **HARALDSSON.** *Pharmazie,* 2000, vol. 3
- **LARSEN et al.** *Biochemical Pharmacology,* 1998, 405
- **LARSEN et al.** *Lipids,* 2005, vol. 40
- **FLOCK et al.** *Acta Chemica Scandinavica,* 1999, vol. 53, 436
- **FLOCK et al.** *Acta chemical scandinavica,* 1999, vol. 53, 436